# EUROPÄISCHE PATENTANMELDUNG

(11) **EP 4 001 296 A1**
(43) Veröffentlichungstag der Anmeldung: **25.05.2022**
(21) Anmeldenummer: 20208947.0
(22) Anmeldetag: 20.11.2020
(51) Int. Cl.: C07K 7/56, A61P 31/04, A61K 38/00

(54) **NEUE ZYKLISCHE VERBINDUNGEN, VERFAHREN ZU DEREN HERSTELLUNG UND DIE VERWENDUNG DIESER ZYKLISCHEN VERBINDUNGEN IN KOSMETISCHEN ZUBEREITUNGEN**

(71) Anmelder: Eberhard Karls Universität Tübingen, 72074 Tübingen (DE)
(72) Erfinder: WIRTZ, Sebastian N., 72072 Tübingen (DE); GROND, Stephanie, 72060 Tübingen (DE); SAUR, Julian S., 72070 Tübingen (DE); KRISMER, Bernhard, 72138 Kirchentellinsfurt (DE)
(74) Vertreter: Uexküll & Stolberg

(57) **Zusammenfassung**

Die vorliegende Erfindung betrifft neue zyklische Verbindungen; ein Verfahren zur Festphasensynthese dieser neuen zyklischen Verbindungen; neue Thiazolidin- und Oxazolidin-Bausteine, die direkt in die Festphasensynthese der neuen zyklischen Verbindungen eingebunden werden können, und ein neues Verfahren zur Synthese der Thiazolidin- und Oxazolidin-Bausteine; sowie kosmetische Zusammensetzungen, die die neuen zyklischen Verbindungen enthalten.

## Beschreibung

Die vorliegende Erfindung betrifft neue zyklische Verbindungen; ein Verfahren zur Festphasensynthese dieser neuen zyklischen Verbindungen; neue Thiazolidin- und Oxazolidin-Bausteine, die direkt in die Festphasensynthese der neuen zyklischen Verbindungen eingebunden werden können, und ein neues Verfahren zur Synthese der Thiazolidin- und Oxazolidin-Bausteine; sowie die Verwendung der zyklischen Verbindungen in kosmetischen Zusammensetzungen.

### Hintergrund der Erfindung

Unreine Haut, Akne und andere Hautphänomene, -zustände und -beschwerden, die der Behandlung mit kosmetischen Präparaten zugänglich sind beeinträchtigen das Wohlbefinden der Betroffenen selbst in leichten Fällen. Praktisch jeder Mensch ist zu irgendeinem Zeitpunkt von solchen Haut-phänomenen, -zuständen oder -beschwerden betroffen. In vielen Fällen können diese mit kosmetischen Präparaten behandelt werden, ohne dass pharmazeutische Präparate mit starken pharmazeutischen Wirkstoffen benötigt werden, welche Nebenwirkungen aufweisen und so für die behandelte Person negative Folgen haben können.

Solche pharmazeutischen Präparate mit hochpotenten pharmazeutischen Wirkstoffen eignen sich in der Regel nicht für die kosmetische Anwendungen, da für den Patient, der mit solchen pharmazeutischen Präparaten behandelt wird, bei ihrer Anwendung oft in irgendeiner Weise z.B. auf der systemischen Ebene, insbesondere in den Stoffwechselfunktionen des Körpers, negative Wirkungen bzw. Folgen auftreten können.

Peptide und Peptidderivate in Einzellern bis hin zu komplexen Mehrzellern wie dem Menschen haben eine wichtige Signalfunktion und koordinieren viele biochemische Prozesse, wobei die jeweiligen Funktionen jeweils durch die spezifische Struktur eines Peptids oder Peptidderivats definiert werden. Wegen dieser strukturspezifischen Funktionsvielfalt stellen sie vielversprechende Bestandteile von Zubereitungen dar, insbesondere in der Kosmetikindustrie, wo ständig nach neuen Verbindungen gesucht wird, die die Haut verschönern können zur Verbesserung des Allgemeinbefindens.

Peptide und Peptidderivate werden in kosmetischen Präparaten seit langem verwendet. Beispielsweise EP 0 296 078, EP 0 462 426, EP 2340856 und US-Patent Nr. 5,1 16,824, 6,541,023 und 5,808,050 offenbaren kosmetische Präparate, die Chitosane, Kollagene und Glykosylaminoglykane enthalten. Die EP 3 062 763 offenbart kosmetische Zubereitung umfassend Glycoprotein 1 und 2.

Bestimmte zyklische Peptide und Peptidderivate mit spezifischen Strukturmerkmalen werden im Stand der Technik zur Verwendung in kosmetischen Präparaten, beispielsweise zur Erhaltung oder Verbesserung des Allgemeinzustandes der Haut oder der Haare, als Alterungsschutz und zur Verringerung des Auftretens von Falten, vorgeschlagen.

Die WO 2009/124754 offenbart beispielsweise die Verwendung von bestimmten zyklischen Peptiden in topischen, kosmetischen und/oder Körperpflegezusammensetzungen mit verbesserten Handhabungseigenschaften, verbesserten Stabilitätseigenschaften und/oder einer vorteilhaften Integrin-modulierender Aktivität, zur Pflege, Erhaltung oder Verbesserung des Allgemeinzustandes der Haut oder der Haare und zur Prophylaxe gegen zeit- und/oder lichtbedingte Alterungsprozesse der menschlichen Haut oder des menschlichen Haares sowie zur Prophylaxe und/oder Behandlung von Hautkrankheiten, die mit einer fehlerhaften Keratinisierung im Zusammenhang mit Differenzierung und Zellproliferation einhergehen.

Die US Patentschrift US 9,364,413 B2 offenbart die Verwendung des zykloaliphatischen Heptapeptids Surfactin in kosmetischen Zusammensetzungen als Alterungsschutz, zur Faltenbekämpfung und zur Erhöhung der Hautpenetration.

WO 2019/149450 A1 offenbart die Verwendung bestimmter zyklischer Peptide aus mindestens fünf Aminosäuren, darunter mindestens ein Prolin (Pro) und mindestens zwei Phenylalanine (Phe), als aktive Komponenten in einer nicht-therapeutischen kosmetischen Behandlung der Haut und/oder ihrer Anhangsgebilde auf Peptidbasis. Diese zyklischen Peptide sollen die Dichte und Dicke der Dermis verbessern, das Auftreten von Falten verringern, die Flexibilität der Epidermis erhalten, die Porengröße der Haut verringern, das unangenehme Gefühl von empfindlicher Haut und von Rötungen verringern, und die Verschlechterung der Qualität des Stützgewebes und die vorzeitigen Alterung der Haut einschränken.

Zyklische Peptidderivate mit beispielsweise fünf bis sieben Aminosäuren oder Aminosäurederivaten und einem Thiazolidin-, Oxazolidin- oder Imidazolidin-Ring werden in der Patentschrift EP 3 072 899 beschrieben. Insbesondere beschreibt die EP 3 072 899 die Auffindung und Isolierung des bakteriziden Peptidantiinfektivum Lugdunin, umfassend sechs Aminosäuren und einen Thiazolidinring, in *S. lugdunensis.* Bitschar et al., Nat. Commun. 2019 06 21;10(1):2730 beschreibt außerdem, dass Lugdunin einen möglicherweise dreistufigen Schutz gegen *S. aureus* in der Haut eines Wirts bereitstellen könnte. Erstens kann es als Bakterizid wirken, bspw. in Synergie mit den menschlichen antimikrobiellen Peptiden DCD-1(L) und LL-37, um *S. aureus* abzutöten; zweitens kann Lugdunin die durch Kommensalen induzierte angeborene Immunantwort in primären menschliche Keratinozyten verstärken; und drittens könnte möglicherweise eine Lugdunin-induzierte Rekrutierung phagozytotischer Zellen zu einer effektiven Abtötung von *S. aureus* beitragen.

Es ist eine Aufgabe der vorliegenden Erfindung ein neues, vereinfachtes Verfahren zur Verfügung zu stellen, welches erlaubt neue Verbindungen einfach und mit hoher Reinheit durch chemische Festphasensynthese herzustellen.

In Anbetracht des oben Beschriebenen ist es zudem eine Aufgabe der vorliegenden Erfindung, diese neuen Verbindungen bei der nicht-therapeutischen Behandlung kosmetischer oder dermatologischer Phänomene zur Verfügung zu stellen.

### Zusammenfassung der Erfindung

Die vorliegenden Erfinder haben nun neue zyklische Verbindungen aufgefunden die sich überraschenderweise aufgrund ihrer vorteilhaften Eigenschaften für die Verwendung in kosmetischen Zubereitungen eignen. Die erfindungsgemäßen neuen zyklischen Verbindungen vereinen eine Reihe von vorteilhaften Eigenschaften.

Insbesondere weisen die erfindungsgemäßen zyklischen Verbindungen beim kosmetischen Einsatz vorzugsweise eine antimikrobielle, insbesondere antivirale, antibakterielle und/oder antimykotische, bevorzugt antivirale und/oder (nicht-selektive) antibakterielle, Wirksamkeit auf. Beispielsweise wurde überraschend gefunden, dass die erfindungsgemäßen zyklischen Verbindungen eine nicht-selektive antimikrobielle, insbesondere nicht-selektive antibakterielle Wirksamkeit, vorzugsweise gegen grampositive Bakterien, aufweisen. Bevorzugt weisen die erfindungsgemäßen zyklischen Verbindungen gegen gramnegative Bakterien dagegen keine oder nur eine minimale antibakterielle Wirksamkeit auf.

Weiterhin haben die erfindungsgemäßen neuen zyklischen Verbindungen eine unterstützende Wirkung auf die angeborene Immunantwort. Beispielsweise sind die neuen erfindungsgemäßen zyklischen Verbindungen überraschenderweise besonders wirksam, im kosmetischen Einsatz, die angeborene Immunantwort der Haut gegen Infektionen mit Mikroorganismen, wie Bakterien, zu unterstützen. Die erfindungsgemäßen zyklischen Verbindungen eignen sich dadurch beispielsweise vorzüglich für den Einsatz als kosmetische Wirkstoffe für den nicht-therapeutischen Einsatz in kosmetischen Zubereitungen, insbesondere für die topische Anwendung.

Außerdem wurde überraschend gefunden, dass die nicht-selektiv antibakteriell wirksamen erfindungsgemäßen zyklischen Verbindungen bei deren Verwendung zu keiner, bzw. nur in äußerst geringem Maße zu einer, Resistenzbildung führen; die erfindungsgemäßen zyklischen Verbindungen insbesondere beispielsweise keine relevante Resistenzbildung aufweisen, die aus dem Stand der Technik bekannte Verbindung Lugdunin dagegen schon und somit ein signifikanter Unterschied in Bezug auf die Resistenzbildung besteht. Überraschenderweise wurde gefunden, dass die erfindungsgemäßen zyklischen Verbindungen sich bezüglich des Wirkungsgeschehens beispielsweise von der bekannten Verbindung Lugdunin unterscheiden.

Die erfindungsgemäßen neuen zyklischen Verbindungen zeichnen sich durch eine ausgezeichnete Löslichkeit und Stabilität in üblichen Lösungsmitteln und Trägern aus, was die Verwendung insbesondere in kosmetischen Zubereitungen begünstigt. Auch werden die erfindungsgemäßen neuen zyklischen Verbindungen überraschenderweise nicht oder nicht ohne Weiteres durch herkömmliche Proteasen abgebaut, was den Einsatz auf der Haut begünstigt.

Die vorliegenden Erfinder haben eine neue effiziente chemische Syntheseroute unter Verwendung von Festphasen-Peptidsynthese-Harz aufgefunden, die es erlaubt die neuen zyklischen Verbindungen mit den genannten vorteilhaften Eigenschaften einfach und in bislang nicht möglichem hohem Reinheitsgrad (>90% Reinheit nach optimiertem wässrigen Waschen, aber noch vor spezifischer Aufreinigung beispielsweise durch präparative Chromatographie) und hoher Ausbeute zu synthetisieren. Diese neu geschaffene Syntheseroute erlaubt die problemlose Herstellung der erfindungsgemäßen neuen zyklischen Verbindungen in kommerziell relevanten Menden, beziehungsweise die einfache Hochskalierung des Syntheseverfahrens im industriellen Rahmen zur Steigerung der Syntheseleistung.

Die erfindungsgemäße neue Syntheseroute basiert auf der für Schritt (i) oder (ii) notwendigen Herstellung bisher unbekannter Alkyl- Aryl- oder Alkinyl-Heterozyklen-Aminosäurederivate, den neuen Thiazolidin- und Oxazolidinbausteinen. Die neuen Thiazolidin- und Oxazolidinbausteine zeichnen sich insbesondere durch eine hervorragende Löslichkeit in organischen Lösungsmitteln und damit verbundene überlegene Kupplungseigenschaften in der Festphasensynthese aus. Weiterhin haben die vorliegenden Erfinder ein Syntheseverfahren für die neuen Thiazolidin- und Oxazolidinbausteine entwickelt.

Die Synthese der Thiazolidin- und Oxazolidinbausteine kann damit erstmals vorteilhaft in die effiziente Methode der Festphasen-Peptidsynthese eingebracht werden. Dies erlaubt eine einfache, kontrollierte und äußerst effiziente Synthese der neuen zyklischen Verbindungen, durch Festphasen-Peptidsynthese über eine lineare Vorstufe (d.h. über ein lineares an die feste Phase gebundenes Peptidderivat), und ein einfacheres Hochskalieren. Nach etabliertem Prinzip kann die lineare Vorstufe abschließend im letzten Schritt zur zyklischen Verbindung unter optimierten Bedingungen zyklisiert und in optimierten Waschschritten mit hohem Reinheitsgrad (>90% Reinheit) isoliert werden.

Somit stellt die vorliegende Erfindung zur Lösung der obengenannten Aufgaben neue zyklische Verbindungen sowie kosmetische, insbesondere topische, Zubereitungen enthaltend die neuen zyklischen Verbindungen als kosmetische Wirkstoffe, zur Verfügung.

Weiterhin stellt die vorliegende Erfindung ein neues chemisches Syntheseverfahren in Form einer Festphasenpeptidsynthese bereit, das es erlaubt die erfindungsgemäßen neuen zyklischen Verbindungen einfach und in hoher Reinheit herzustellen. In Verbindung mit diesem neuen Syntheseverfahren, werden außerdem neue Verbindungen in Form von Thiazolidin- und Oxazolidin-Bausteinen bereitgestellt, die, über einen neuen Syntheseweg, als Ausgangspunkt der Synthese der neuen zyklischen Verbindungen direkt in das neue Festphasenpeptidsyntheseverfahrens vorteilhaft eingebunden sind. Die vorliegende Erfindung betrifft die in den folgenden Punkten 1 bis 13 definierten Gegenstände:
[1] Zyklische Verbindung der Formel (I): 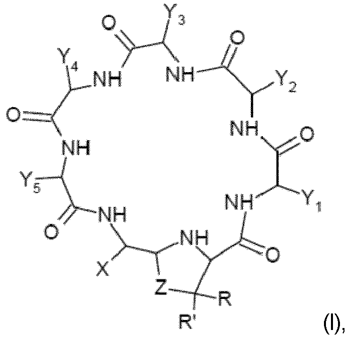 wobei:
   - X und Y₁ bis Y₅ jeweils ausgewählt sind aus der Gruppe bestehend aus: Methyl, Ethyl, *n*-Propyl, 2-Propenyl (H₂C=CH-CH₂-), 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl (HC=C-CH₂-), 1*H*-Indol-3-ylmethyl ( 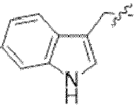 ), 1*N*-Methyl-1*H*-indol-3-ylmethyl ( 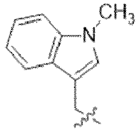 ), 3-Benzothienylmethyl ( 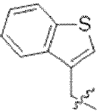 ), 1-Naphtylmethyl ( 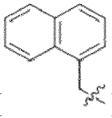 ), 9Anthracenylmethyl ( 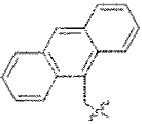 ) und Pyrenylmethyl;
   - Z gleich O oder S ist;
   - R und R' jeweils ausgewählt sind aus der Gruppe bestehend aus H, Methyl, Ethyl, *n-*Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
   - die C-Atome, die direkt mit den Substituenten Y₁, Y₂, Y₃, Y₄, Y₅ und X verbunden sind, in dieser Reihenfolge, eine jeweils alternierende absolute stereochemische Konfiguration aufweisen; und
   - das C-Atom, welches mit dem Substituenten X verbunden ist, und das C-Atom in Position 4 des Thiazolidin- oder Oxazolidin-Rings, die gleiche absolute stereochemische Konfiguration aufweisen, wenn Z gleich O ist, und eine entgegengesetzte absolute stereochemische Konfiguration aufweisen, wenn Z gleich S ist;
      unter der Bedingung, dass, wenn X, Y₁, Y₄ und Y₅ gleich 1-Methylethyl sind, Y₂ gleich 1*H*-Indol-3-ylmethyl ist, Y₃ gleich 2-Methylpropyl ist, R gleich H ist und R' gleich Methyl ist, Z nicht O ist;
      und die Salze hiervon, die Solvate hiervon und die Solvate der Salze hiervon.
[2] Zyklische Verbindung gemäß [1], wobei
   - R und R' beide Methyl sind; Y₂ gleich 1*H*-Indol-3-ylmethyl oder Pyrenylmethyl ist, falls Y₅ gleich 1-Methylethyl ist, oder Y₂ gleich 1-Methylethyl ist, falls Y₅ gleich 1*H-*Indol-3-ylmethyl oder Pyrenylmethyl ist; und X gleich 1-Methylethyl ist;
   - R gleich H ist; R' gleich Methyl, Ethyl, *n*-Propyl, 1-Methylethyl, Benzyl oder Propargyl ist; Y₂ gleich 1*H*-Indol-3-ylmethyl oder Pyrenylmethyl ist, falls Y₅ gleich 1-Methylethyl ist, oder Y₂ gleich 1-Methylethyl ist, falls Y₅ gleich 1*H*-Indol-3-ylmethyl oder Pyrenylmethyl ist; und X gleich 1-Methylethyl ist;
   - R gleich H ist; R' gleich Methyl, *n*-Propyl, 1-Methylethyl, Benzyl oder Propargyl ist; Y₂ gleich 1*H*-Indol-3-ylmethyl oder Pyrenylmethyl ist, falls Y₅ gleich 1-Methylethyl ist, oder Y₂ gleich 1-Methylethyl ist, falls Y₅ gleich 1*H*-Indol-3-ylmethyl oder Pyrenylmethyl ist; und X gleich 1-Methylethyl oder 2-Methylpropyl ist; oder
   - R und R' beide 1-Methylethyl sind; und Y₂ gleich 1*H*-Indol-3-ylmethyl ist; und/oder
   - gekennzeichnet durch eine der folgenden Formeln: 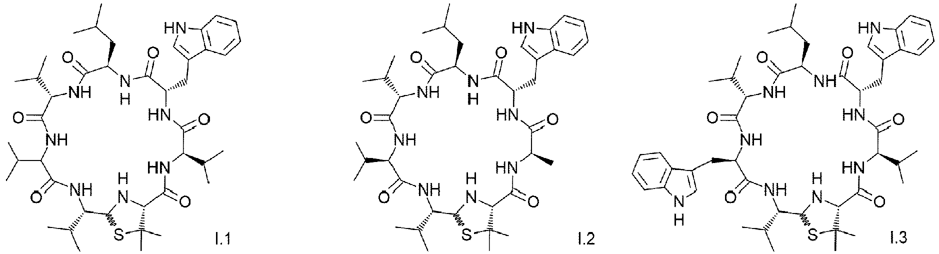 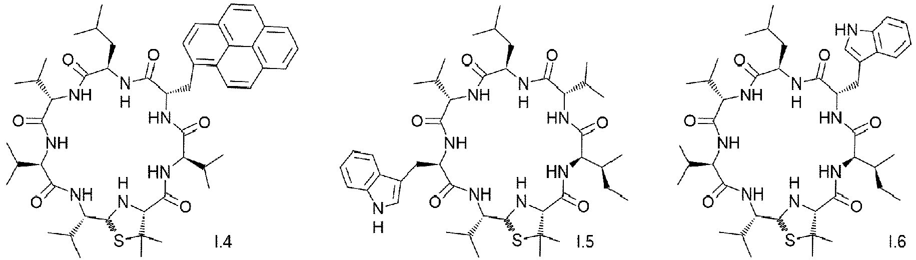 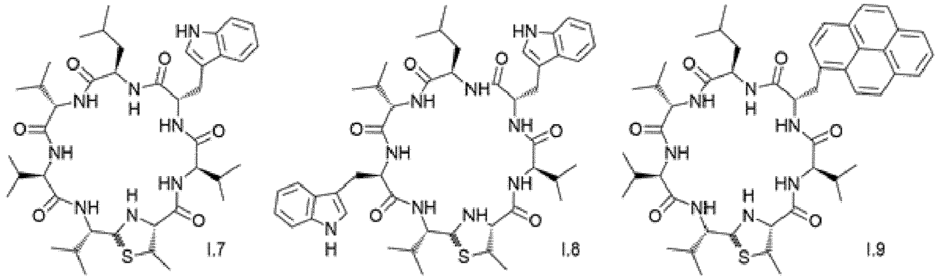 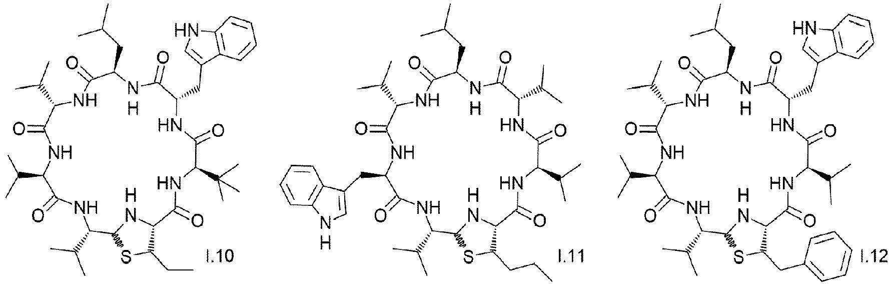 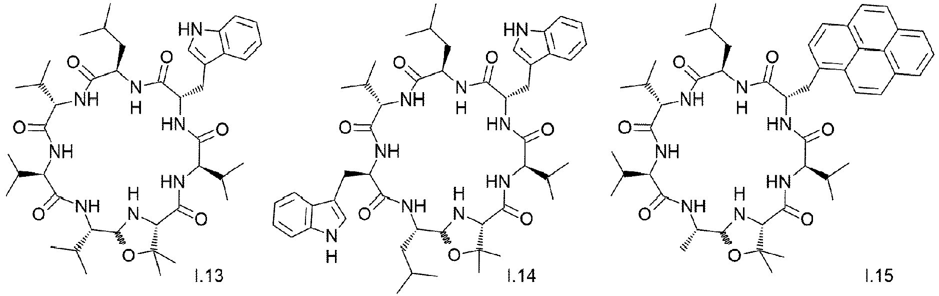 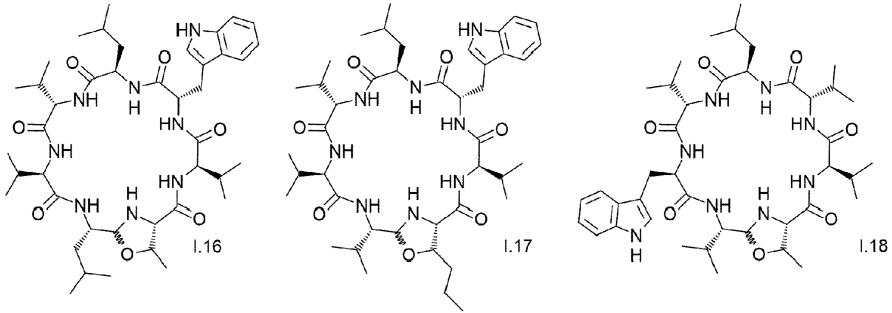
[3] Zyklische Verbindung gemäß [1] oder [2] mit antimikrobieller, insbesondere antiviraler oder nicht-selektiver antibakterieller (vorzugsweise gegen grampositive Bakterien, jedoch bevorzugt nicht oder nur minimal gegen gramnegative Bakterien), Wirksamkeit und/oder unterstützender Wirkung auf die angeborene Immunantwort, insbesondere der Haut.
[4] Thiazolidin- oder Oxazolidin-Baustein der Formel (II): 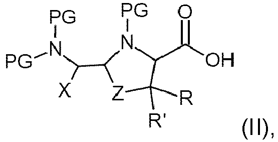 wobei:
   - Z gleich O oder S ist;
   - R und R' jeweils ausgewählt sind aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
   - PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
   - X ausgewählt ist aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl, Propargyl, 1*H*-Indol-3-ylmethyl, 1*N-*Methyl-1*H*-indol-3-ylmethyl, 3-Benzothienylmethyl, 1-Naphtylmethyl, 9-Anthracenylmethyl und Pyrenylmethyl;
   - das C-Atom, welches mit dem Substituenten X verbunden ist, und das C-Atom in Position 4 des Thiazolidin- oder Oxazolidin-Rings, die gleiche absolute stereochemische Konfiguration aufweisen, wenn Z gleich O ist, und eine entgegengesetzte absolute stereochemische Konfiguration aufweisen, wenn Z gleich S ist;
   unter der Bedingung, dass, wenn Z gleich S ist und X gleich Methyl, Benzyl oder 1*H-*Indol-3-ylmethyl ist, R nicht Methyl ist;
   und die Salze hiervon, die Solvate hiervon und die Solvate der Salze hiervon.
[5] Thiazolidin- oder Oxazolidin-Baustein gemäß [4], gekennzeichnet durch eine der folgenden Formeln: 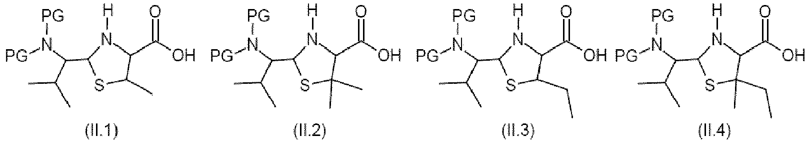 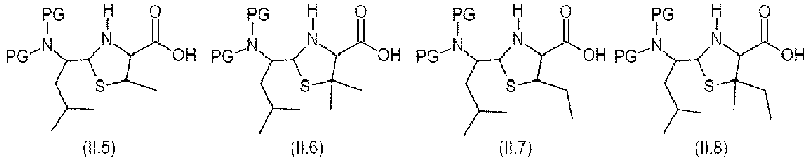 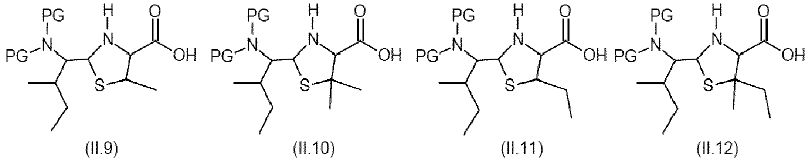 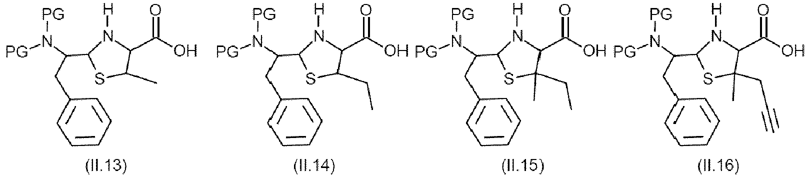 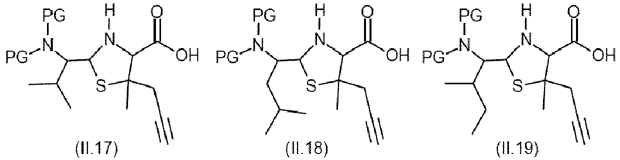 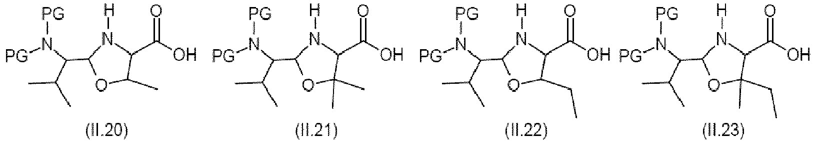 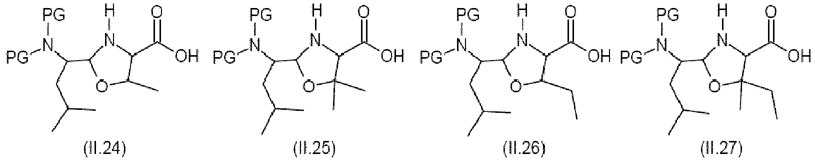 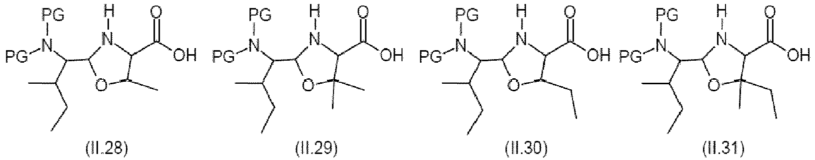 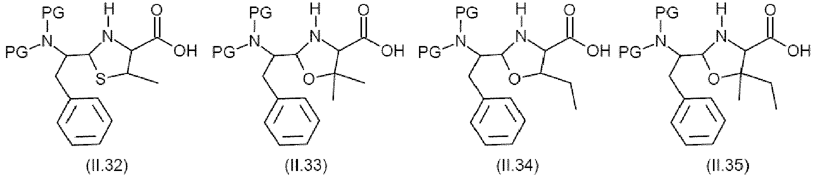 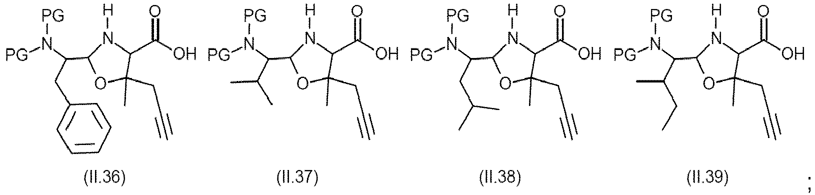 wobei vorzugsweise zwei Substituenten PG für ein H stehen und ein Substituent PG (vorzugsweise in der terminalen Aminogruppe) für eine Schutzgruppe steht.
[6] Verfahren zur Synthese eines Thiazolidin- oder Oxazolidin-Bausteins nach [4] oder [5], umfassend die folgenden Schritte (a) bis (d):
   (a) Bereitstellen eines geschützten Aminosäurederivats der Formel (III): 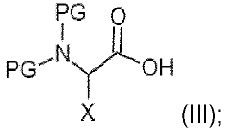
   (b) Reduzieren des Aminosäurederivats (III) aus Schritt (a) zum Alkohol der Formel (IV) durch
      (b1) in einem ersten Aktivierungsschritt, Aktivieren des Aminosäurederivats der Formel (III), mit einem Aktivierungsreagenz, in einem inerten Lösungsmittel; und
      (b2) in einem zweiten Schritt, Reduzieren des Aktivierungsprodukts aus Schritt (b1), zu einer Verbindung der Formel (IV): 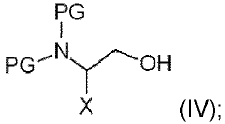
   (c) Oxidieren der Verbindung (IV) aus Schritt (b), mit einem Oxidationsmittel, in einem aprotischen Lösungsmittel, und gegebenenfalls einem Wasserzusatz, zum entsprechenden Aldehyd der Formel (V): 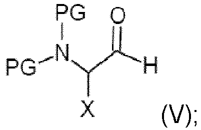
   (d) Umsetzen (Kondensation) der Verbindung (V) aus Schritt (c), vorzugsweise in einem Lösungsmittelgemisch aus Wasser und einem polar-protischen Lösungsmittel, in einem Verhältnis Wasser: polar-protisches Lösungsmittel von ≤ 1 : 1 (v/v) und bei einer Reaktionstemperatur von mindestens 30 °C, mit einer Verbindung der Formel (VI): 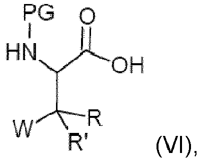 um einen Thiazolidin- oder Oxazolidin-Baustein gemäß [4] oder [5] zu erhalten; wobei:
      - W gleich SH oder OH ist;
      - R und R' jeweils ausgewählt sind aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
      - PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
      - X ausgewählt ist aus der Gruppe bestehend aus: Methyl, Ethyl, n-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl;
   unter der Bedingung, dass, wenn Z gleich S ist und X gleich Methyl, Benzyl oder 1*H-*Indol-3-ylmethyl ist, R nicht Methyl ist.
[7] Verfahren zur Synthese eines Thiazolidin- oder Oxazolidin-Bausteins nach [6]
   - wobei in Schritt (b1) das Aktivierungsreagenz ausgewählt ist aus Thionylchlorid, Ethylenchloroformiat, Oxalylchlorid, N,O-Dimethylhydroxylamin, oder 1,1'-Carbonyldiimidazol (CDI), bevorzugt CDI; ein inertes zyklisches Etherlösungsmittel wie 1,4-Dioxan, Cyclopentylmethylether, 2-Methyltetrahydrofuran (2-Me-THF), Tetrahydrofuran (THF), bevorzugt THF, verwendet wird; und/oder die Aktivierung bei 15 bis 25 °C für mindestens 5 min, durchgeführt wird;
   - wobei in Schritt (b2) das Aktivierungsprodukt unter Verwendung von Pd/C/H₂, Pd/C/Triethylsilan, NiCl₂/NaBH₄, Lithiumaluminumhydrid (LiAlH₄), Diisobutylaluminiumhydrid (DIBAL-H), Natriumcyanoborhydrid (NaBH₃CN) oder Natriumborhydrid (NaBH₄), bevorzugt NaBH₃CN oder NaBH₄, bei 0 °C für mindestens 15 min, reduziert wird;
   - wobei in Schritt (c) das Oxidationsmittel Oxalylchlorid/DMSO/NEt₃ oder DMP, mehr bevorzugt DMP, ist; das aprotische Lösungsmittel Ethylacetat, Aceton, Toluol, THF, Chloroform, oder Dichlormethan (DCM), bevorzugt Toluol, THF, Chloroform oder DCM, bevorzugt DCM, ist; das Oxidationsmittel zwischen 1.0 bis 2.0 Äquiv.; und/oder zur Reaktion Wasser, bevorzugt zwischen 1.0 bis 1.5 Äquiv. zugegeben wird; und/oder
   - wobei in Schritt (d) das Verhältnis von Wasser zu polar-protischem Lösungsmittel ≤ 1 : 1 (v/v) beträgt; das polar-protische Lösungsmittel Ameisensäure, Essigsäure, Ethanol, Isopropanol oder Methanol, bevorzugt Ethanol, Isopropanol oder Methanol, mehr bevorzugt Methanol, ist; und/oder die Reaktion bei mindestens 50 °C, bevorzugt zwischen 55 °C bis 75 °C durchgeführt wird.
[8] Verfahren zur Festphasen-Peptidsynthese einer zyklischen Verbindung gemäß einem der [1] bis [3], umfassend die folgenden Schritte (i) bis (iv):
   (i) Bereitstellen eines, über seine terminale Carboxylgruppe an eine feste Phase gebundenen,
      - Aminosäurederivats der Formel (VII): 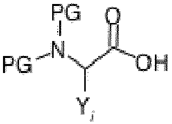 (VII), wobei i gleich 1 bis 5 ist;
      - linearen Peptids bestehend aus zwei, drei, vier, oder fünf Aminosäurederivaten der Formel (VII); oder
      - Thiazolidin- oder Oxazolidin-Bausteins gemäß [4] oder [5];
      wobei die terminale Aminogruppe durch mindestens eine Schutzgruppe geschützt ist;
   (ii) ausgehend von dem Aminosäurederivat der Formel (VII), dem Peptid, bzw. dem Thiazolidin- oder Oxazolidin-Baustein aus Schritt (i), durch Durchführen einer oder mehrerer konsekutiver Kupplungsreaktionen, unter Anfügen
      - eines oder mehrerer Aminosäurederivate der Formel (VII),
      - eines oder mehrerer linearer Peptide bestehend aus zwei bis fünf Aminosäurederivaten der Formel (VII), oder
      - eines Thiazolidin- oder Oxazolidin-Bausteins gemäß [4] oder [5],
      wobei die terminale Aminogruppe durch mindestens eine Schutzgruppe geschützt ist,
      schrittweise Festphasensynthese einer linearen, an die feste Phase gebundene, Verbindung, umfassend fünf Aminosäurederivate der Formel (VII), wobei i gleich 1 bis 5 ist, und ein Thiazolidin- oder Oxazolidin-Baustein nach [4] oder [5];
   (iii) Entfernen der Schutzgruppen des linearen, an die feste Phase gebundenen, Produkts aus Schritt (ii), und Abspalten von der festen Phase unter Bildung einer linearen, nicht an die feste Phase gebundenen, Verbindung;
   (iv) Makrozyklisierung der linearen, nicht an die feste Phase gebundenen, Verbindung aus Schritt (iii), vorzugsweise durch Makrolaktamisierung, wodurch die zyklische Verbindung gemäß einem der [1] bis [3] erhalten wird;
      wobei:
      - X und Y₁ bis Y₅ jeweils ausgewählt sind aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl;
      - Z gleich O oder S ist;
      - PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
      - R und R' jeweils ausgewählt sind aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
      - unter der Bedingung, dass, wenn X, Y₁, Y₄ und Y₅ gleich 1-Methylethyl sind, Y₂ gleich 1*H*-Indol-3-ylmethyl ist, Y₃ gleich 2-Methylpropyl ist, R gleich H ist und R' gleich Methyl ist, Z nicht O ist.
[9] Verfahren gemäß [8], wobei der in Schritt (i) oder Schritt (ii) eingesetzte Thiazolidin- oder Oxazolidin-Baustein nach dem Verfahren gemäß [6] oder [7] hergestellt wird.
[10] Verfahren gemäß [8] oder [9], wobei
   - in Schritt (i) ein lineares Peptid bestehend aus zwei bis fünf, vorzugsweise fünf, Aminosäurederivaten der Formel (VII), wobei die terminale Aminogruppe durch mindestens eine Schutzgruppe geschützt ist, bereitgestellt wird;
   - in Schritt (ii) die anzufügenden Aminosäurederivate der Formel (VII) oder Peptide, oder der anzufügende Thiazolidin- oder Oxazolidin-Baustein nicht an eine feste Phase gebunden sind bzw. ist; und in jeder der einen oder mehreren Kupplungsreaktionen, jeweils zunächst die mindestens eine Schutzgruppe von dem Aminosäurederivat der Formel (VII), dem Peptid, bzw. dem Thiazolidin- oder Oxazolidin-Baustein aus Schritt (i), entfernt wird, und dann die Kupplung mit dem, nicht an eine feste Phase gebunden, Aminosäurederivat der Formel (VII), dem Peptid, bzw. dem Thiazolidin- oder Oxazolidin-Baustein durchgeführt wird;
   - in Schritt (iii) das Entschützen und Abspalten vom festen Träger durch eine erste Behandlung mit 0,5-4 % (v/v) DBU (1,8-Diazabicyclo[5.4.0]undec-7-en) und 5-15 % (v/v) Morpholin in DMF und eine zweite Behandlung mit Trifluoressigsäure (TFA), Triisopropylsilan (TIPS) und Wasser, vorzugsweise in einem Verhältnis von 90-92,5 : 2,5-5 : 5 (v/v/v), durchgeführt wird; und/oder
   - in Schritt (iv) eine Makrolaktamisierung durchgeführt wird, wobei die lineare, an die feste Phase gebundene, Verbindung aus Schritt (iii) bei 15 bis 25 °C mit HATU, DIPEA und HOAt, unter Verwendung eines polaren aprotischen Lösungsmittels, vorzugsweise DMF, zyklisiert wird.
[11] Zyklische Verbindung gemäß einem der [1] bis [3], Thiazolidin- oder Oxazolidin-Baustein gemäß [4], Verfahren gemäß [6] oder [7], und/oder Verfahren gemäß einem der [8] bis [10], wobei:
   - X ausgewählt ist aus der Gruppe bestehend aus: 1-Methylethyl, 2-Methylpropyl, 1-Methylpropyl, Benzyl, Propargyl, 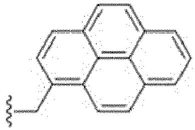 (1-Pyrenylmethyl) und 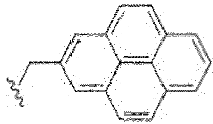 (2-Pyrenylmethyl);
   - Y₁ bis Y₅ jeweils ausgewählt sind aus der Gruppe bestehend aus: Methyl, 1-Methylethyl, 2-Methylpropyl, 1-Methylpropyl, Benzyl, 1*H*-Indol-3-yl-methyl, Propargyl, 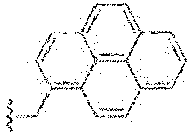 (1-Pyrenylmethyl) und 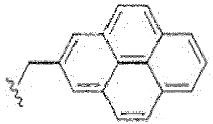 (2-Pyrenylmethyl);
   - W gleich OH oder SH ist;
   - Z gleich O oder S ist; und/oder
   - R und R' jeweils ausgewählt sind aus der Gruppe bestehend aus H, Methyl, Ethyl, und Propargyl.
[12] Kosmetische Zubereitung, enthaltend
   (a) eine oder mehrere zyklische Verbindungen nach einem der [1] bis [3]; und
   (b) einen oder mehrere kosmetisch akzeptable Träger.
[13] Kosmetische Zubereitung nach [12], wobei die kosmetische Zubereitung in Form einer dermatologischen Zubereitung zur topischen Anwendung vorliegt; und/oder wobei die eine oder mehreren zyklischen Verbindungen eine antimikrobielle, vorzugsweise antivirale, antibakterielle und/oder antimykotische, insbesondere antivirale und/oder nicht-selektive antibakterielle (vorzugsweise gegen grampositive Bakterien, jedoch bevorzugt nicht oder nur minimal gegen gramnegative Bakterien), Wirksamkeit aufweisen und/oder eine unterstützende Wirkung auf die angeborene Immunantwort, insbesondere der Haut, haben.

### Kurze Beschreibung der Abbildungen

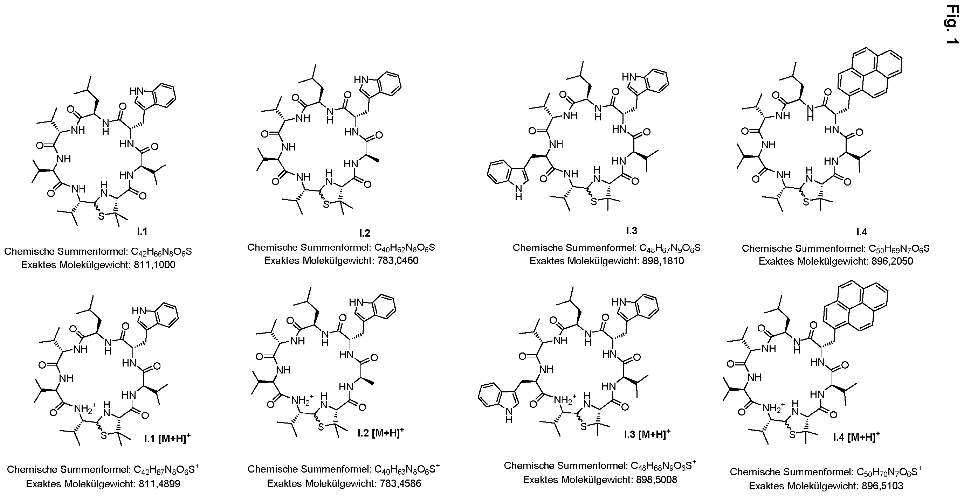

**Fig. 1****:** Strukturformeln der in den Beispielen verwendeten zyklischen Verbindungen, I.1 (ISW 1-0), I.2 (ISW 1-1), I.3 (ISW 1-2) und I.4 (ISW 1-4), einschließlich Molekülmasse.

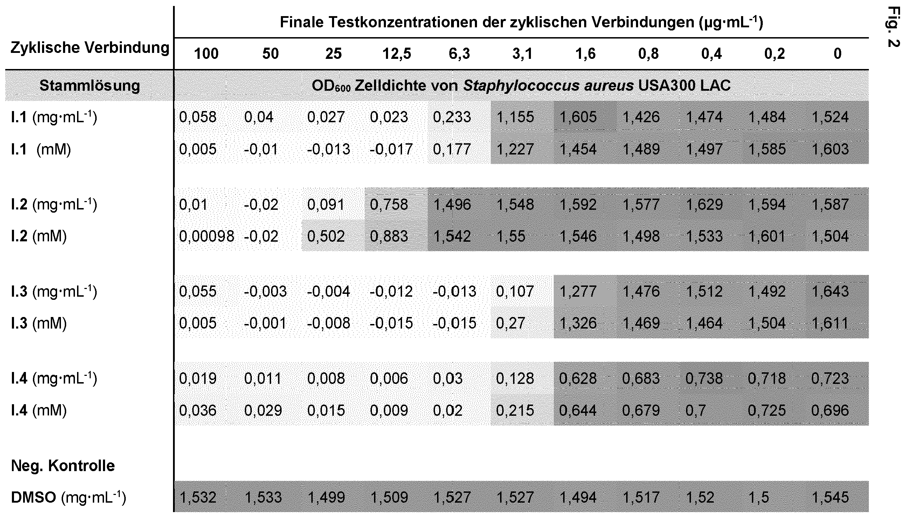

**Fig. 2****:** Bioassay mit *Staphylococcus aureus* USA300 LAC (37 °C, 160 rpm, 22 h) unter Verwendung der vier zyklischen Verbindungen I.1, I.2, I. 3 und I.4: Tabelle mit OD₆₀₀ Werten im Tabellenkörper, nach Wachstum in verschiedenen Konzentrationen der zyklischen Verbindungen; DMSO als Negativkontrolle; Farbkodierung: Minimale Hemmkonzentration (MHK; in Engl.: minimal inhibitory contentration, MIC) ; rot (bzw. grau): Wachstum; weiß: kein Wachstum.

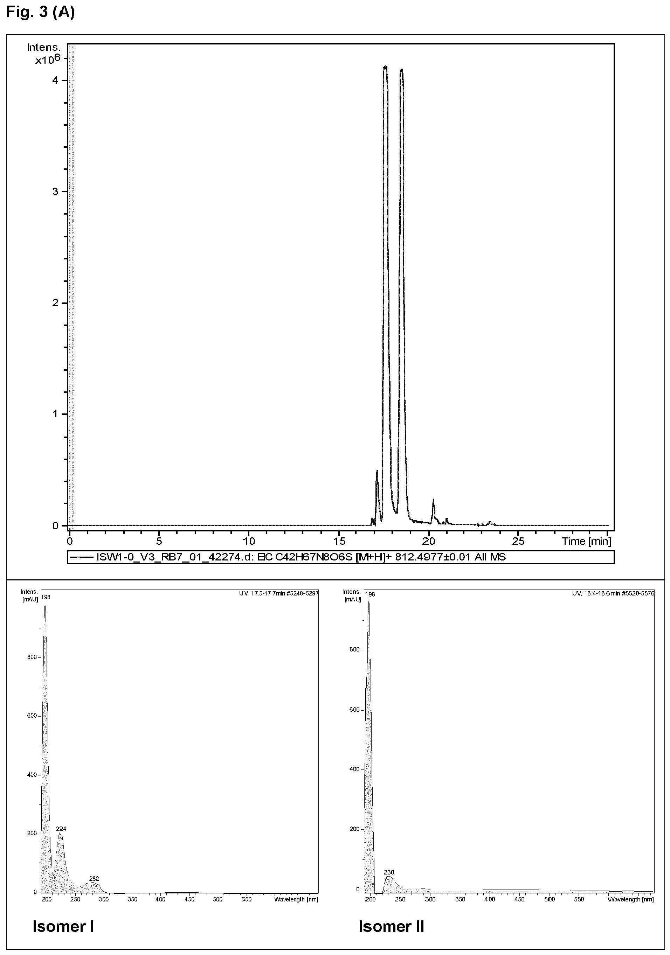

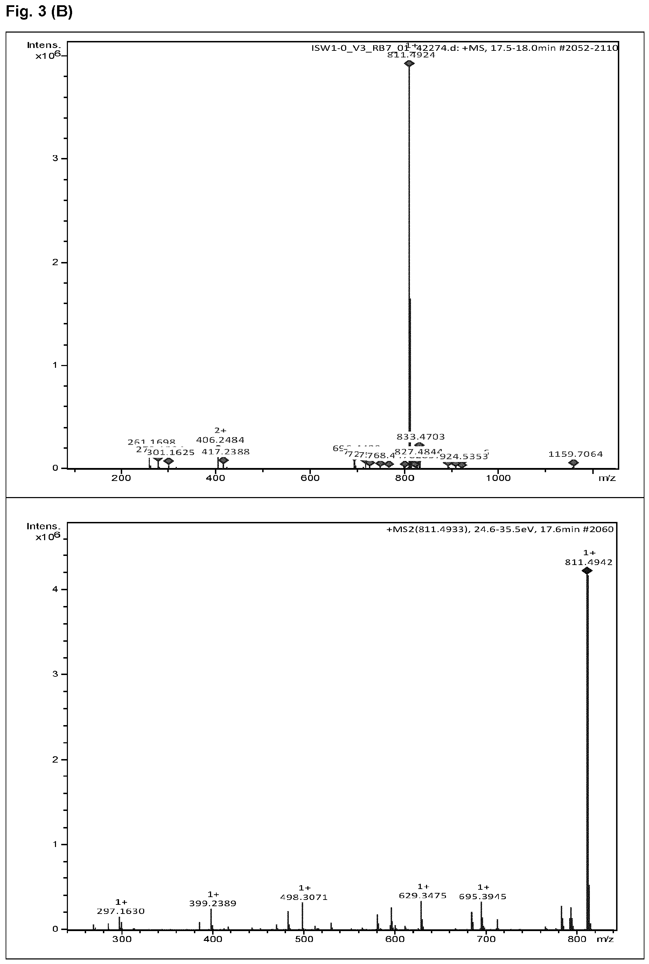

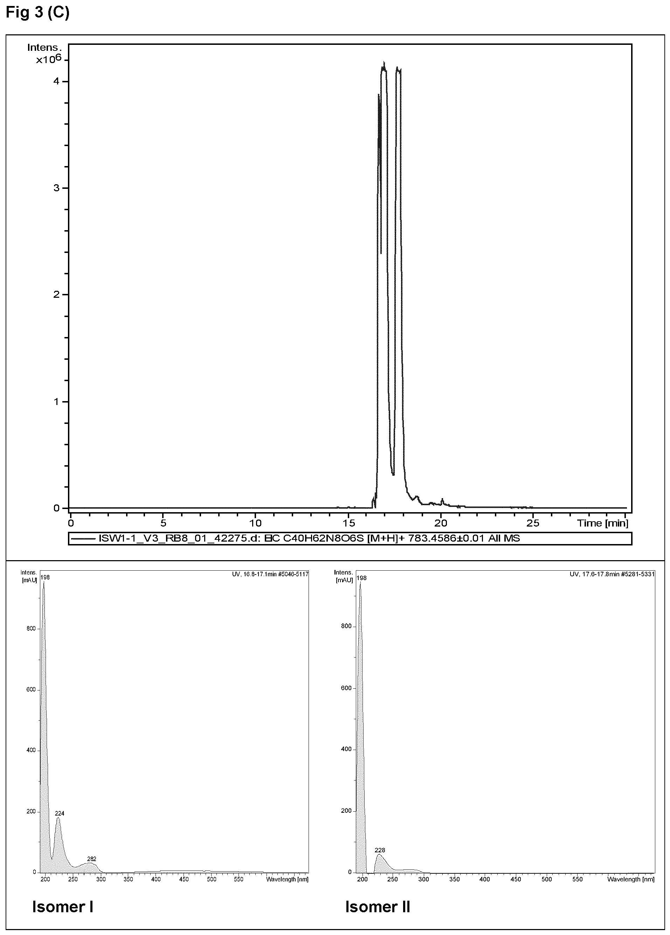

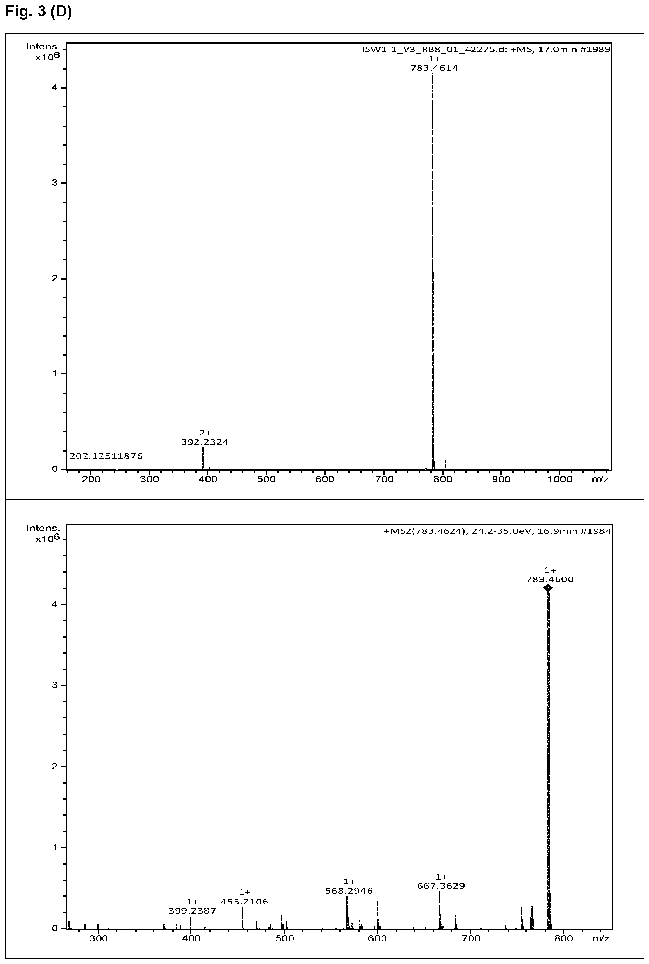

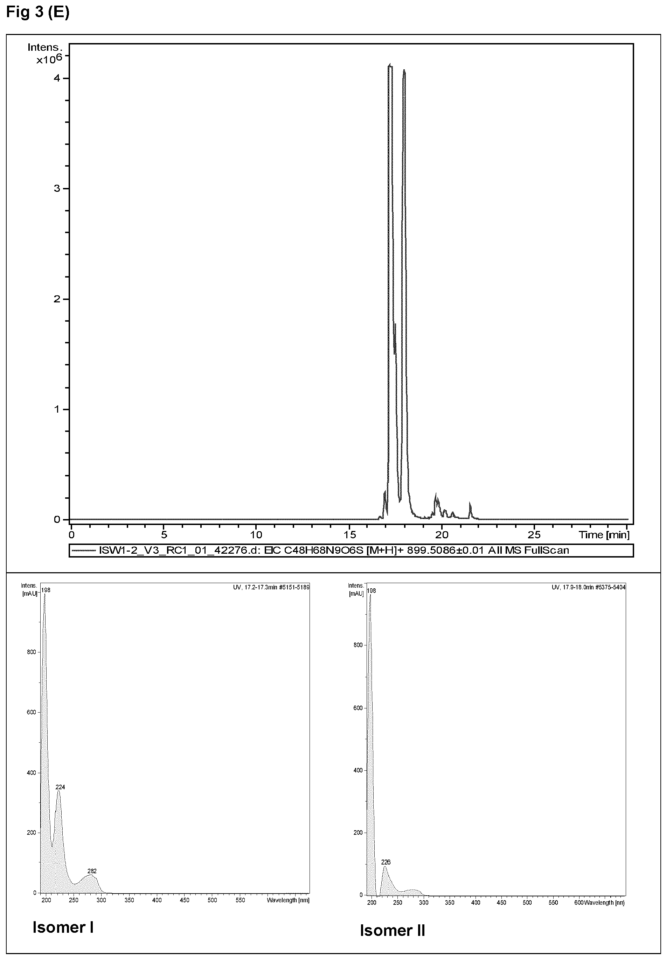

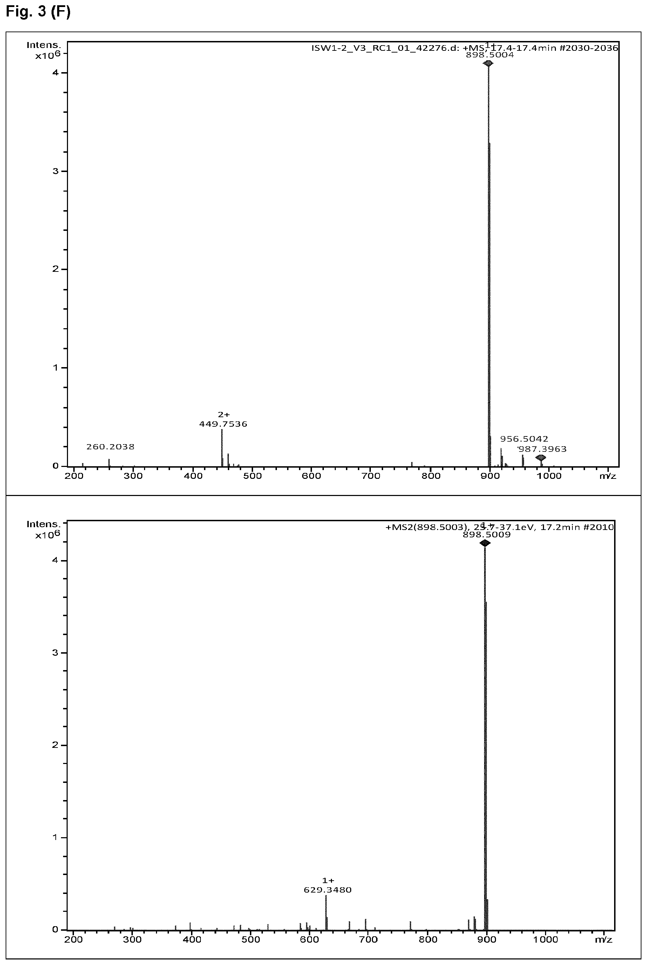

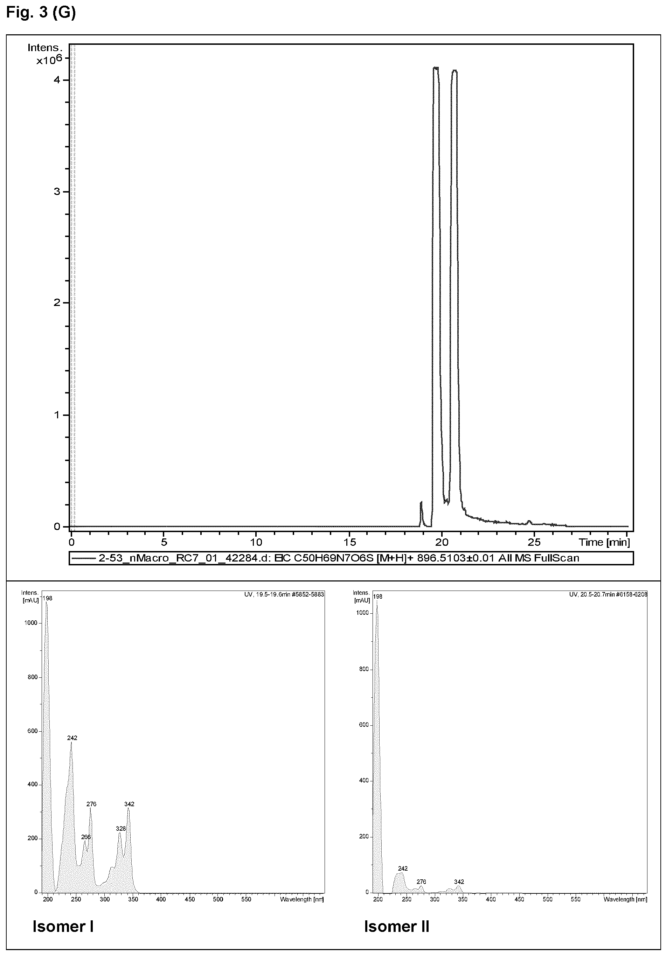

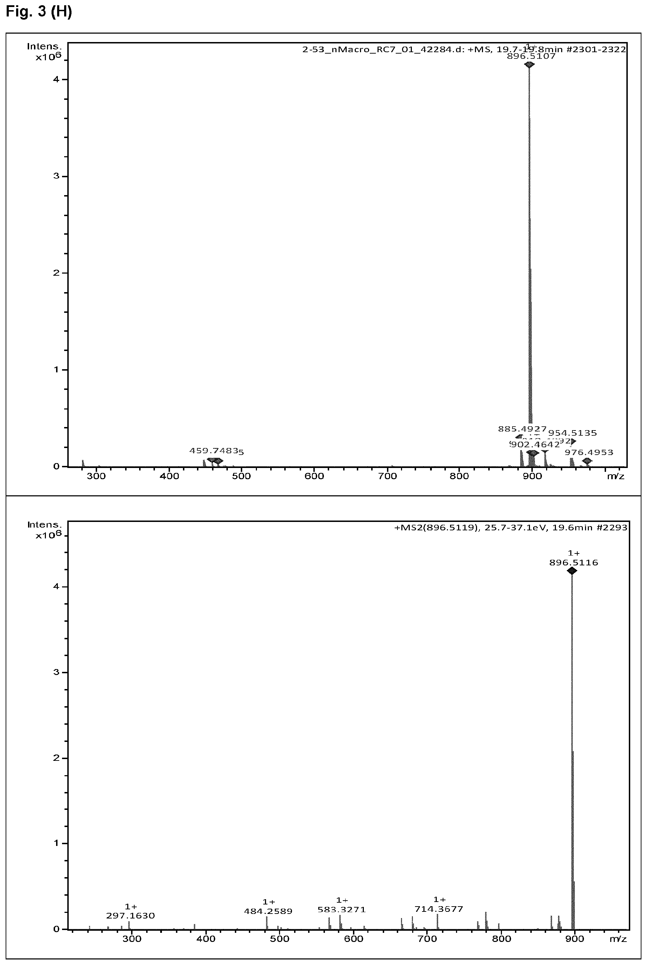

**Fig. 3****:** Spektren der HPLC-Massenspektrometrie (HPLC-MS) Analyse der zyklischen Verbindungen: **(A) & (B)** MS-Spektren der Verbindung I.1, **(C) & (D)** MS-Spektren der Verbindung I.2, **(E) & (F)** MS-Spektren der Verbindung I.3, und **(G) & (H)** MS-Spektren der Verbindung I.4; aufgenommen mit einem ESI-QTOF-Massenspektrometer (MaXis 4G, Bruker Daltonics GmbH).

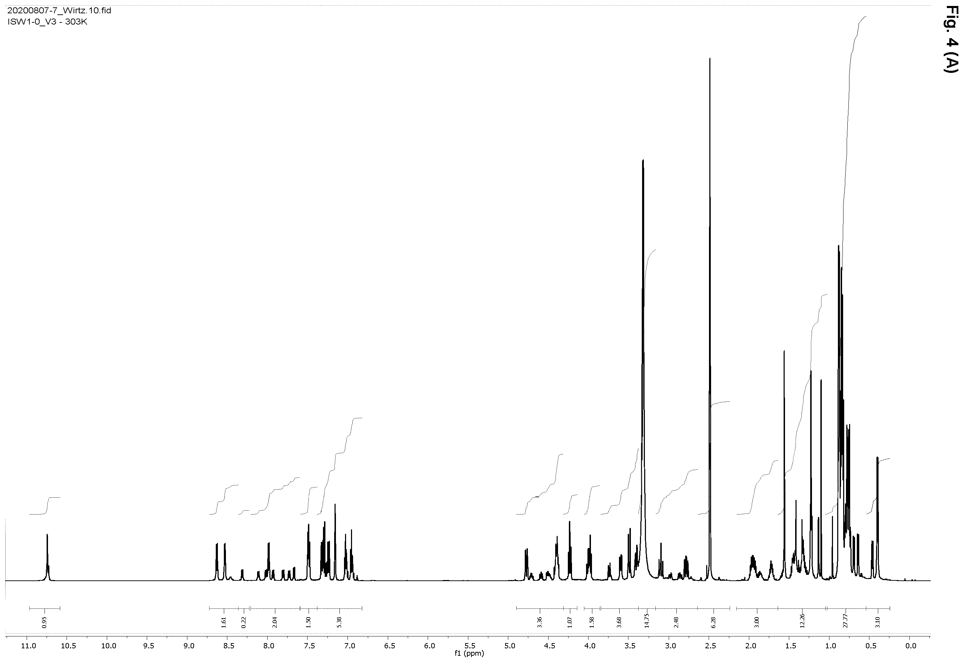

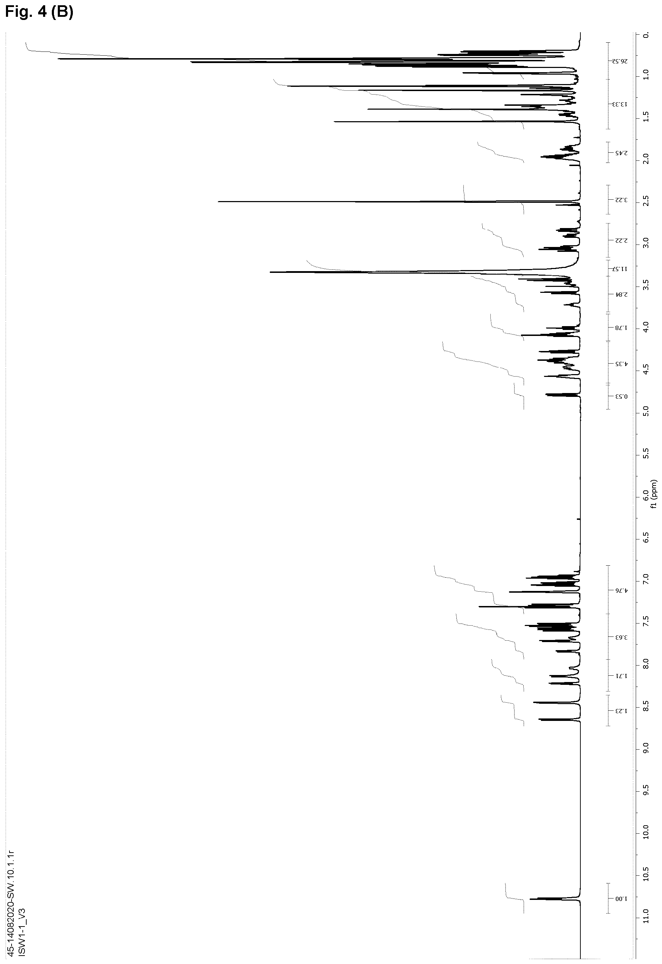

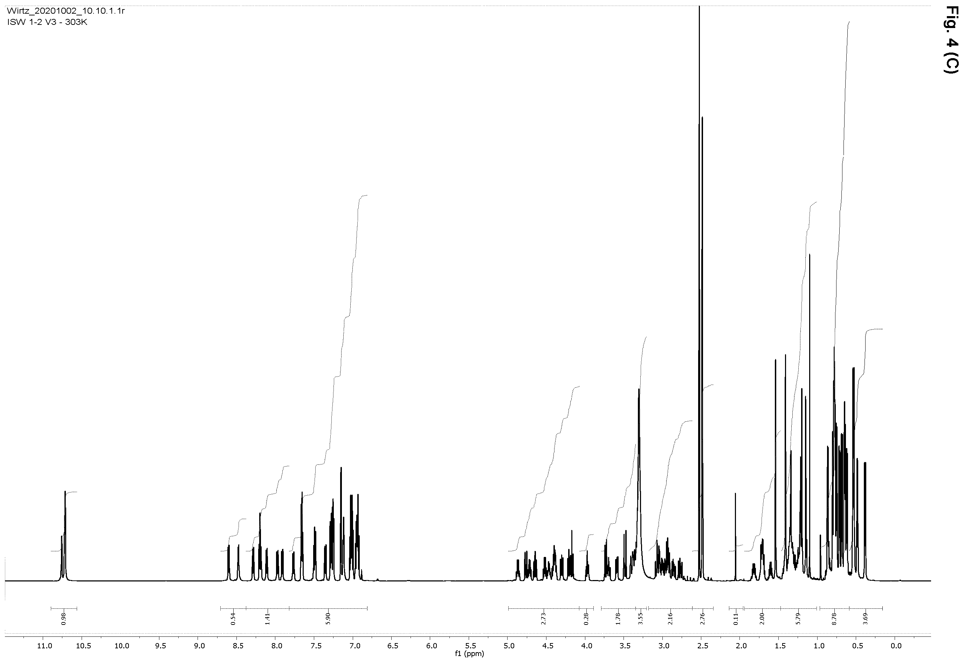

**Fig. 4****:** Spektren der ¹H-NMR-Analyse der zyklischen Verbindungen: **(A)** ¹H-NMR Spektrum der Verbindung **I.1** (600 MHz, DMSO-D₆, 303 K), **(B)** ¹H-NMR Spektrum der Verbindung **I.2** (700 MHz, DMSO-D₆, 303 K), **(C)** ¹H-NMR Spektrum der Verbindung **I.3** (600 MHz, DMSO-D₆, 303 K); aufgenommen mit einem Bruker AMX-600 NMR-Spektrometer oder einem Bruker Avancelll-700 NMR-Spektrometer (Bruker BioSpin GmbH).

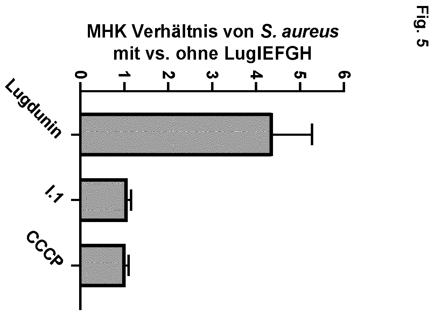

**Fig. 5****:** Verhältnis der minimalen Hemmkonzentration (MHK) der erfindungsgemäßen zyklischen Verbindung I.1, sowie der bekannten Verbindungen Lugdunin und CCCP (Carbonylcyanid-3-chrlophenylhydrazon) für einen rekombinanten *S. aureus* Stamm mit den Lugdunin Transporter-Genen LuglEFGH und den *S. aureus* N315 Wildtyp: MHK_{(*S. aureus* LugIEFGH}) / MHK_{(*S*. *aureus* Wt)}. Aus den Ergebnissen geht hervor, dass Lugdunin, jedoch weder I.1 noch CCCP, von den Transportern erkannt und transportiert wird.

### Detaillierte Beschreibung der Erfindung

### Zyklische Verbindung der Formel (I)

In einem ersten Aspekt ist die vorliegende Erfindung gerichtet auf eine neue zyklische Verbindung der Formel (I): 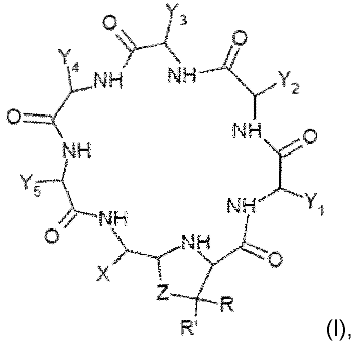 wobei:
- X und Y₁ bis Y₅ jeweils ausgewählt sind aus der Gruppe bestehend aus: Methyl, Ethyl, *n-*Propyl (mit der Formel CH₃-CH₂-CH₂-), 2-Propenyl (mit der Formel H₂C=CH-CH₂-), 1-Methylethyl (oder Isopropyl, mit der Formel (CH₃)₂CH-), *n*-Butyl (mit der Formel CH₃-CH₂-CH₂-CH₂-), 2-Methylpropyl (oder Isobutyl, mit der Formel (CH₃)₂CH-CH₂-), 1-Methylpropyl (oder Butan-2-yl bzw. *sec*-Butyl, mit der Formel CH₃-CH₂-CH(CH₃)-), 1,1-Dimethylethyl (oder *tert*-Butyl mit der Formel (CH₃)₃CH-), *n*-Pentyl (mit der Formel CH₃-CH₂-CH₂-CH₂-CH₂-), 3-Methylbutyl (oder Isopentyl, mit der Formel (CH₃)₂CH-CH₂-CH₂-), Benzyl (oder Bn mit der Formel Phenyl-CH₂-), Propargyl (oder Ethinylmethyl, mit der Formel HC≡C-CH₂-), 1*H*-Indol-3-ylmethyl (mit der Formel 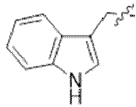 ), 1*N*-Methyl-1*H*-indol-3-ylmethyl (mit der Formel 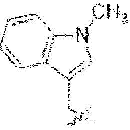 ), 3-Benzothienylmethyl (mit der Formel 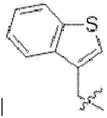 ), 1-Naphtylmethyl (mit der Formel 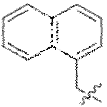 ), 9-Anthracenylmethyl (mit der Formel 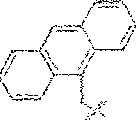 ) und Pyrenylmethyl (d.h. 1-Pyrenylmethyl mit der Formel 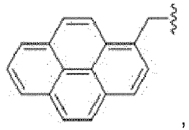 2-Pyrenylmethyl mit der 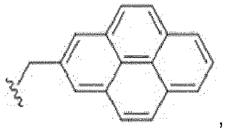 etc.);
- Z gleich O oder S ist;
- R und R' jeweils ausgewählt sind aus der Gruppe bestehend aus H, Methyl, Ethyl, *n-*Propyl, 1-Methylethyl, *n*-Butyl, 2-Methylpropyl (Isobutyl), 1-Methylpropyl (Butan-2-yl oder sec-Butyl), Benzyl und Propargyl, unter der Bedingung, dass, wenn R gleich H ist, R' nicht H ist;
- die C-Atome, die direkt mit den Substituenten Y₁, Y₂, Y₃, Y₄, Y₅ und X verbunden sind, in dieser Reihenfolge, eine jeweils alternierende absolute stereochemische Konfiguration aufweisen; und
- das C-Atom, welches mit dem Substituenten X verbunden ist, und das C-Atom in Position 4 des Thiazolidin- oder Oxazolidin-Rings, die gleiche absolute stereochemische Konfiguration aufweisen, wenn Z gleich O ist, und eine entgegengesetzte absolute stereochemische Konfiguration aufweisen, wenn Z gleich S ist;
unter der Bedingung, dass, wenn X, Y₁, Y₄ und Y₅ gleich 1-Methylethyl sind, Y₂ gleich 1*H-*Indol-3-yl ist, Y₃ gleich 2-Methylpropyl ist, R gleich H ist und R' gleich Methyl ist, Z nicht O ist; und die Salze hiervon, die Solvate hiervon und die Solvate der Salze hiervon.

Die folgenden Erklärungen bezüglich der unterschiedlichen Substituenten und Parameter gelten gleichermaßen für alle Aspekte und Ausführungsformten der vorliegenden Erfindung.

Der Begriff "jeweils ausgewählt" in Bezug auf die Parameter X und Y₁ bis Y₅, sowie R und R' bedeutet abgesehen von gewissen Ausnahmen grundsätzlich, dass diese Substituenten jeweils für gleiche Reste aus den entsprechenden obigen Listen oder jeweils unterschiedliche Reste dieser Listen stehen können. In der erfindungsgemäßen zyklischen Verbindung der Formel (I) ist Z nicht O, wenn in summa X, Y₁, Y₄ und Y₅ gleich 1-Methylethyl sind, Y₂ gleich 1*H*-Indol-3-ylmethyl ist, Y₃ gleich 2-Methylpropyl ist, R gleich H ist und R' gleich Methyl ist. Außerdem gilt für die Substituenten R und R', im Hinblick auf die vorliegende Erfindung, dass, wenn R gleich H ist, R' nicht H ist.

Die erfindungsgemäße zyklische Verbindung der Formel (I) vereint eine Reihe von, insbesondere für den kosmetischen Einsatz, vorteilhaften Eigenschaften.

Die erfindungsgemäße zyklische Verbindung der Formel (I) hat eine ausgezeichnete Löslichkeit und Stabilität in üblichen Lösungsmitteln und Trägern, vor allem kosmetisch akzeptablen Lösungsmitteln und kosmetisch akzeptablen Trägern, was die Verwendung in kosmetischen Zubereitungen begünstigt. Darüber hinaus wurde überraschenderweise gefunden, dass die erfindungsgemäße zyklische Verbindung der Formel (I) nicht oder nicht ohne Weiteres durch herkömmliche Proteasen abgebaut wird. Das führt zu einer vorteilhaften Stabilität beispielsweise beim Einsatz auf der Haut z.B. gegenüber bakteriellen und mykotischen Proteasen hautansässiger Mikroorganismen und hauteigener Proteasen.

Als kosmetischer Wirkstoff zur Verwendung in der nicht-therapeutischen Behandlung ist die neue zyklische Verbindung der Formel (I) hochpotent und dadurch bereits in kleinen Mengen bzw. geringen Konzentrationen für den kosmetischen Gebrauch einsetzbar. Beim Einsatz der erfindungsgemäßen Verbindung der Formel (I), als kosmetischer Wirkstoff zur nicht-therapeutischen Behandlung und/oder Vorbeugung eines kosmetischen oder dermatologischen Phänomens, das mit dem (insbesondere kutanen) Auftreten von Viren, Bakterien, Mykota, Parasiten und Protozoen zusammenhängt, können die körpereigenen Mechanismen, insbesondere die Haut-eigenen-Mechanismen, vorteilhaft ausgenutzt beziehungsweise unterstützt werden.

Die in Bezug auf die Substituenten X, Y₁ bis Y₅, R und R' aufgelisteten Reste, im Sinne der vorliegenden Erfindung, sind dem Fachmann auch wie folgt bekannt (wobei "-" am Ende der jeweiligen vereinfachten Strukturformeln, beziehungsweise ' 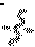 " am Ende der jeweiligen Strukturformeln, jeweils andeutet, wo der Rest mit der infrage stehenden Verbindung verbunden ist).

Im Sinne der vorliegenden Erfindung weist der Rest Methyl die Formel CH₃- auf und wird auch als "Me" abgekürzt. Der Rest Ethyl, mit der Formel CH₃-CH₂-, wird vorliegend auch als "Et" abgekürzt. Der Rest *n*-Propyl weist die Formel CH₃-CH₂-CH₂- auf. Der Rest 2-Propenyl weist die Formel H₂C=CH-CH₂- auf. Der Rest 1-Methylethyl im Sinne der vorliegenden Erfindung ist dem Fachmann auch bekannt als Isopropyl und weist die Formel (CH₃)₂CH- auf. Der Rest *n*-Butyl weist die Formel CH₃-CH₂-CH₂-CH₂- auf. Der Rest 2-Methylpropyl im Sinne der vorliegenden Erfindung ist dem Fachmann auch bekannt als Isobutyl und weist die Formel (CH₃)₂CH-CH₂- auf. Der Rest 1-Methylpropyl ist dem Fachmann auch bekannt als Butan-2-yl oder sec-Butyl und weist die Formel CH₃-CH₂-CH(CH₃)- auf. Der Rest 1,1-Dimethylethyl im Sinne der vorliegenden Erfindung ist dem Fachmann auch bekannt als *tert*-Butyl und weist die Formel (CH₃)₃CH- auf. Der Rest *n*-Pentyl weist die Formel CH₃-CH₂-CH₂-CH₂-CH₂- auf. Der Rest 3-Methylbutyl ist dem Fachmann auch bekannt als Isopentyl und weist die Formel (CH₃)₂CH-CH₂-CH₂- auf.

Der Rest Benzyl (bzw. (1-Phenyl)methyl) im Sinne der vorliegenden Erfindung wird auch mit "Bn" abgekürzt und weist die Formel Phenyl-CH₂- auf. Der Rest Propargyl ist dem Fachmann auch bekannt als Ethinylmethyl oder 2-Propynyl und weist die Formel HC≡C-CH₂- auf. Der Rest 1*H*-Indol-3-ylmethyl im Sinne der vorliegenden Erfindung, im Sinne der vorliegenden

Erfindung, auch bekannt als Indol-3-ylmethyl, weist die Formel 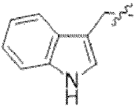 auf. Der Rest 1*N-*Methyl-1*H*-indol-3-ylmethyl, im Sinne der vorliegenden Erfindung, weist die Formel 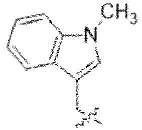 auf. Der Rest 3-Benzothienylmethyl, im Sinne der vorliegenden Erfindung, weist die Formel 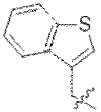 auf. Der Rest 1-Naphtylmethyl, im Sinne der vorliegenden Erfindung, weist die Formel 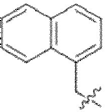 auf. Der Rest 9-Anthracenylmethyl weist die Formel 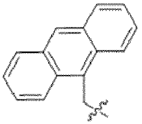 auf.

Der Rest Pyrenylmethyl (bzw. Pyren-ylmethyl), wie hierin verwendet, steht für einen Pyrenring, der über eines seiner äußeren Ringkohlenstoffatome C1 bis C10 mit einem Methylrest verbunden ist, welcher wiederum als Verbindungsstelle für den Pyrenylmethyl-Gesamtrest dient, d.h. 1-Pyrenylmethyl, 2-Pyrenylmethyl, 3-Pyrenylmethyl, 4-Pyrenylmethyl, 5-Pyrenylmethyl, etc. (bzw. 1-Pyren-ylmethyl, 2-Pyren-ylmethyl, 3-Pyren-ylmethyl, 4-Pyren-ylmethyl, 5-Pyren-ylmethyl, etc.). Der Rest 1-Pyrenylmethyl (bzw. (1-Pyrenyl)methyl, Pyren-1-ylmethyl, oder (Pyren-1-yl)methyl) beispielsweise weist die Formel 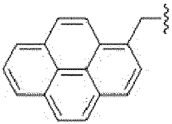 auf. Der Rest 2-Pyrenylmethyl (bzw. (2-Pyrenyl)methyl, Pyren-2-ylmethyl, oder (Pyren-2-yl)methyl) beispielsweise weist die Formel 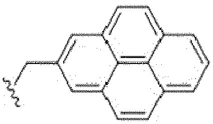 auf, etc.

Die unter die Formel (I) fallenden erfindungsgemäßen zyklischen Verbindungen sind zusammengesetzt aus fünf Aminosäurederivaten und einem Thiazolidin- oder Oxazolidin-Baustein. Unter einem "Aminosäurederivat" im Sinne der vorliegenden Erfindung sind grundsätzlich die klassischen 20 natürlichen L- und D-α-Aminosäuren und deren Diastereomere aber auch modifizierte Derivate hiervon mit abweichenden Resten (R) zu verstehen. Die erfindungsgemäße zyklische Verbindung der Formel (I) umfasst dabei lediglich Aminosäurederivate mit Resten (R) die den für die Substituenten Y₁ bis Y₅ definierten Resten entsprechen.

Ein Thiazolidin- oder Oxazolidin-Baustein im Sinne der vorliegenden Erfindung umfasst einen 1,3 Thiazolidin-Ring oder einen 1,3-Oxazolidin-Ring, welcher in Position 2 mit einem Kohlenstoffatom (C-Atom) verbunden ist, welches wiederum mit dem Substituenten X (und auch mit einer Aminogruppe) verbunden ist. Die Positionen im Thiazolidin- oder Oxazolidin-Ring ergeben sich aus der Standardnomenklatur, wie sie dem Fachmann bekannt ist, d.h: 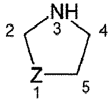 wobei Z gleich S oder O ist und die Zahlen 1-5 die Positionen im Ring definieren.

Der 1,3 Thiazolidin-Ring oder 1,3-Oxazolidin- Ring des Thiazolidin- oder Oxazolidin-Bausteins ist in Position 4, im freien (d.h. im nicht in ein Peptidderivat integrierten) Zustand mit einer Carboxylgruppe verbunden, und ist im linearen Peptidderivat (d.h in der linearen Vorstufe vor Zyklisierung zur erfindungsgemäßen zyklischen Verbindung) oder im zyklischen Peptidderivat (d.h. in der erfindungsgemäßen zyklischen Verbindung) über eine Amidgruppe (d.h. im C-Terminus des Thiazolidin- oder Oxazolidin-Bausteins) mit dem Peptidderivat-Rückgrat verbunden; im linearen Peptidderivat natürlich nur, falls der Thiazolidin- oder Oxazolidin-Baustein nicht C-terminal ist. Der 1,3 Thiazolidin-Ring oder 1,3-Oxazolidin-Ring ist in Position 5 an die Reste R und R' gebunden.

In der erfindungsgemäßen zyklischen Verbindung der Formel (I) weisen die C-Atome, die direkt mit den Substituenten Y₁, Y₂, Y₃, Y₄, Y₅ und X verbunden sind, in dieser Reihenfolge, eine jeweils alternierende absolute, und auch relative, stereochemische Konfiguration auf. Das heißt beispielsweise in der erfindungsgemäßen zyklischen Verbindung der Formel (I) weisen direkt aufeinanderfolgende Aminosäurederivate, eine jeweils alternierende absolute, und auch relative, stereochemische Konfiguration des α-Kohlenstoffs (Ca) auf.

Die Begriffe "*(alternierende) absolute stereochemische Konfiguration*" und "*(alternierende) relative stereochemische Konfiguration*" sind dem Fachmann bekannt. Der Begriff "*absolute stereochemische Konfiguration*", wie im Kontext der vorliegenden Erfindung verwendet, bezieht sich auf die *R*-/*S*-Nomenklatur (nicht aber auf die relative *D*-/*L*-Nomenklatur). Eine "*alternierende absolute stereochemische Konfiguration*" im Sinne der vorliegenden Erfindung bedeutet somit, dass, wenn die absolute stereochemische Konfiguration eines direkt mit einem Substituenten Y₁ bis Y₅ oder X verbundenen Kohlenstoffatoms (C-Atoms) eine R-Konfiguration ist, die entlang des Kettenrückrats der zyklischen Verbindung der Formel (I) auf beiden Seiten am nächsten liegenden C-Atome, welche auch mit einem Substituenten Y₁ bis Y₅ oder X direkt verbunden sind, eine S-Konfiguration aufweisen. Beispielsweise wenn in der zyklischen Verbindung der Formel (I) das C_{Y3} (das heißt, das C-Atom, welches direkt mit dem Substituenten Y₃ verbunden ist) eine R-Konfiguration aufweist, hat C_{Y4} (und auch C_{Y2}) eine S-Konfiguration, C_{Y5} eine R-Konfiguration, C_{X} eine S-Konfiguration, etc. Dagegen wenn C_{Y3} eine S-Konfiguration aufweist, hat C_{Y4} (und auch C_{Y2}) eine R-Konfiguration, C_{Y5} eine S-Konfiguration, C_{X} eine R-Konfiguration, etc.

Auch der Begriff "*alternierende relative stereochemische Konfiguration*" im Sinne der vorliegenden Erfindung ist in diesem Sinne zu verstehen. Der Begriff "*relative stereochemische Konfiguration*"*,* wie im Kontext der vorliegenden Erfindung verwendet, bezieht sich auf die D,L-Nomenklatur für Aminosäuren und deren Derivate. Eine "*alternierende stereochemische relative Konfiguration*" im Sinne der vorliegenden Erfindung bedeutet somit, dass, wenn die relative stereochemische Konfiguration eines direkt mit einem Substituenten Y₁ bis Y₅ oder X verbundenen C-Atoms eine L-Konfiguration ist, die entlang des Kettenrückrats der zyklischen Verbindung (I) auf beiden Seiten am nächsten liegenden C-Atome, welche auch mit einem Substituenten Y₁ bis Y₅ oder X direkt verbunden sind, eine D-Konfiguration aufweisen. Beispielsweise wenn in der zyklischen Verbindung der Formel (I) das C_{Y4} (da heißt, das C-Atom, welches direkt mit dem Substituenten Y₄ verbunden ist) eine L-Konfiguration aufweist, hat C_{Y5} (und auch C_{Y3}) eine D-Konfiguration, C_{X} eine L-Konfiguration, C_{Y1} eine D-Konfiguration etc. Dagegen wenn C_{Y4} eine D-Konfiguration aufweist, hat C_{Y5} (und auch C_{Y3}) eine L-Konfiguration, C_{X} eine D-Konfiguration, C_{Y1} eine L-Konfiguration etc.

Gleichermaßen sind die Begriffe "*entgegengesetzte absolute stereochemische Konfiguration*" und "*gleiche absolute stereochemische Konfiguration*" und "entgegengesetzte relative s*tereochemische* Konfiguration" bzw. "*gleiche relative stereochemische Konfiguration*" in diesem Sinne zu verstehen. Das heißt, eine "*entgegengesetzte absolute stereochemische Konfiguration*", im Sinne der vorliegenden Erfindung, bedeutet, dass, wenn die eine absolute stereochemische Konfiguration eine R-Konfiguration ist, die entgegengesetzte absolute stereochemische Konfiguration eine S-Konfiguration ist. Und eine "*gleiche absolute stereochemische Konfiguration*" im Sinne der vorliegenden Erfindung bedeutet, dass, wenn die eine absolute Konfiguration eine R-Konfiguration ist, die gleiche absolute Konfiguration auch eine R-Konfiguration ist. Dies gilt analog für die relative stereochemische Konfiguration, so dass eine "*entgegengesetzte relative stereochemische Konfiguration*" im Sinne der vorliegenden Erfindung bedeutet, dass wenn die eine relative stereochemische Konfiguration eine D-Konfiguration ist, die entgegengesetzte relative stereochemische Konfiguration eine L-Konfiguration ist, etc.

Dabei ist für die erfindungsgemäße zyklische Verbindung der Formel (I) nur die alternierende stereochemische Konfiguration vorgegeben (sowie die entsprechende entgegengesetzte bzw. gleiche absolute stereochemische Konfiguration für den Thiazolidin- bzw. Oxazolidinbaustein, wie im Folgenden beschrieben); eine konkrete Zuteilung der absoluten bzw. relativen stereochemischen Konfiguration einzelner C-Atome in der erfindungsgemäßen zyklischen Verbindung der Formel (I) dagegen nicht.

Insbesondere kann die erfindungsgemäße zyklische Verbindung der Formel (I) also beispielsweise abhängig von ihrer spezifischen Struktur in stereoisomeren Formen (Enantiomere, Diastereomere) vorliegen. Die Erfindung umfasst daher auch die Enantiomere oder Diastereomere und entsprechende Gemische davon. Die stereoisomeren einheitlichen Bestandteile können auf bekannte Weise aus solchen Gemischen von Enantiomeren und/oder Diastereomeren isoliert werden. Wenn die Verbindung der Erfindung in tautomeren Formen auftreten kann, umfasst die vorliegende Erfindung alle tautomeren Formen.

Im Thiazolidin- oder Oxazolidin-Baustein der erfindungsgemäßen zyklischen Verbindung der Formel (I) ist das C-Atom, welches direkt mit dem Substituenten X verbunden ist auch über eine direkte Bindung mit einem Thiazolidin- oder Oxazolidin-Ring in Position 2 verbunden.

In der erfindungsgemäßen zyklischen Verbindung der Formel (I) weist das C-Atom, welches direkt mit dem Substituenten X verbunden ist, und das C-Atom in Position 4 des Thiazolidin-oder Oxazolidin-Rings, die gleiche absolute stereochemische Konfiguration auf, wenn Z gleich O ist (also falls es sich um einen Oxazolidin-Ring handelt), und eine entgegengesetzte absolute stereochemische Konfiguration auf, wenn Z gleich S ist (also falls es sich um einen Thiazolidin-Ring handelt) ist. Die Begriffe "*entgegengesetzte absolute stereochemische Konfiguration*" und "*gleiche absolute stereochemische Konfiguration*" sind dabei wie oben definiert zu verstehen. Das heißt beispielsweise, wenn eine erfindungsgemäße zyklische Verbindung (I) einen Oxazolidin-Ring aufweist und dieser in Position 4 eine R-Konfiguration aufweist, auch das C-Atom, welches direkt mit dem Substituenten X verbunden ist, eine R-Konfiguration aufweist, bzw. wenn eine erfindungsgemäße zyklische Verbindung (I) einen Thiazolidin-Ring aufweist und dieser in Position 4 eine R-Konfiguration aufweist, das C-Atom, welches direkt mit dem Substituenten X verbunden ist, eine S-Konfiguration aufweist.

Für die Zwecke der vorliegenden Erfindung bevorzugte Salze sind physiologisch (insbesondere kosmetisch) verträgliche Salze der erfindungsgemäßen Verbindung der Formel (I). Eingeschlossen sind jedoch auch Salze, die selbst nicht für kosmetische Anwendungen geeignet sind, sondern beispielsweise zur Isolierung oder Reinigung der erfindungsgemäßen Verbindung der Formel (I) verwendet werden können.

Beispiele für kosmetisch verträgliche Salze der zyklischen Verbindung der Formel (I) umfassen Salze anorganischer Basen wie Ammoniumsalze, Alkalimetallsalze, insbesondere Natrium-oder Kaliumsalze, Erdalkalimetallsalze, insbesondere Magnesium- oder Calciumsalze; Salze organischer Basen, insbesondere Salze, die von Cyclohexylamin, Benzylamin, Octylamin, Ethanolamin, Diethanolamin, Diethylamin, Triethylamin, Ethylendiamin, Procain, Morpholin, Pyrrolin, Piperidin, N-Ethylpiperidin, N-Methylmorpholin, Piperazin als organische Base abgeleitet sind; oder Salze mit basischen Aminosäuren, insbesondere Lysin, Arginin, Ornithin und Histidin.

Beispiele für kosmetisch verträgliche Salze der Verbindung der Formel (I) umfassen auch Salze anorganischer Säuren wie Hydrochloride, Hydrobromide, Sulfate, Phosphate oder Phosphonate; Salze organischer Säuren, insbesondere Acetate, Formiate, Propionate, Lactate, Citrate, Fumarate, Maleate, Benzoate, Tartrate, Malate, Methansulfonate, Ethansulfonate, Toluolsulfonate oder Benzolsulfonate; oder Salze mit sauren Aminosäuren, insbesondere Aspartat oder Glutamat.

Solvate im Sinne der Erfindung beziehen sich auf jene Formen der erfindungsgemäßen Verbindung der Formel (I), die im festen oder flüssigen Zustand durch Koordination mit Lösungsmittelmolekülen einen Komplex bilden. Hydrate sind eine spezielle Form von Solvaten, bei denen die Koordination mit Wasser erfolgt. Die erfindungsgemäße Verbindung der Formel (I) kann auch komplexiert sein, z.B. mit Eisen, Calcium usw., wobei die Verbindung der Formel (I) als Ligand wirken kann, so dass auch entsprechende Komplexe Gegenstand der vorliegenden Erfindung sind.

In einer bevorzugten Ausführungsform der erfindungsgemäßen zyklischen Verbindung der Formel (I) ist der Substituent X ausgewählt aus der Gruppe bestehend aus Ethyl, *n*-Propyl, 2-Propenyl, 1-Methylethyl, *n*-Butyl, 2-Methylpropyl, 1-Methylpropyl, 1,1-Dimethylethyl, n-Pentyl, 3-Methylbutyl, Benzyl, Propargyl, 1*N*-Methyl-1*H*-indol-3-ylmethyl, 3-Benzothienylmethyl, 1-Naphtylmethyl, 9-Anthracenylmethyl und Pyrenylmethyl (wobei als Pyrenylmethyl bevorzugt 1-Pyrenylmethyl oder 2-Pyrenylmethyl, insbesondere bevorzugt 1-Pyrenylmethyl, ausgewählt ist). In einer weiteren bevorzugten Ausführungsform ist X ausgewählt aus der Gruppe bestehend aus *n*-Propyl, 1-Methylethyl, *n*-Butyl, 2-Methylpropyl, 1-Methylpropyl, 1,1-Dimethylethyl, Benzyl, Propargyl, und Pyrenylmethyl (bevorzugt 1-Pyrenylmethyl oder 2-Pyrenylmethyl, insbesondere bevorzugt 1-Pyrenylmethyl). In noch einer weiteren bevorzugten Ausführungsform ist X ausgewählt aus der Gruppe bestehend aus 1-Methylethyl, 2-Methylpropyl, 1-Methylpropyl, 1,1-Dimethylethyl, Benzyl, Propargyl, und Pyrenylmethyl (bevorzugt 1-Pyrenylmethyl oder 2-Pyrenylmethyl, insbesondere bevorzugt 1-Pyrenylmethyl). In einer alternativ bevorzugten Ausführungsform ist X ausgewählt aus der Gruppe bestehend aus *n*-Propyl, 1-Methylethyl, *n*-Butyl, 2-Methylpropyl, 1-Methylpropyl, 1,1-Dimethylethyl, *n-*Pentyl und 3-Methylbutyl.

In einer bevorzugten Ausführungsform der vorliegenden Erfindung sind die Substituenten Y₁ bis Y₅ jeweils ausgewählt aus der Gruppe bestehend aus Methyl, Ethyl, *n*-Propyl, 2-Propenyl, 1-Methylethyl, *n*-Butyl, 2-Methylpropyl, 1-Methylpropyl, 1,1-Dimethylethyl, Benzyl, Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl, und Pyrenylmethyl (vorzugsweise 1-Pyrenylmethyl oder 2-Pyrenylmethyl, insbesondere bevorzugt 1-Pyrenylmethyl). In einer weiteren bevorzugten Ausführungsform der vorliegenden Erfindung sind Y₁ bis Y₅ jeweils ausgewählt aus der Gruppe bestehend aus Methyl, 1-Methylethyl, 2-Methylpropyl, 1-Methylpropyl, Benzyl, 1*H*-Indol-3-ylmethyl, Propargyl, und Pyrenylmethyl (bevorzugt 1-Pyrenylmethyl oder 2-Pyrenylmethyl, insbesondere bevorzugt 1-Pyrenylmethyl).

Für Y₂ beispielsweise bevorzugte Reste sind 1*H*-Indol-3-ylmethyl, 1*N*-Methyl-1*H*-indol-3-ylmethyl, 3-Benzothienylmethyl, 1-Naphtylmethyl, 9-Anthracenylmethyl, und Pyrenylmethyl (bevorzugt 1-Pyrenylmethyl oder 2-Pyrenylmethyl, insbesondere bevorzugt 1-Pyrenylmethyl), mehr bevorzugt 1*H*-Indol-3-ylmethyl oder Pyrenylmethyl (bevorzugt 1-Pyrenylmethyl oder 2-Pyrenylmethyl, insbesondere bevorzugt 1-Pyrenylmethyl), wobei dann Y₅ vorzugsweise kein aromatischer Rest ist (insbesondere kein 1*H*-Indol-3-ylmethyl oder Pyrenylmethyl ist). Für Y₃ beispielsweise bevorzugte Reste sind *n*-Propyl, 1-Methylethyl, *n*-Butyl, 2-Methylpropyl, 1-Methylpropyl und 1,1-Dimethylethyl, insbesondere bevorzugt ist 2-Methylpropyl. Für Y₄ beispielsweise bevorzugte Reste sind Methyl, Ethyl, *n*-Propyl, 1-Methylethyl, *n*-Butyl, 2-Methylpropyl, 1-Methylpropyl, 1,1-Dimethylethyl, insbesondere bevorzugt 1-Methylethyl. Für Y₅ beispielsweise bevorzugte Reste sind 1*H*-Indol-3-ylmethyl, 1*N*-Methyl-1*H*-indol-3-ylmethyl, 3-Benzothienylmethyl, 1-Naphtylmethyl, 9-Anthracenylmethyl, und Pyrenylmethyl (bevorzugt 1-Pyrenylmethyl oder 2-Pyrenylmethyl, insbesondere bevorzugt 1-Pyrenylmethyl), mehr bevorzugt 1*H*-Indol-3-ylmethyl oder Pyrenylmethyl (bevorzugt 1-Pyrenylmethyl oder 2-Pyrenylmethyl, insbesondere bevorzugt 1-Pyrenylmethyl), bspw. insbesondere bevorzugt 1*H-*Indol-3-ylmethyl, wobei dann Y₂ vorzugsweise kein aromatischer Rest ist (insbesondere kein 1*H*-Indol-3-ylmethyl oder Pyrenylmethyl ist).

In einer weiteren bevorzugten Ausführungsform der vorliegenden Erfindung ist Y₂ gleich 1*H-*Indol-3-ylmethyl oder Pyrenylmethyl (bevorzugt 1-Pyrenylmethyl oder 2-Pyrenylmethyl, insbesondere bevorzugt 1-Pyrenylmethyl), falls Y₅ gleich 1-Methylethyl ist, oder ist Y₂ gleich 1-Methylethyl, falls Y₅ gleich 1*H*-Indol-3-ylmethyl oder Pyrenylmethyl (bevorzugt 1-Pyrenylmethyl oder 2-Pyrenylmethyl, insbesondere bevorzugt 1-Pyrenylmethyl) ist. In noch einer weiteren bevorzugten Ausführungsform der vorliegenden Erfindung ist Y₂ gleich 1*H-*Indol-3-ylmethyl oder Pyrenylmethyl (bevorzugt 1-Pyrenylmethyl oder 2-Pyrenylmethyl, insbesondere bevorzugt 1-Pyrenylmethyl), und Y₅ gleich 1-Methylethyl, oder ist Y₂ gleich 1-Methylethyl, und Y₅ gleich 1*H*-Indol-3-ylmethyl oder Pyrenylmethyl (bevorzugt 1-Pyrenylmethyl oder 2-Pyrenylmethyl, insbesondere bevorzugt 1-Pyrenylmethyl).

In einer insbesondere bevorzugten Ausführungsform der erfindungsgemäßen zyklischen Verbindung der Formel (I) ist Z gleich S. In einer alternativen bevorzugten Ausführungsform der erfindungsgemäßen zyklischen Verbindung der Formel (I) ist Z gleich O.

In einer bevorzugten Ausführungsform der erfindungsgemäßen zyklischen Verbindung der Formel (I) sind R und R' jeweils ausgewählt aus der Gruppe bestehend aus H, Methyl, Ethyl, *n*-Propyl, 1-Methylethyl, Benzyl und Propargyl. In einer weiteren bevorzugten Ausführungsform der erfindungsgemäßen zyklischen Verbindung der Formel (I) sind R und R' jeweils ausgewählt aus der Gruppe bestehend aus H, Methyl, Ethyl, und Propargyl. In einer weiteren bevorzugten Ausführungsform der zyklischen Verbindung der Formel (I) sind R und R' beide Methyl. In einer weiteren bevorzugten Ausführungsform der zyklischen Verbindung der Formel (I) ist R gleich H; und R' gleich Methyl, Ethyl, *n*-Propyl, 1-Methylethyl, Benzyl oder Propargyl.

In einer konkreten bevorzugten Ausführungsform der erfindungsgemäßen zyklischen Verbindung der Formel (I) ist X ausgewählt aus der Gruppe bestehend aus 1-Methylethyl, 2-Methylpropyl, 1-Methylpropyl, Benzyl, Propargyl, Pyrenylmethyl (bevorzugt 1-Pyrenylmethyl oder 2-Pyrenylmethyl, insbesondere bevorzugt 1-Pyrenylmethyl); sind Y₁ bis Y₅ jeweils ausgewählt sind aus der Gruppe bestehend aus Methyl, Ethyl, *n*-Propyl, 1-Methylethyl, 2-Methylpropyl, 1-Methylpropyl, Benzyl, 1*H*-Indol-3-yl-methyl, Propargyl, Pyrenylmethyl (bevorzugt 1-Pyrenylmethyl oder 2-Pyrenylmethyl, insbesondere bevorzugt 1-Pyrenylmethyl); ist Z gleich O oder S; und/oder sind R und R' jeweils ausgewählt aus der Gruppe bestehend aus H, Methyl, Ethyl, und Propargyl, unter der Bedingung, dass, wenn R gleich H ist, R' nicht H ist.

Überraschenderweise wurde gefunden, dass die zyklische Verbindung der Formel (I), wobei entweder der Substituent Y₂ oder der Substituent Y₅, bevorzugt aber nicht in Positionen Y₂ und Y₅ gleichzeitig, ein Pyrenylmethyl ist eine, im Vergleich zu einer zyklischen Verbindung der Formel (I) bei der der Substituent Y₂ oder der Substituent Y₅ ein 1*H*-Indol-3-ylmethyl ist, eine hohe nicht-selektive antimikrobielle, insbesondere nicht-selektive antibakterielle, Aktivität aufweist. In einer bevorzugten Ausführungsform der zyklischen Verbindung der Formel (I) ist der Substituent Y₂ oder der Substituent Y₅ ein Pyrenylmethyl (vorzugsweise 1-Pyrenylmethyl oder 2-Pyrenylmethyl, insbesondere bevorzugt 1-Pyrenylmethyl). In einer weiteren bevorzugten Ausführungsform der zyklischen Verbindung der Formel (I) ist entweder der Substituent Y₂ oder der Substituent Y₅ Pyrenylmethyl (vorzugsweise 1-Pyrenylmethyl oder 2-Pyrenylmethyl, insbesondere bevorzugt 1-Pyrenylmethyl), und vorzugsweise kein anderer Substituent X und Y₁ bis Y₅, ein Pyrenylmethyl.

In einer weiteren bevorzugten Ausführungsform der zyklischen Verbindung der Formel (I) sind R und R' beide Methyl; Y₂ ist gleich 1*H*-Indol-3-ylmethyl oder Pyrenylmethyl, falls Y₅ gleich 1-Methylethyl ist, oder Y₂ ist gleich 1-Methylethyl, falls Y₅ gleich 1*H*-Indol-3-ylmethyl oder Pyrenylmethyl ist; und X gleich 1-Methylethyl. In einer weiteren bevorzugten Ausführungsform der zyklischen Verbindung der Formel (I) sind R und R' beide Methyl; ist Y₂ gleich 1*H*-Indol-3-ylmethyl oder Pyrenylmethyl, und Y₅ gleich 1-Methylethyl, oder ist Y₂ gleich 1-Methylethyl, und Y₅ gleich 1H-Indol-3-ylmethyl oder Pyrenylmethyl; ist X gleich 1-Methylethyl; und Z bevorzugt S. Das Pyrenylmethyl ist dabei vorzugsweise 1-Pyrenylmethyl oder 2-Pyrenylmethyl, insbesondere bevorzugt 1-Pyrenylmethyl.

In einer weiteren bevorzugten Ausführungsform der zyklischen Verbindung der Formel (I) ist R gleich H; R' gleich Methyl, Ethyl, *n*-Propyl, 1-Methylethyl, Benzyl oder Propargyl; Y₂ gleich 1*H*-Indol-3-ylmethyl oder Pyrenylmethyl, falls Y₅ gleich 1-Methylethyl ist, oder Y₂ gleich 1-Methylethyl, falls Y₅ gleich 1*H*-Indol-3-ylmethyl oder Pyrenylmethyl ist; X gleich 1-Methylethyl; und Z bevorzugt S. In noch einer weiteren bevorzugten Ausführungsform der zyklischen Verbindung der Formel (I) ist R gleich H; R' gleich Methyl, Ethyl, *n*-Propyl, 1-Methylethyl, Benzyl oder Propargyl; ist Y₂ ist gleich 1*H*-Indol-3-ylmethyl oder Pyrenylmethyl, und Y₅ gleich 1-Methylethyl, oder ist Y₂ gleich 1-Methylethyl, und Y₅ gleich 1*H*-Indol-3-ylmethyl oder Pyrenylmethyl; ist X gleich 1-Methylethyl; und Z bevorzugt S. Das Pyrenylmethyl ist dabei vorzugsweise 1-Pyrenylmethyl oder 2-Pyrenylmethyl, insbesondere 1-Pyrenylmethyl.

In noch einer weiteren bevorzugten Ausführungsform der zyklischen Verbindung der Formel (I) ist R gleich H; R' gleich Methyl, *n*-Propyl, 1-Methylethyl, Benzyl oder Propargyl; Y₂ gleich 1*H*-Indol-3-ylmethyl oder Pyrenylmethyl, falls Y₅ gleich 1-Methylethyl ist, oder Y₂ gleich 1-Methylethyl, falls Y₅ gleich 1*H*-Indol-3-ylmethyl oder Pyrenylmethyl ist; X gleich 1-Methylethyl oder 2-Methylpropyl. In noch einer weiteren bevorzugten Ausführungsform der zyklischen Verbindung der Formel (I) ist R gleich H; R' gleich Methyl, n-Propyl, 1-Methylethyl, Benzyl oder Propargyl; ist Y₂ gleich 1*H*-Indol-3-ylmethyl oder Pyrenylmethyl, und Y₅ gleich 1-Methylethyl, oder ist Y₂ gleich 1-Methylethyl, und Y₅ gleich 1*H*-Indol-3-ylmethyl oder Pyrenylmethyl; ist X gleich 1-Methylethyl oder 2-Methylpropyl. Das Pyrenylmethyl ist dabei vorzugsweise 1-Pyrenylmethyl oder 2-Pyrenylmethyl, insbesondere bevorzugt 1-Pyrenylmethyl.

In noch einer weiteren bevorzugten Ausführungsform der zyklischen Verbindung der Formel (I) sind R und R' beide 1-Methylethyl; und ist Y₂ gleich 1*H*-Indol-3-ylmethyl. In noch einer weiteren bevorzugten Ausführungsform der zyklischen Verbindung der Formel (I) sind R und R' jeweils H oder Methyl; ist Y₂ gleich 1*H*-Indol-3-yl; und X gleich 1-Methylethyl.

In einer alternativ bevorzugten Ausführungsform liegt in der erfindungsgemäßen zyklischen Verbindung (I), falls Y₅ gleich Pyrenylmethyl ist, das C-Atom welches direkt mit dem Substituenten Y₅ verbunden ist in einer (absoluten) S-Konfiguration (d.h. L-Konfiguration) vor.

In spezifischen bevorzugten Ausführungsformen ist die zyklische Verbindung der Formel (I) gekennzeichnet durch eine der folgenden Formeln I.1a bis I.18a:

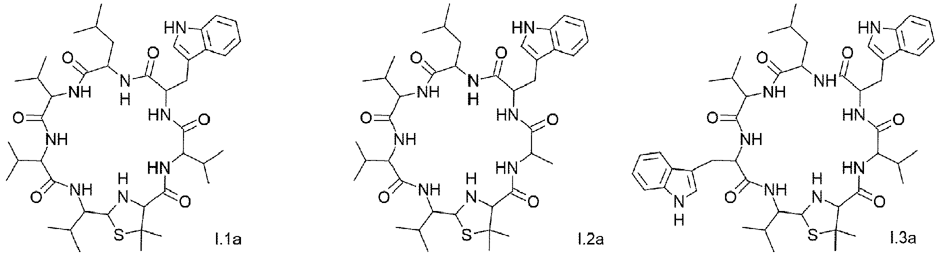

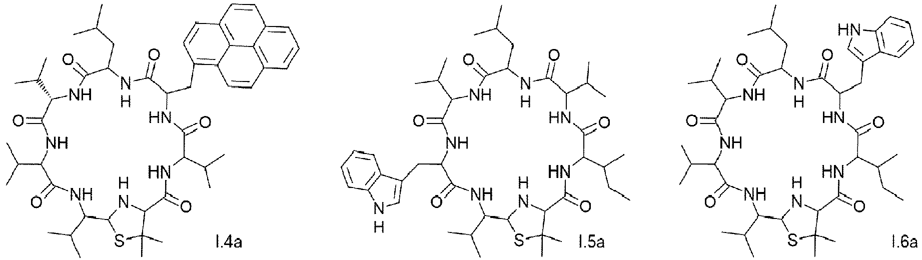

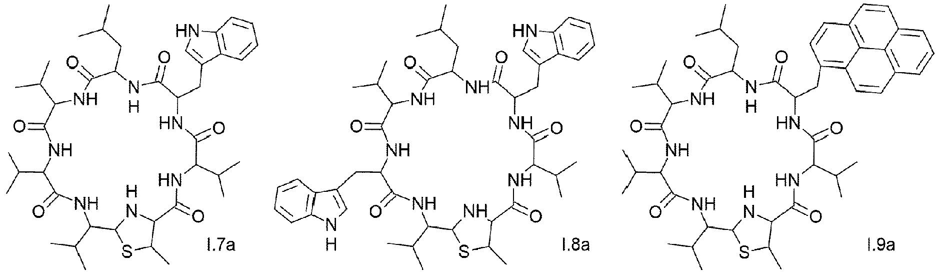

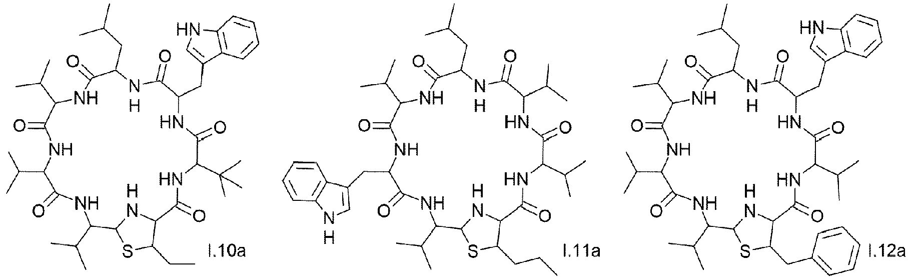

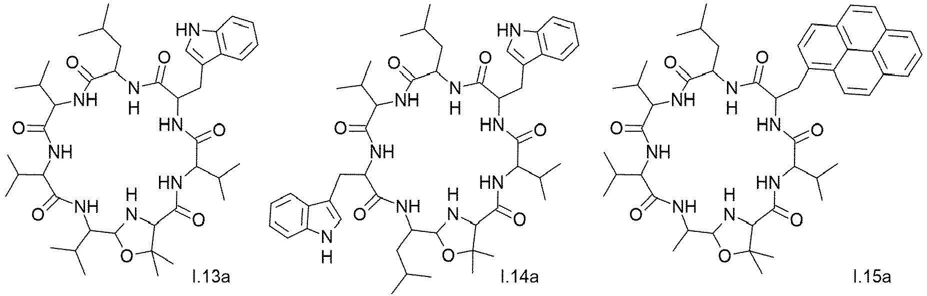

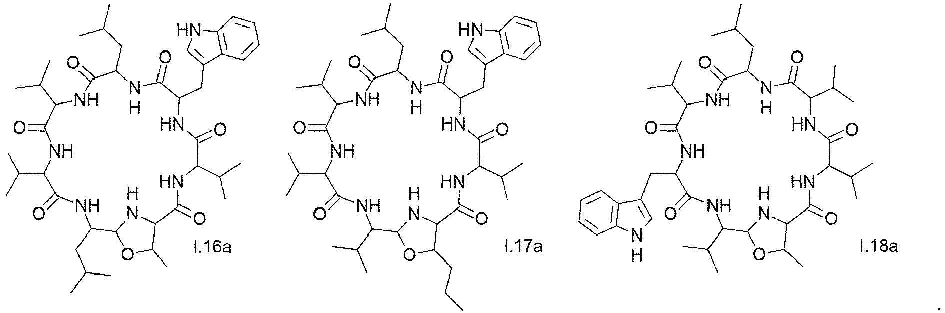

In weiteren spezifischen bevorzugten Ausführungsformen ist die zyklische Verbindung der Formel (I) gekennzeichnet durch eine der folgenden Formeln I.1 bis I.18:

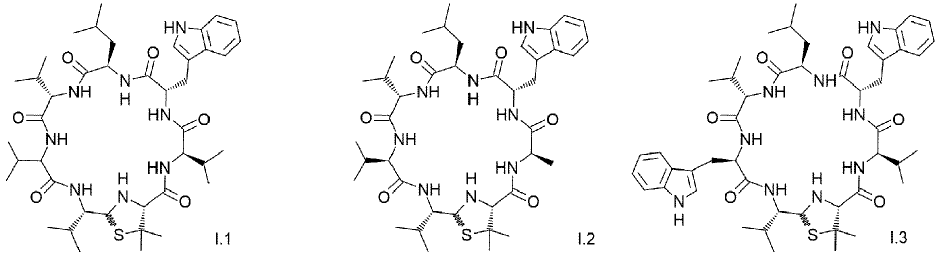

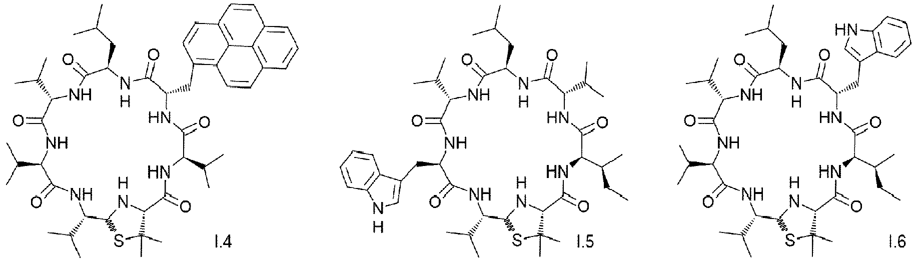

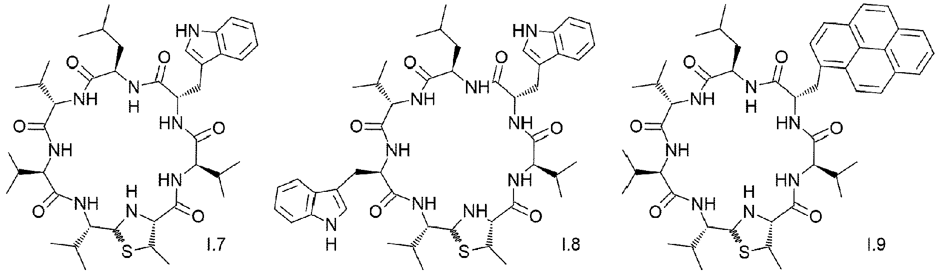

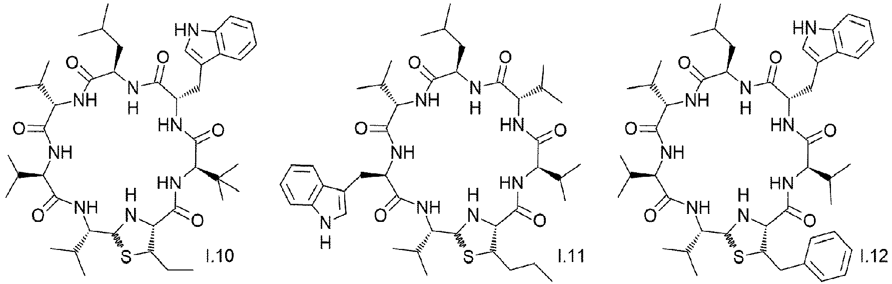

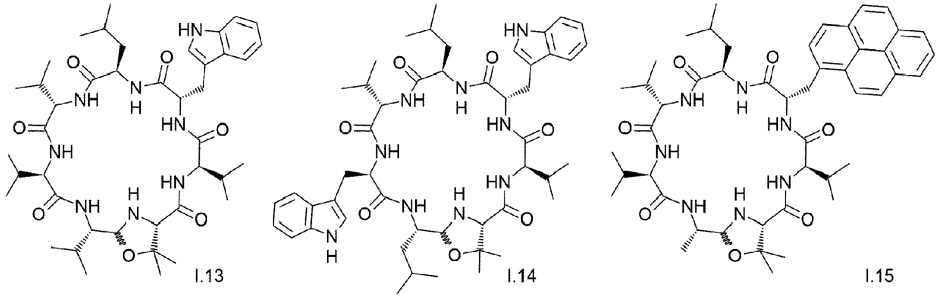

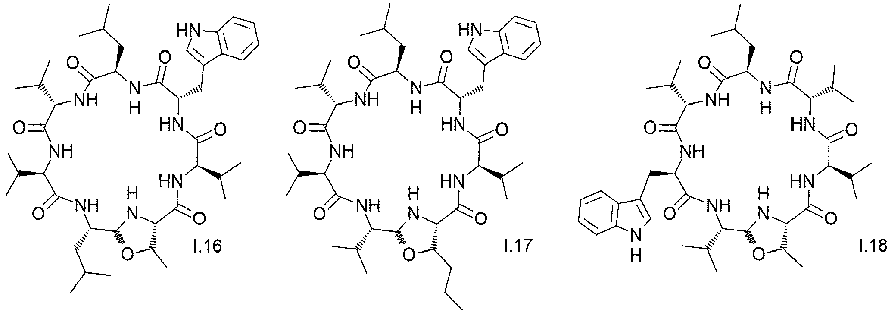

Insbesondere bevorzugt sind die zyklischen Verbindungen der Formel I.1a bis I.4a bzw. I.1bis I.4. In noch weiteren spezifischen bevorzugten Ausführungsformen liegt in den Formeln I.14a bis I.18a bzw. I.14 bis I.18 anstatt eines Oxazolidinrings ein Thiazolidinring vor.

Überraschenderweise haben die vorliegenden Erfinder gefunden, dass die erfindungsgemäße Verbindung der Formel (I) eine Reihe von, insbesondere für den kosmetischen Einsatz, vorteilhaften Eigenschaften aufweist. So weist die erfindungsgemäße zyklische Verbindung der Formel (I) eine ausgezeichnete antimikrobielle, insbesondere auch antivirale und/oder nicht-selektive antibakterielle, Wirksamkeit auf. Aus diesem Grund ist die erfindungsgemäße Verbindung der Formel (I) vorzüglich beispielsweise für den Einsatz als kosmetischer Wirkstoff in kosmetischen Zubereitungen, insbesondere für die topische Anwendung geeignet, wo er seine antimikrobiellen, insbesondere antiviralen, (nicht-selektive) antibakteriellen und/oder antimykotischen, bevorzugt antiviralen und/oder nicht-selektiven antibakteriellen, Eigenschaften entfalten kann.

So wurde beispielsweise überraschend gefunden, dass die erfindungsgemäße zyklische Verbindung der Formel (I) eine nicht-selektive antimikrobielle, insbesondere nicht-selektive antibakterielle Wirksamkeit, vorzugsweise konkret gegen grampositive Bakterien, aufweist. Bevorzugt weisen die erfindungsgemäßen zyklischen Verbindungen gegen gramnegative Bakterien dagegen keine oder nur eine minimale antibakterielle Wirksamkeit auf. Unter einer "nicht-selektiven antimikrobiellen Wirksamkeit" ist vorliegend vorzugsweise zu verstehen, dass die erfindungsgemäße zyklische Verbindung der Formel (I) breit auf verschiedenste Mikroben (also nicht spezifisch) antimikrobiell wirkt. Unter einer "nicht-selektiven antibakteriellen Wirksamkeit" ist vorliegend vorzugsweise zu verstehen, dass die erfindungsgemäße zyklische Verbindung der Formel (I) breit gegen verschiedenste Bakterien (also nicht spezifisch), vorzugsweise aber konkret gegen grampositive Bakterien, jedoch bevorzugt nicht oder nur minimal (d.h. nicht nennenswert) gegen gramnegative Bakterien, antibakteriell wirkt.

Weiterhin hat die erfindungsgemäße zyklische Verbindung der Formel (I) eine unterstützende Wirkung auf die angeborene Immunantwort.

Außerdem wurde überraschend gefunden, dass die nicht-selektiv antibakteriell wirksame erfindungsgemäße zyklische Verbindung der Formel (I) bei deren Verwendung zu keiner, bzw. nur in einem äußerst geringen Maße zu einer, Resistenzbildung führt; die erfindungsgemäße zyklische Verbindung der Formel (I) insbesondere beispielsweise keine relevante Resistenzbildung aufweist, die aus dem Stand der Technik bekannte Verbindung Lugdunin dagegen schon und somit ein signifikanter Unterschied in Bezug auf die Resistenzbildung besteht. Der Begriff "Resistenzbildung" ist dabei in für den Fachmann bekannter Weise und vorzugsweise in Bezug auf die antimikrobielle, insbesondere die antibakterielle, Wirksamkeit zu verstehen. Überaschenderweise wurde gefunden, dass die erfindungsgemäße Verbindung der Formel (I) sich in Bezug auf das Wirkungsgeschehen beispielsweise von der bekannten Verbindung Lugdunin unterscheidet.

In einer bevorzugten Ausführungsform der vorliegenden Erfindung weist die erfindungsgemäße Verbindung der Formel (I) eine antimikrobielle, insbesondere antivirale, antibakterielle und/oder antimykotische, vorzugsweise antivirale und/oder nicht-selektive antibakterielle (bevorzugt gegen grampositive Bakterien, jedoch bevorzugt nicht oder nur minimal [d.h. nicht nennenswert, bspw. mit einer mindestens 10-fach, bevorzugt mindestens 20-fach, mehr bevorzugt mindestens 30-fach, etc., größeren minimalen Hemmkonzentration (MHK) im Vergleich zu grampositiven Bakterien] gegen gramnegative Bakterien), Wirksamkeit auf, und/oder hat eine unterstützende Wirkung auf die angeborene Immunantwort (bspw. der Haut).

In einer besonders bevorzugten Ausführungsform weist die erfindungsgemäße Verbindung der Formel (I), beispielsweise bei topischem Einsatz in einer kosmetischen Zubereitung auf der Haut, eine antivirale, antibakterielle und/oder antimykotische, vorzugsweise antivirale und/oder nicht-selektive antibakterielle (bevorzugt lediglich gegen grampositive Bakterien, jedoch bevorzugt nicht oder nur minimal (d.h. nicht nennenswert) gegen gramnegative Bakterien), Wirksamkeit auf, und/oder hat eine unterstützende Wirkung auf die angeborene Immunantwort (bspw. der Haut).

### Thiazolidin- oder Oxazolidin-Baustein der Formel (II)

In einem zweiten Aspekt ist die vorliegende Erfindung gerichtet auf einen neuen Thiazolidin-oder Oxazolidin-Baustein der Formel (II): 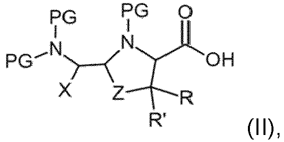 wobei:
- Z gleich O oder S ist;
- R und R' jeweils ausgewählt sind aus der Gruppe bestehend aus H, Methyl, Ethyl, *n-*Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
- PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), *tert*-Butyloxycarbonyl (Boc), Benzyloxycarbonyl (Cbz), Benzyl (Bn), Trityl (Trt), Acetyl (Ac) und Tosyl (Ts);
- X ausgewählt ist aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl;
- das C-Atom, welches mit dem Substituenten X verbunden ist, und das C-Atom in Position 4 des Thiazolidin- oder Oxazolidin-Rings, die gleiche absolute stereochemische Konfiguration aufweisen, wenn Z gleich O ist, und eine entgegengesetzte absolute stereochemische Konfiguration aufweisen, wenn Z gleich S ist;
unter der Bedingung, dass, wenn Z gleich S ist und X gleich Methyl, Benzyl oder 1*H*-Indol-3-ylmethyl ist, R nicht Methyl ist;
und die Salze hiervon, die Solvate hiervon und die Solvate der Salze hiervon.

Der erfindungsgemäßen Thiazolidin- oder Oxazolidinbaustein der Formel (II) zeichnet sich insbesondere durch eine hervorragende Löslichkeit und Stabilität in organischen Lösungsmitteln und damit verbundene überlegene Kupplungseigenschaften bei der Einbringung als Baustein in eine Festphasensynthese. Dies ist insbesondere deshalb vorteilhaft weil es erlaubt die zyklischen Verbindungen der Formel (I) bis auf die Zyklisierung komplett über Festphasensynthese aus Aminosäurederivaten und einem Thiazolidin- oder Oxazolidinbaustein herzustellen.

In einer insbesondere bevorzugten Ausführungsform des erfindungsgemäßen Thiazolidin-oder Oxazolidinbausteins ist Z gleich S. In einer alternativen bevorzugten Ausführungsform der erfindungsgemäßen zyklischen Verbindung der Formel (I) ist Z gleich O.

R und R' sind jeweils ausgewählt aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist. Dies bedeutet, dass beispielsweise R gleich H und R' gleich Methyl sein kann oder auch beispielsweise R und R' beide gleich Methyl sein können, nicht aber beispielsweise R und R' gleich H sein können, etc. In einer bevorzugten Ausführungsform des erfindungsgemäßen Thiazolidin- oder Oxazolidinbausteins sind R und R' jeweils ausgewählt aus der Gruppe bestehend aus H, Methyl, Ethyl, *n*-Propyl, 1-Methylethyl, Benzyl und Propargyl. In einer weiteren bevorzugten Ausführungsform der erfindungsgemäßen zyklischen Verbindung der Formel (I) sind R und R' jeweils ausgewählt aus der Gruppe bestehend aus H, Methyl, Ethyl, und Propargyl.

PG steht jeweils für H oder eine Schutzgruppe, wobei einzelne PG gleich oder verschieden sein können, sodass beispielsweise ein oder mehrere PG für H stehen können und ein oder mehrere PG für Schutzgruppen stehen können, wobei die Schutzgruppen gleich oder verschieden sein können. Das heißt, dass in dem Thiazolidin- oder Oxazolidinbaustein der Formel (II) alle drei Substituenten PG für ein H stehen können, oder dass zwei Substituenten PG für ein H stehen können wobei dann ein Substituent PG für eine Schutzgruppe (bevorzugt dann ein PG der terminalen Aminogruppe) steht, oder dass ein Substituent PG für ein H stehen kann wobei dann zwei Substituenten PG für Schutzgruppen (bevorzugt dann die beiden PG der terminalen Aminogruppe) stehen, oder dass alle drei Substituenten PG für eine Schutzgruppe stehen können, wobei die unterschiedlichen Schutzgruppen gleich oder unterschiedlich sein können. Vorzugsweise stehen in dem Thiazolidin- oder Oxazolidinbaustein der Formel (II) zwei Substituenten PG für ein H und ein Substituent PG (vorzugsweise in der terminalen Aminogruppe) für eine Schutzgruppe.

Geeignete Schutzgruppen und deren Verwendung zum (vorübergehenden) Schutz von bestimmten funktionellen Gruppen sind dem Fachmann bekannt. Bei den für die Zwecke der vorliegenden Erfindung geeigneten Schutzgruppen handelt es sich bevorzugt um gängige Amino-Schutzgruppen. Für die vorliegende Erfindung geeignete Schutzgruppen sind beispielsweise 9-Fluorenylmethoxycarbonyl (Fmoc), tert-Butyloxycarbonyl (Boc), Benzyloxycarbonyl (Cbz), Tosyl (Ts, 4-Methylphenylsulfonyl), Benzyl (Bn), Acetyl (Ac) und Trityl (Trt).

Erfindungsgemäß ist die (bzw. sind die) Schutzgruppe(n) vorzugsweise ausgewählt aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts). Mehr bevorzugt ist/sind die Schutzgruppe(n) ausgewählt aus 9-Fluorenylmethoxycarbonyl (Fmoc), tert-Butyloxycarbonyl (Boc), Benzoyloxycarbonyl (Cbz), Benzyl (Bn) und Trityl (Trt), noch bevorzugter aus 9-Fluorenylmethoxycarbonyl (Fmoc) und tert-Butyloxycarbonyl (Boc). Eine besonders bevorzugte Schutzgruppe ist 9-Fluorenylmethoxycarbonyl (Fmoc).

In einer bevorzugten Ausführungsform des erfindungsgemäßen Thiazolidin- oder Oxazolidinbausteins der Formel (II) ist jener Substituent PG, welcher direkt mit dem Thiazolidin- oder Oxazolidinring verbunden ist, ein H, und die beiden Substituenten PG, welche mit der terminalen Aminogruppe (also nicht direkt mit dem Thiazolidin- oder Oxazolidinring) verbunden sind, ein H und eine Schutzgruppe oder zwei Schutzgruppen, insbesondere bevorzugt ein H und eine Schutzgruppe, sind. Die Schutzgruppen werden dabei wie oben ausgeführt ausgewählt.

In dem erfindungsgemäßen Thiazolidin- oder Oxazolidinbaustein der Formel (II) ist X ausgewählt aus der Gruppe bestehend aus Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl (wobei als Pyrenylmethyl bevorzugt 1-Pyrenylmethyl oder 2-Pyrenylmethyl, insbesondere bevorzugt 1-Pyrenylmethyl, ausgewählt ist). In einer bevorzugten Ausführungsform des erfindungsgemäßen Thiazolidin- oder Oxazolidinbaustein der Formel (II) ist X ausgewählt aus der Gruppe bestehend aus Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl, Propargyl, 1*N*-Methyl-1*H*-indol-3-ylmethyl, 3-Benzothienylmethyl, 1-Naphtylmethyl, 9-Anthracenylmethyl und Pyrenylmethyl (bevorzugt 1-Pyrenylmethyl oder 2-Pyrenylmethyl, insbesondere 1-Pyrenylmethyl).

In einer weiteren bevorzugten Ausführungsform ist X ausgewählt aus der Gruppe bestehend aus *n*-Propyl, 1-Methylethyl, *n*-Butyl, 2-Methylpropyl, 1-Methylpropyl, 1,1-Dimethylethyl, Benzyl, Propargyl, und Pyrenylmethyl (bevorzugt 1-Pyrenylmethyl oder 2-Pyrenylmethyl, insbesondere 1-Pyrenylmethyl). In noch einer weiteren bevorzugten Ausführungsform ist X ausgewählt aus der Gruppe bestehend aus 1-Methylethyl, 2-Methylpropyl, 1-Methylpropyl, 1,1-Dimethylethyl, Benzyl, Propargyl, und Pyrenylmethyl (bevorzugt 1-Pyrenylmethyl oder 2-Pyrenylmethyl, insbesondere 1-Pyrenylmethyl). In einer alternativ bevorzugten Ausführungsform ist X ausgewählt aus der Gruppe bestehend aus *n*-Propyl, 1-Methylethyl, *n-*Butyl, 2-Methylpropyl, 1-Methylpropyl, 1,1-Dimethylethyl, *n*-Pentyl und 3-Methylbutyl.

Im Hinblick auf passende Salze, Solvate und Solvate der Salze für den Thiazolidin- oder Oxazolidin-Baustein der Formel (II) gelten die obigen Ausführungen bezüglich der erfindungsgemäßen Verbindung der Formel (I) analog.

Im erfindungsgemäßen Thiazolidin- oder Oxazolidin-Baustein der Formel (II) weisen das C-Atom, welches mit dem Substituenten X verbunden ist, und das C-Atom in Position 4 des Thiazolidin- oder Oxazolidin-Rings, die gleiche absolute stereochemische Konfiguration auf, wenn Z gleich O ist (also falls es sich um einen Oxazolidin-Ring handelt), und eine entgegengesetzte absolute stereochemische Konfiguration auf, wenn Z gleich S ist (also falls es sich um einen Thiazolidin-Ring handelt) ist. Die Begriffe "*entgegengesetzte absolute stereochemische Konfiguration*" und "*gleiche absolute stereochemische Konfiguration*" sind dabei wie oben im ersten Aspekt definiert zu verstehen. Das heißt beispielsweise, wenn ein erfindungsgemäßer Thiazolidin- oder Oxazolidin-Baustein einen Oxazolidin-Ring aufweist und dieser in Position 4 eine R-Konfiguration aufweist, auch das C-Atom, welches mit dem Substituenten X verbunden ist, eine R-Konfiguration aufweist, bzw. wenn ein erfindungsgemäßer Thiazolidin- oder Oxazolidin-Baustein einen Thiazolidin-Ring aufweist und dieser in Position 4 eine R-Konfiguration aufweist, das C-Atom, welches mit dem Substituenten X verbunden ist, eine S-Konfiguration aufweist.

In spezifischen bevorzugten Ausführungsformen ist der erfindungsgemäße Thiazolidin- oder Oxazolidin-Baustein der Formel (II) gekennzeichnet durch eine der Formeln II.1 bis II.39:

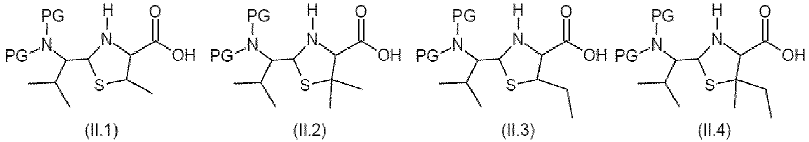

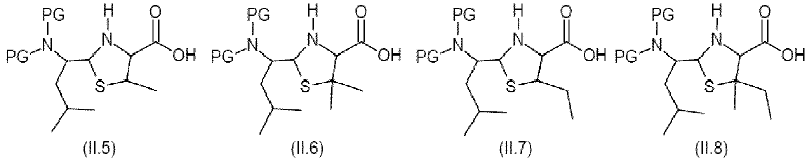

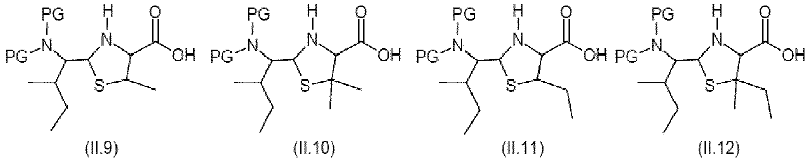

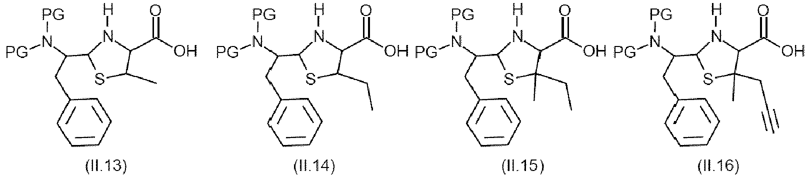

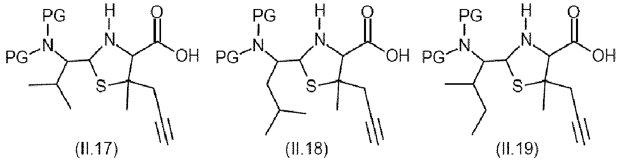

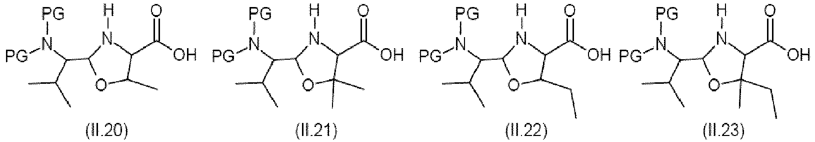

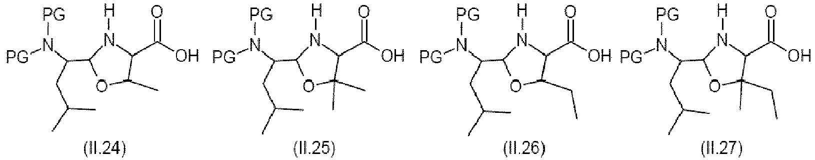

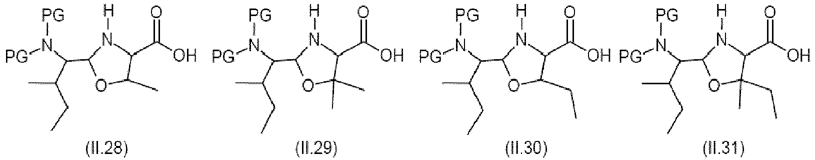

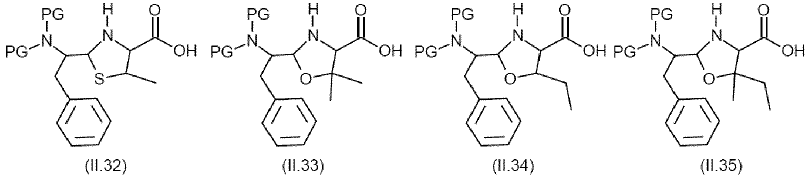

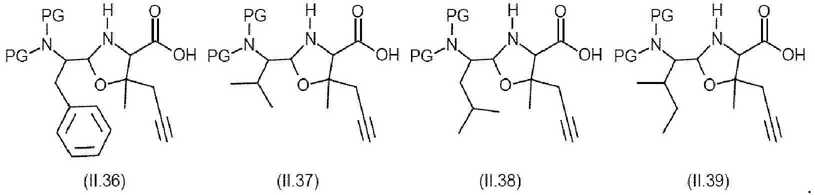

### Verfahren zur Synthese eines Thiazolidin- oder Oxazolidin-Bausteins der Formel (II)

In einem dritten Aspekt ist die vorliegende Erfindung gerichtet auf ein Verfahren zur Synthese eines erfindungsgemäßen Thiazolidin- oder Oxazolidin-Bausteins der Formel (II), umfassend die folgenden Schritte (a) bis (d):
(a) Bereitstellen eines geschützten Aminosäurederivats der Formel (III): 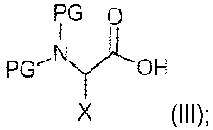
(b) Reduzieren des Aminosäurederivats (III) aus Schritt (a) zum Alkohol der Formel (IV) durch
   b1) in einem ersten Aktivierungsschritt, Aktivieren des Aminosäurederivats der Formel (III), mit einem Aktivierungsreagenz, bevorzugt ausgewählt aus der Gruppe bestehend aus Ethylendimethylamino-propylcarbodiimid (EDC), Dieethylaminoschwefeltrifluorid (DAST), N-Hydroxysuccinimid (NHS), Propanphosphorsäureanhydrid (T3P), Thionylchlorid, Ethylenchloroformiat, Oxalylchlorid, N,O-Dimethylhydroxylamin (Weinreb-Amid), oder 1,1'-Carbonyldiimidazol (CDI), in einem inerten Lösungsmittel; und
   b2) in einem zweiten Schritt, Reduzieren des Aktivierungsprodukts aus Schritt (b1), zu einer Verbindung der Formel (IV): 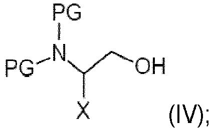
(c) Oxidieren der Verbindung (IV) aus Schritt (b), mit einem Oxidationsmittel, in einem oder mehreren aprotischen Lösungsmitteln, und gegebenenfalls einem Wasserzusatz, zum entsprechenden Aldehyd der Formel (V): 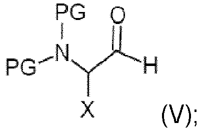
(d) Umsetzen (Kondensation) der Verbindung (V) aus Schritt (c), vorzugsweise in einem Lösungsmittelgemisch aus Wasser und einem polar-protischen Lösungsmittel, vorzugsweise in einem Verhältnis Wasser : polar-protisches Lösungsmittel von höchstens 1 : 1 (v/v) und bei einer Reaktionstemperatur von mindestens 30 °C, mit einer Verbindung der Formel (VI): 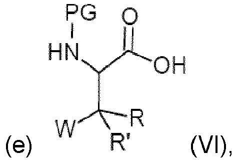 um einen erfindungsgemäßen Thiazolidin- oder Oxazolidin-Baustein der Formel (I) zu erhalten;
   wobei:
   - W gleich SH oder OH ist;
   - R und R' jeweils ausgewählt sind aus der Gruppe bestehend aus H, Methyl, Ethyl, *n-*Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
   - PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
   - X ausgewählt ist aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl;
   unter der Bedingung, dass, wenn Z gleich S ist und X gleich Methyl, Benzyl oder 1*H*-Indol-3-ylmethyl ist, R nicht Methyl ist.

In Schritt (a) des Verfahrens wird als Ausgangssubstanz ein geschütztes Aminosäurederivat der Formel (III) bereitgestellt. Dabei steht PG jeweils für H oder eine Schutzgruppe, wobei einzelne PG gleich oder verschieden sein können, und wobei die Schutzgruppe vorzugsweise ausgewählt ist aus der Gruppe der gängigen Amino-Schutzgruppen, wie beispielsweise 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts).

In einer bevorzugten Ausführungsform steht im geschützten Aminosäurederivat der Formel (III) ein Substituent PG für ein H und ein Substituent PG für eine Schutzgruppe, bevorzugt ausgewählt aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts), mehr bevorzugt ausgewählt aus 9-Fluorenylmethoxycarbonyl (Fmoc), tert-Butyloxycarbonyl (Boc), Benzyloxycarbonyl (Cbz), Benzyl (Bn) und Trityl (Trt), noch bevorzugter ausgewählt aus 9-Fluorenylmethoxycarbonyl (Fmoc), tert-Butyloxycarbonyl (Boc), besonders bevorzugt 9-Fluorenylmethoxycarbonyl (Fmoc). In einer alternativ bevorzugten Ausführungsform stehen im geschützten Aminosäurederivat der Formel (III) beide Substituenten PG für Schutzgruppen, wobei die Schutzgruppen bevorzugt ausgewählt sind aus der obigen Gruppe und wobei die beiden Schutzgruppen gleich oder verschieden sein können, und vorzugsweise gleich sind.

Der Substituent X des geschützten Aminosäurederivats der Formel (III) ist ausgewählt aus der Gruppe bestehend aus Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl (wobei das Pyrenylmethyl bevorzugt 1-Pyrenylmethyl oder 2-Pyrenylmethyl, insbesondere 1-Pyrenylmethyl, ist).

In einer bevorzugten Ausführungsform des Verfahrens ist X ausgewählt aus der Gruppe bestehend aus Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl, Propargyl, 1*N*-Methyl-1*H-*indol-3-ylmethyl, 3-Benzothienylmethyl, 1-Naphtylmethyl, 9-Anthracenylmethyl und Pyrenylmethyl (bevorzugt 1-Pyrenylmethyl oder 2-Pyrenylmethyl, insbesondere 1-Pyrenylmethyl). In einer weiteren bevorzugten Ausführungsform ist X ausgewählt aus der Gruppe bestehend aus *n*-Propyl, 1-Methylethyl, *n*-Butyl, 2-Methylpropyl, 1-Methylpropyl, 1,1-Dimethylethyl, Benzyl, Propargyl, und Pyrenylmethyl (bevorzugt 1-Pyrenylmethyl oder 2-Pyrenylmethyl, insbesondere 1-Pyrenylmethyl). In noch einer weiteren bevorzugten Ausführungsform ist X ausgewählt aus der Gruppe bestehend aus 1-Methylethyl, 2-Methylpropyl, 1-Methylpropyl, 1,1-Dimethylethyl, Benzyl, Propargyl, und Pyrenylmethyl (bevorzugt 1-Pyrenylmethyl oder 2-Pyrenylmethyl, insbesondere 1-Pyrenylmethyl). In einer alternativ bevorzugten Ausführungsform ist X ausgewählt aus der Gruppe bestehend aus *n-*Propyl, 1-Methylethyl, *n*-Butyl, 2-Methylpropyl, 1-Methylpropyl, 1,1-Dimethylethyl, *n*-Pentyl und 3-Methylbutyl.

Die Substituenten R und R' des geschützten Aminosäurederivats der Formel (III) aus Schritt (a) sind jeweils ausgewählt aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist. In einer bevorzugten Ausführungsform dieses Verfahrens sind R und R' jeweils ausgewählt aus der Gruppe bestehend aus H, Methyl, Ethyl, *n*-Propyl, 1-Methylethyl, Benzyl und Propargyl. In einer weiteren bevorzugten Ausführungsform dieses Verfahrens sind R und R' jeweils ausgewählt sind aus der Gruppe bestehend aus H, Methyl, Ethyl, und Propargyl.

Der Substituent W ist gleich SH oder OH. In einer insbesondere bevorzugten Ausführungsform dieses Verfahrens ist W gleich SH. In einer alternativen bevorzugten Ausführungsform dieses Verfahrens ist W gleich OH.

In Schritt (b) des Verfahrens wird das Aminosäurederivat der Formel (III) aus Schritt (a) zum Alkohol der Formel (IV) reduziert. Geeignete Reaktionsbedingungen für Schritt (b) sind dem Fachmann bekannt. Dabei wird in einem initialen Aktivierungsschritt (b1) zunächst das Aminosäurederivat der Formel (III), mit einem Aktivierungsreagenz, welches bevorzugt ausgewählt wird aus der Gruppe bestehend aus Ethylendimethylamino-propylcarbodiimid (EDC), Dieethylaminoschwefeltrifluorid (DAST), N-Hydroxysuccinimid (NHS), Propanphosphorsäureanhydrid (T3P), Thionylchlorid, Ethylenchloroformiat, Oxalylchlorid, N,O-Dimethylhydroxylamin (Weinreb-amid) und 1,1'-Carbonyldiimidazol (CDI), mehr bevorzugt Thionylchlorid, Ethylenchloroformiat, Oxalylchlorid, und 1,1'-carbonyldiimidazol (CDI), besonders bevorzugt 1,1'-Carbonyldiimidazol (CDI), in einem inerten Lösungsmittel aktiviert.

Als inertes Lösungsmittel wird bevorzugt ein inertes Etherlösungsmittel, mehr bevorzugt ein inertes zyklisches Etherlösungsmittel, verwendet. Vorzugsweise wird als inertes Lösungsmittel Diethylether, Diisopropylether, Methyl-*tert*-butylether (MTBE), 1,4-Dioxan, Cyclopentylmethylether, 2-Methyltetrahydrofuran (2-MHF), oder Tetrahydrofuran (THF), mehr bevorzugt 1,4-Dioxan, Cyclopentylmethylether, 2-Methyltetrahydrofuran (2-Me-THF), oder Tetrahydrofuran (THF), noch bevorzugter THF, verwendet. Es können auch Gemische aus mehr als einem inerten Lösungsmittel verwendet werden.

Der initiale Aktivierungsschritt (b1) wird bevorzugt bei 15 bis 25 °C für mindestens 5 min, mehr bevorzugt für 10 bis 30 min, noch bevorzugter für 15 min, durchgeführt. Das Aktivierungsreagenz wird mindestens zu 1 Äquiv., mehr bevorzugt zwischen 1.0 und 2.0 Äquiv., noch bevorzugter zwischen 1.0 und 1.5 Äquiv., noch bevorzugter zu 1.3 Äquiv., zugesetzt.

In einem zweiten Schritt (b2) wird das Aktivierungsprodukt aus Schritt (b1), zu einer Verbindung der Formel (IV) reduziert:

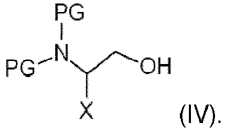

Unter dem Begriff "Aktivierungsprodukts aus Schritt (b1)" ist dabei das Produkt des initialen Aktivierungsschritts (b1) zu verstehen.

Geeignete Reduktionsmittel für Schritt (b2) sind dem Fachmann bekannt. Als Reduktionsmittel kann beispielsweise Pd/C/H₂, Pd/C/Triethylsilan, NiCl₂/NaBH₄, Lithiumaluminumhydrid (LiAlH₄), Diisobutylaluminiumhydrid (DIBAL-H), Natriumcyanoborhydrid (NaBH₃CN) oder Natriumborhydrid (NaBH₄), bevorzugt NaBH₃CN oder NaBH₄, mehr bevorzugt NaBH₄, verwendet werden. Schritt (b2) wird vorzugsweise bei einer Reaktionstemperatur von 0 °C und für mindestens 15 min, mehr bevorzugt 15-30 min, noch bevorzugter 25 min durchgeführt.

In einer bevorzugten Ausführungsform wird in Schritt (b2) das Aktivierungsprodukt aus Schritt (b1) unter Verwendung von NaBH₃CN oder NaBH₄, bevorzugt NaBH₄, bei 0 °C für mindestens 15 min, mehr bevorzugt 15 bis 30 min, noch bevorzugter für 25 min, reduziert.

In Schritt (c) des Verfahrens wird Verbindung (IV) aus Schritt (b) mit einem Oxidationsmittel, in einem aprotischen Lösungsmittel, und gegebenenfalls einem Wasserzusatz, zum entsprechenden Aldehyd der Formel (V) oxidiert:

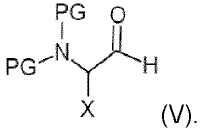

Geeignete Oxidationsmittel und Reaktionsbedingungen sind dem Fachmann bekannt. Geeignete Oxidationsmittel sind beispielsweise Pyridiniumchlorochromat (PCC, CrO₃/Pyridin), Tetrapropylammoniumperruthenat/*N*-Methylmorpholin-*N*-Oxid (TPAP/NMO), Tetramethylpiperidinyloxyl/Natriumhypochlorit (TEMPO/NaOCl oder Oxone), Oxalylchlorid/DMSO/NEt₃ (Swern Oxidation) und Dess-Martin-Periodinan (DMP). Bevorzugt wird als Oxidationsmittel Oxalylchlorid/DMSO/NEt₃ oder DMP, mehr bevorzug DMP, gewählt.

Aprotische Lösungsmittel sind dem Fachmann bekannt. Es können für Schritt (c) auch Gemische von mehr als einem aprotischen Lösungsmittel verwendet werden. Als aprotisches Lösungsmittel kann beispielsweise Ethylacetat, Aceton, Toluol, THF, Chloroform, und/oder Dichlormethan (DCM) gewählt werden. Bevorzugte aprotische Lösungsmittel sind beispielsweise Toluol, THF, Chloroform und DCM. Besonders bevorzugt ist DCM.

Das Oxidationsmittel kann beispielsweise zwischen 1.0 bis 2.0 Äquiv., bevorzugt zwischen 1.0 bis 1.5 Äquiv., mehr bevorzugt zwischen 1.4 bis 1.5 Äquiv., eingesetzt werden. Zur Reaktion kann ein Wasserzusatz hinzugegebenen werden, bevorzugt zwischen 1.0 bis 1.5 Äquiv., mehr bevorzugt 1.1 Äquiv. Wie in Tetrahedron Lett., 2000, 41, 1359-1362 und J. Org. Chem., 1994, 59, 7549-7552 beschrieben, begünstigt und beschleunigt ein Wasserzusatz von beispielsweise einem 1 Äquiv. H₂O die Umsetzung.

Bevorzugt wird das Oxidationsmittel und der optionale Wasserzusatz über die ersten 15 bis 90 min der Reaktion, mehr bevorzugt über die ersten 30 bis 60 min zugegeben, wobei die Reaktionszeit bevorzugt 1 bis 40 Stunden, mehr bevorzugt 2 bis 24 Stunden, noch bevorzugter 5 bis 16 Stunden beträgt. Schritt (c) wird bevorzugt bei einer Reaktionstemperatur von mindestens 10 °C, mehr bevorzugt bei 15 bis 30 °C, noch bevorzugter bei 15 bis 25 °C durchgeführt.

Das Oxidationsmittel wird bevorzugt zwischen 1.0 bis 2.0 Äquiv., mehr bevorzugt zwischen 1.0 bis 1.5 Äquiv., mehr bevorzugt zwischen 1.4 bis 1.5 Äquiv. eingesetzt und beispielsweise über die ersten 30 min, bevorzugt die ersten 15 min, mehr bevorzugt über die ersten 10 min der Reaktion zum Reaktionsgemisch gegeben. Zur Reaktion wird vorzugsweise Wasser, bevorzugt zwischen 1.0 bis 1.5 Äquiv., mehr bevorzugt 1.1 Äquiv. über die ersten 15 bis 90 min der Reaktion, mehr bevorzugt über die ersten 30 bis 60 min zugegeben, wobei die Reaktionszeit 1 bis 40 Stunden, mehr bevorzugt 2 bis 24 Stunden, mehr bevorzugt 5 bis 16 Stunden beträgt. Die Oxidation wird bevorzugt bei einer Reaktionstemperatur von mindestens 10 °C, mehr bevorzugt bei 15 bis 30 °C, mehr bevorzugt bei 15 bis 25 °C durchgeführt.

Im finalen Schritt (d) wird schließlich die Verbindung (V) aus Schritt (c), vorzugsweise in einem Lösungsmittelgemisch aus Wasser und einem polar-protischen Lösungsmittel, mit einer Verbindung der Formel (VI): 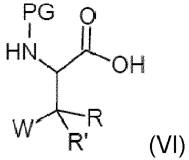 umgesetzt (Kondensation), um einen erfindungsgemäßen Thiazolidin- oder Oxazolidin-Baustein der Formel (II) zu erhalten.

Geeignete polar-protische Lösungsmittel für Schritt (d) sind beispielsweise Ameisensäure, Essigsäure, Ethanol, Isopropanol oder Methanol, bevorzugt Ethanol, Isopropanol oder Methanol, mehr bevorzugt Methanol. Falls ein Lösungsmittelgemisch aus Wasser und einem polar-protischen Lösungsmittel verwendet wird beträgt das Verhältnis Wasser : polar-protisches Lösungsmittel in dem Lösungsmittelgemisch bevorzugt ≤ 1 : 1 (v/v), mehr bevorzugt 1 : 1 bis 1 : 3 (v/v), noch bevorzugter 1 : 2 (v/v).

Die Reaktionstemperatur in Schritt (d) beträgt bevorzugt mindestens 30 °C, mehr bevorzugt mindestens 50 °C, noch bevorzugter zwischen 55 °C bis 75 °C, noch bevorzugter zwischen 55 °C bis 65 °C. Die Reaktionszeit beträgt bevorzugt mindestens 6 Stunden, mehr bevorzugt mindestens 15 Stunden, noch bevorzugter zwischen 15 bis 30 Stunden, insbesondere bevorzugt 24 Stunden.

In Schritt (d) kann das Reaktionsprodukt aus der Umsetzung abschließend beispielsweise filtriert werden, bevorzugt über eine Pore 4 Fritte, und dann bevorzugt mindestens mit dem 5-fachen Überschuss, mehr bevorzugt mit dem 10-fachen Überschuss an Wasser, relativ zum Reaktionslösungsmittelvolumen, gewaschen werden.

In einer bevorzugten Ausführungsform wird in Schritt (d) ein Lösungsmittelgemisch aus Wasser und einem polar-protischen Lösungsmittel verwendet, wobei das Verhältnis von Wasser zu polar-protischem Lösungsmittel ≤ 1 : 1 (v/v) beträgt; ist das polar-protische Lösungsmittel Ameisensäure, Essigsäure, Ethanol, Isopropanol oder Methanol, mehr bevorzugt Ethanol, Isopropanol oder Methanol, mehr bevorzugt Methanol; und/oder wird die Reaktion bei mindestens 50 °C, mehr bevorzugt zwischen 55 °C bis 75 °C durchgeführt, und/oder für mindestens 6 Stunden, mehr bevorzugt zwischen 15 bis 30 Stunden.

In Bezug auf die Substituenten und weiteren Parameter der so hergestellten Thiazolidin- oder Oxazolidinbauteins der Formel (II) und dessen Synthesevorstufen gilt das unter dem zweiten erfindungsgemäßen Aspekt beschriebene entsprechend.

### Verfahren zur Festphasen-Peptidsynthese der erfindungsgemäßen zyklischen Verbindung (I)

In einem vierten Aspekt ist die vorliegende Erfindung gerichtet auf ein Verfahren zur Festphasen-Peptidsynthese einer erfindungsgemäßen zyklischen Verbindung der Formel (I), umfassend die folgenden Schritte (i) bis (iv):
(i) Bereitstellen eines, über seine terminale Carboxylgruppe an eine feste Phase gebundenen,
   - Aminosäurederivats der Formel (VII): 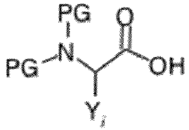 (VII), wobei *i* gleich 1 bis 5 ist;
   - eines linearen Peptids umfassend/bestehend aus zwei, drei, vier, oder fünf Aminosäurederivaten der Formel (VII); oder
   - eines Thiazolidin- oder Oxazolidin-Bausteins der Formel (II) wie in Bezug auf den zweiten Aspekt der vorliegenden Erfindung detailliert beschrieben;
   wobei die terminale Aminogruppe durch mindestens eine Schutzgruppe geschützt ist;
(ii) ausgehend von dem Aminosäurederivat der Formel (VII), dem Peptid, bzw. dem Thiazolidin- oder Oxazolidin-Baustein aus Schritt (i), durch Durchführen einer oder mehrerer konsekutiver Kupplungsreaktionen, unter jeweiligem Anfügen
   - eines oder mehrerer Aminosäurederivate der Formel (VII),
   - eines oder mehrerer linearer Peptide umfassend/bestehend aus zwei bis fünf Aminosäurederivaten der Formel (VII), oder
   - eines Thiazolidin- oder Oxazolidin-Bausteins der Formel (II) wie in Bezug auf den zweiten Aspekt der vorliegenden Erfindung detailliert beschrieben,
   wobei die terminale Aminogruppe durch mindestens eine Schutzgruppe geschützt ist,
   schrittweise Festphasensynthese einer linearen, an die feste Phase gebundenen, Verbindung, umfassend/bestehend aus fünf Aminosäurederivaten der Formel (VII), wobei i gleich 1 bis 5 ist, und ein Thiazolidin- oder Oxazolidin-Baustein der Formel (II), wie er in Bezug auf den zweiten Aspekt der vorliegenden Erfindung bereits detailliert beschrieben wurde;
(iii) Entfernen der Schutzgruppen ("Entschützen") des linearen, an die feste Phase gebundenen, Produkts aus Schritt (ii), und Abspalten von der festen Phase unter Bildung einer linearen, nicht an die feste Phase gebundenen, Verbindung; und
(iv) Makrozyklisierung der linearen, nicht an die feste Phase gebundenen, Verbindung aus Schritt (iii), vorzugsweise durch Makrolaktamisierung, wodurch die erfindungsgemäße zyklische Verbindung der Formel (I) erhalten wird;
wobei:
- X und Y₁ bis Y₅ jeweils ausgewählt sind aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N-*Methyl-1*H*-indol-3-ylmethyl, 3-Benzothienylmethyl, 1-Naphtylmethyl, 9-Anthracenylmethyl und Pyrenylmethyl;
- Z gleich O oder S ist;
- PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
- R und R' jeweils ausgewählt sind aus der Gruppe bestehend aus H, Methyl, Ethyl, *n-*Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
unter der Bedingung, dass, wenn X, Y₁, Y₄ und Y₅ gleich 1-Methylethyl sind, Y₂ gleich 1*H-*Indol-3-ylmethyl ist, Y₃ gleich 2-Methylpropyl ist, R gleich H ist und R' gleich Methyl ist, Z nicht O ist.

Das erfindungsgemäße Verfahren zur Festphasen-Peptidsynthese einer erfindungsgemäßen zyklischen Verbindung der Formel (I) hat gegenüber anderen aus dem Stand der Technik bekannten Verfahren zur Herstellung von zyklischen Peptidderivaten umfassend einen Aminosäurederivat-Baustein mit einem Thiazolidin- oder Oxazolidinring, den deutlichen Vorteil, dass durch die Verwendung der erfindungsgemäßen Thiazolidin- oder Oxazolidinbausteins gemäß Formel (II), als konkreter Baustein in der schrittweisen Festphasen-Peptidsynthese, die Synthese der zyklischen Verbindung, bis zur Makrozyklisierung, gebunden an eine feste Phase durchgeführt werden kann.

Die neue Syntheseroute basiert auf der für den ersten oder zweiten Schritt notwendigen Herstellung bisher unbekannter Alkyl- Aryl- oder Alkinyl-Heterozyklen-Aminosäurederivate, den neuen Thiazolidin- und Oxazolidinbausteinen der Formel (II), welche sich insbesondere durch eine hervorragende Löslichkeit und Stabilität in organischen Lösungsmitteln und damit verbundene überlegene Kupplungseigenschaften in der Festphasensynthese auszeichnen.

Die Synthese solcher Thiazolidin- und Oxazolidinbausteine kann damit erstmals vorteilhaft in die effiziente Methode der Festphasen-Peptidsynthese eingebracht werden. Dies erlaubt eine einfache, kontrollierte und äußerst effiziente Synthese der neuen zyklischen Verbindungen der Formel (I), durch Festphasen-Peptidsynthese über eine lineare Vorstufe (d.h. über ein lineares an die feste Phase gebundenes Peptidderivat), und ein einfacheres Hochskalieren. Nach etabliertem Prinzip kann die lineare Vorstufe abschließend im letzten Schritt zur zyklischen Verbindung unter optimierten Bedingungen zyklisiert und in optimierten Waschschritten mit hohem Reinheitsgrad (>90% Reinheit) und hoher Ausbeute isoliert werden.

Trotz der vielen synthetischen Risikofaktoren, wie Razemisierung, Tautomerisierung, Spaltung oder andere Nebenreaktionen bei der Entschützung von Zwischenprodukten und dergleichen, bei der Synthese eines komplexen Moleküls mit vielen möglichen Isomeren, wie der erfindungsgemäßen zyklischen Verbindung der Formel (I), ist es gelungen, ein Herstellungsverfahren für die Festphasensynthese zu finden, das es erlaubt, die zyklische Verbindung der Formel (I) in hoher Ausbeute, Reinheit und/oder der erforderlichen stereoisomeren Reinheit herzustellen, vorzugsweise alle drei. Auf der Grundlage dieses neuen Syntheseweges ist die Synthese großer Mengen möglich bzw. wird die Herstellung von Produkten im industriellen Maßstab ermöglicht, die vorzugsweise die Menge der Nebenprodukte reduziert und die Ausbeute verbessert.

Das erfindungsgemäße Verfahren zur Festphasen-Peptidsynthese einer erfindungsgemäßen zyklischen Verbindung der Formel (I) umfasst in Schritt (i) zunächst das Bereitstellen eines, über die terminale Carboxylgruppe an eine feste Phase gebundenen,
- Aminosäurederivats der Formel (VII): 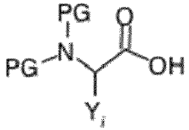 (VII), wobei *i* gleich 1 bis 5 ist;
- eines linearen Peptids umfassend/bestehend aus zwei, drei, vier, oder fünf Aminosäurederivaten der Formel (VII); oder
- eines Thiazolidin- oder Oxazolidin-Bausteins der Formel (II), wie er bereits in Bezug auf den zweiten Aspekt der vorliegenden Erfindung detailliert beschrieben wurde.

Hinsichtlich des Aminosäurederivats der Formel (VII) sind Y₁ bis Y₅ (d.h. Y*ᵢ* mit i = 1-5) jeweils ausgewählt aus der Gruppe bestehend aus Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl (bevorzugt 1-Pyrenylmethyl oder 2-Pyrenylmethyl, insbesondere 1-Pyrenylmethyl). Hinsichtlich der bevorzugten Ausführungsformen der Substituenten Y₁ bis Y₅ gilt das bereits im ersten erfindungsgemäßen Aspekt Beschriebene entsprechend.

Hinsichtlich des Thiazolidin- oder Oxazolidin-Bausteins der Formel (II), gilt das was bereits in Bezug auf den zweiten Aspekt der vorliegenden Erfindung im Detail beschrieben wurde.

Hinsichtlich des linearen Peptids bestehend aus zwei, drei, vier, oder fünf Aminosäurederivaten der Formel (VII) versteht es sich von selbst, dass die Substituenten Y₁ bis Y₅ der einzelnen Aminosäurederivaten im Peptid untereinander nicht gleich sind (das heißt, dass nur ein Aminosäurederivat mit Substituent Y₁, nur ein Aminosäurederivat mit Y₂, etc. im linearen Peptid vorhanden ist, wobei die einzelnen Substituenten Y₁, Y₂, etc., wie bereits beschrieben aber für gleiche oder unterschiedliche Reste stehen können) und auch in ihrer Reihenfolge so angeordnet sind, dass, bei Durchführen des erfindungsgemäßen Verfahrens, mit Hilfe des Peptids als Baustein, schlussendlich eine erfindungsgemäße zyklische Verbindung der Formel (I) (das heißt mit der richtigen Anordnung/Abfolge der Substituenten X und Y₁-Y₅) synthetisiert werden kann. Vorzugsweise ist das lineare Peptid bestehend aus zwei, drei, vier, oder fünf Aminosäurederivaten der Formel (VII) auch durch Festphasen-Peptidsynthese hergestellt worden.

Die terminale Aminogruppe des Aminosäurederivats der Formel (VII), des Peptids oder des Thiazolidin- oder Oxazolidin-Bausteins in Schritt (i) ist durch mindestens eine Schutzgruppe geschützt, vorzugsweise durch genau eine Schutzgruppe geschützt (d.h. die terminale Aminogruppe weist noch eine N-H Bindung auf). Geeignete Schutzgruppen und deren Verwendung zum (vorübergehenden) Schutz von bestimmten funktionellen Gruppen, insbesondere in Aminosäure- und Peptidderivaten, sind dem Fachmann bekannt.

Bei den für die Zwecke der vorliegenden Erfindung geeigneten Schutzgruppen handelt es sich bevorzugt um gängige Amino-Schutzgruppen. Erfindungsgemäß ist die mindestens eine Schutzgruppe vorzugsweise ausgewählt aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts). Mehr bevorzugt ist/sind die Schutzgruppe(n) ausgewählt aus 9-Fluorenylmethoxycarbonyl (Fmoc), tert-Butyloxycarbonyl (Boc), Benzoyloxycarbonyl (Cbz), Benzyl (Bn) und Trityl (Trt), noch bevorzugter 9-Fluorenylmethoxycarbonyl (Fmoc) und tert-Butyloxycarbonyl (Boc). Eine besonders bevorzugte Schutzgruppe ist 9-Fluorenylmethoxycarbonyl (Fmoc).

Geeignete feste Phasen sind beispielsweise herkömmliche feste Phasen die dem Fachmann bekannt sind. Bei der festen Phase handelt es sich vorzugsweise um einen Harz. Die feste Phase ist vorzugsweise auszuwählen aus der Gruppe bestehend aus der Divinylbenzyl (DVB) verknüpften Polystyren Harzen und Polyethylenglycol (PEG)/Polystyren basierten Mischharzen, wobei die Polyethylenglycol (PEG)/Polystyren basierten Mischharze besonders bevorzug sind. Die säurelabile Verknüpfungskomponente (Linker) zwischen fester Phase und Aminosäurederivat der Formel (VII) / linearem Peptid / Thiazolidin- oder Oxazolidin-Baustein wird bevorzugt ausgewählt aus der Gruppe bestehend aus Trityl-basierten Linkern (TRT), Rink-Amid Linkern (RAM), Phenylhydroxybenzyl-basierten Linkern (PHB), und Hydroxymethylphenoxyacetat-basierten Linkern, mehr bevorzugt aus der Gruppe bestehend aus Rink-Amid Linkern (RAM), Phenylhydroxybenzyl-basierten Linkern (PHB), und Hydroxymethylphenoxyacetat-basierten Linkern, noch bevorzugter aus der Gruppe bestehend aus Phenylhydroxybenzyl-basierten Linkern (PHB), und Hydroxymethylphenoxyacetat-basierten Linkern. Besonders bevorzugt sind Hydroxymethylphenoxyacetat-basierte Linker.

In einer bevorzugten Ausführungsform wird in Schritt (i) ein lineares Peptid bestehend aus zwei bis fünf, mehr bevorzugt drei bis fünf, noch bevorzugter vier bis fünf, besonders bevorzugt fünf, Aminosäurederivaten der Formel (VII), wobei die terminale Aminogruppe durch mindestens eine (vorzugweise durch zwei) Schutzgruppe(n) geschützt ist, bereitgestellt.

In Schritt (ii) des erfindungsgemäßen Verfahrens zur Festphasen-Peptidsynthese einer erfindungsgemäßen zyklischen Verbindung der Formel (I) erfolgt ausgehend von dem Aminosäurederivat der Formel (VII), dem Peptid, bzw. dem Thiazolidin- oder Oxazolidin-Baustein aus Schritt (i), durch Durchführen einer oder mehrerer konsekutiver Kupplungsreaktionen, unter jeweilgem Anfügen
- eines oder mehrerer Aminosäurederivate der Formel (VII)
- eines oder mehrerer linearer Peptide umfassend/bestehen aus zwei bis fünf Aminosäurederivaten der Formel (VII) (aber keine anderen Aminosäurederivate), oder
- eines Thiazolidin- oder Oxazolidin-Bausteins, wie in Bezug auf den zweiten Aspekt der vorliegenden Erfindung bereits detailliert beschrieben,
wobei die terminale Aminogruppe durch mindestens eine Schutzgruppe geschützt ist (N-terminale Schutzgruppe, wobei vorzugsweise genau ein H in der terminalen Aminogruppe durch eine Schutzgruppe ersetzt ist aber das zweite H nicht),
die schrittweise Festphasensynthese einer linearen, an die feste Phase gebundenen, Verbindung, umfassend fünf Aminosäurederivate der Formel (VII), wobei *i* gleich 1 bis 5 ist, und ein Thiazolidin- oder Oxazolidin-Baustein, wie er in Bezug auf den zweiten Aspekt der vorliegenden Erfindung bereits detailliert beschrieben wurde.

Das heißt es werden so viele Kupplungsreaktionen (Kupplungsschritte) durchgeführt - bei mehr als einer Kupplungsreaktion unter Verwendung des Produkts der vorhergehende Kupplungsreaktion - bis das Produkt eine lineare, an die feste Phase gebundene, Verbindung, umfassend fünf Aminosäurederivate der Formel (VII), wobei i gleich 1 bis 5 ist, und einen Thiazolidin- oder Oxazolidin-Baustein, ist. Hinsichtlich der Substituenten (X, Y₁ bis Y₅, PG, etc.) gilt das in Schritt (i) Beschriebene.

Es versteht sich dabei von selbst, dass die relative Anordnung der Aminosäurederivate der Formel (VII) und des Thiazolidin- oder Oxazolidin-Bausteins zueinander in der linearen, an die feste Phase gebundenen, Verbindung so gewählt wird, dass die Substituenten X, Y₁ bis Y₅ der zyklischen Verbindung, untereinander nicht gleich sind, und nach Abschluss des erfindungsgemäßen Verfahren in ihrer Reihenfolge so angeordnet sind, dass es der Anordnung in der erfindungsgemäßen zyklischen Verbindung der Formel (I) (das heißt mit der richtigen Anordnung der Substituenten X und Y₁-Y₅) entspricht.

Es versteht sich dabei auch von selbst, dass die einzelnen Aminosäurederivate der Formel (VII), das oder die lineare(n) Peptid(e) und der Thiazolidin- oder Oxazolidin-Baustein so zu wählen sind, dass die für die erfindungsgemäße zyklische Verbindung der Formel (I) vorgegebene alternierender absolute stereochemischer Konfiguration eingehalten wird. Dies ist dem Fachmann durch sein allgemeines Fachwissen auch einfach möglich.

Der Begriff "Kupplungsreaktion" oder "Kupplungsschritt" in diesem Zusammenhang ist dem Fachmann bekannt. Eine Kupplungsreaktion umfasst dabei vorzugsweise zwei Teilschritte, 1) das Entfernen mindestens einen N-terminalen Schutzgruppe des an die feste Phase gebundenen Aminosäurederivats der Formel (VII), linearen Peptids, oder Thiazolidin- oder Oxazolidin-Bausteins ("Entschützen"), und 2) die eigentliche Kupplungsreaktion mit einem Aminosäurederivat der Formel (VII), einem linearem Peptid, oder Thiazolidin- oder Oxazolidin-Baustein, das bzw. der selbst auch N-terminal durch mindestens eine Schutzgruppe geschützt ist, aber nicht an die feste Phase gebunden ist ("Kondensation").

Das Entfernen der N-terminalen Schutzgruppe ("Entschützen") und die Kupplungsreaktion können dabei auf dem Fachmann bekannte Art und Weise durchgeführt werden. Vorzugsweise wird das Entschützen unter Einsatz bekannter Entschützungs-Bedingungen durchgeführt, beispielsweise für eine Fmoc Schutzgruppe bevorzugt unter Verwendung von DBU, Morpholin und/oder Piperidin in DMF, mehr bevorzugt unter Verwendung von DBU, Morpholin und Piperidin in DMF.

Die Amidbindung aufbauende Kupplungsreaktion wird bevorzugt bei 15 bis 25 °C durchgeführt, bevorzugt über einen Zeitraum von 30 bis 45 min. Die Kupplungsreaktion wird bevorzugt in einem oder mehreren polar-aprotischen Lösungsmittel wie Dimethvsulfoxid (DMSO), Acentonitril (ACN), Dichlormethan (DCM), N-Methylpyrrolidon (NMP), oder Dimethylformamid (DMF), mehr bevorzugt N-Methylpyrrolidon (NMP) oder Dimethylformamid (DMF), noch bevorzugter DMF, unter Verwendung eines oder mehrerer Kupplungsreagenzien, durchgeführt.

Bevorzugte Kupplungsreagenzien sind dabei Carbodiimid-basierte Kupplungsreagenzien (EDC, DIC), Uronium-basierte Kupplungsreagenzien (HATU, HBTU, TBTU), Phosphonium-basierte Kupplungsreagenzien (BOP, PyBOP, PyBrOP), eine Kombination aus Uronium- und Phosphonium-basierten Kupplungsreagenzien (HATU/PyBOP) und DIPEA (N-Ethyl-N-(propan-2-yl)propan-2-amin), und NMM (4-Methylmorpholin), mehr bevorzugt Uronium-basierte Kupplungsreagenzien (HATU, HBTU, TBTU), Phosphonium-basierte Kupplungsreagenzien (BOP, PyBOP, PyBrOP), eine Kombination aus Uronium- und Phosphonium- basierten Kupplungsreagenzien (HATU/PyBOP) und DIPEA, und NMM, noch bevorzugter Phosphonium-basierte Kupplungsreagenzien (BOP, PyBOP, PyBrOP), eine Kombination aus Uronium- und Phosphonium- basierten Kupplungsreagenzien (HATU/PyBOP) und DIPEA, und NMM, noch bevorzugter eine Kombination aus Uronium- und Phosphonium- basierten Kupplungsreagenzien (HATU/PyBOP) und DIPEA, und NMM. Besonders bevorzugt ist NMM. In einer bevorzugten Ausführungsform wird für die Kupplungsreaktion ein Kupplungsadditiv eingesetzt, wobei es sich bei dem Kupplungsadditiv bevorzugt um Hydroxyiminocyanessigsäureethylester (Oxyma), oder Benzotriazol-1-ol (HOBt), mehr bevorzugt um Benzotriazol-1-ol (HOBt), handelt.

Durch eine Reihe alternierender Entschützungs- und Kupplungsreaktionen kann somit eine lineare, an die feste Phase gebundene, Verbindung mit vorgegebener Aminosäurederivat-Abfolge, und vorgegebener alternierender absoluter stereochemischer Konfiguration der C-Atome die direkt mit den Substituenten X und Y₁ bis Y₅ verbunden sind, synthetisiert werden.

In einer bevorzugten Ausführungsform sind, bzw. ist, in Schritt (ii) die anzufügenden (d.h. nicht das Aminosäurederivat der Formel (VII), das Peptid oder der Thiazolidin- oder Oxazolidin-Baustein aus Schritt (i), sondern die neu hinzukommenden) Aminosäurederivate der Formel (VII) oder Peptide, oder der anzufügende Thiazolidin- oder Oxazolidin-Baustein nicht an eine feste Phase gebunden. In einer weiteren bevorzugten Ausführungsform wird in jeder der einen oder mehreren Kupplungsreaktionen in Schritt (ii), jeweils zunächst die mindestens eine Schutzgruppe von dem Aminosäurederivat der Formel (VII), dem Peptid, bzw. dem Thiazolidin- oder Oxazolidin-Baustein aus Schritt (i), entfernt, und dann die Kupplung mit dem, nicht an eine feste Phase gebunden, Aminosäurederivat der Formel (VII), dem Peptid, bzw. dem Thiazolidin- oder Oxazolidin-Baustein durchgeführt.

In einer bevorzugten Ausführungsform wird in Schritt (ii) für die Kupplungsreaktionen jeweils ein Aminosäurederivat der Formel (VII) oder ein Thiazolidin- oder Oxazolidin-Baustein (nicht aber ein Peptid) verwendet, d.h. wird pro Kupplungsschritt nur ein Aminosäurederivat der Formel (VII) oder ein Thiazolidin- oder Oxazolidin-Baustein (falls bis dahin noch kein Thiazolidin- oder Oxazolidin-Baustein verwendet wurde), in die wachsende lineare, an die feste Phase gebundene, Verbindung eingebaut.

In einer bevorzugten Ausführungsform des Verfahrens zur Festphasen-Peptidsynthese der erfindungsgemäßen zyklischen Verbindungen, ist, falls in Schritt (i) ein Peptid bereitgestellt wird, dieses Peptid durch Festphasen-Peptidsynthese, durch konsekutives Einfügen von Aminosäurederivaten der Formel (VII) mit jeweils alternierender absoluter stereochemischer Konfiguration des C-Atoms das direkt mit dem Substituenten Y₁₋₅ verbunden ist, hergestellt worden.

In der linearen, an die feste Phase gebundenen, Verbindung aus Schritt (ii) weisen, unter den C-Atomen, die direkt mit einem Substituenten Y₁₋₅ oder X verbunden sind, jene die entlang des Kettenrückrats der linearen Verbindung, jeweils direkt aufeinander folgen (d.h. die entlang des Kettenrückrats nächstgelegenen) eine alternierender absolute stereochemischer Konfiguration auf; d.h. also, dass die lineare, an die feste Phase gebundene, Verbindung aus Schritt (b) in Bezug auf die C-Atome, die direkt mit einem Substituenten Y₁₋₅ oder X verbunden sind, entlang des Kettenrückrats der linearen Verbindung eine alternierender absolute stereochemischer Konfiguration auf.

Dies bedeutet, dass in der linearen, an die feste Phase gebundenen, Verbindung aus Schritt (ii) jedes C-Atom, das direkt mit einem Substituenten Y₁₋₅ oder X verbunden ist (und nicht der erste oder letzte Substituent entlang des Kettenrückrats der linearen Verbindung ist), gegenüber den auf beiden Seiten dieses C-Atoms entlang des Kettenrückrats der linearen Verbindung nächstgelegenen C-Atome die auch direkt mit einem Substituenten Y₁₋₅ und X verbunden sind, eine entgegengesetzte absolute stereochemische Konfiguration hat.

In einer exemplarischen bevorzugten Ausführungsform des Verfahrens wird in Schritt (ii) zunächst eine erste Kupplungsreaktion durchgeführt. Dabei wird die terminale Aminogruppe des Aminosäurederivats des Peptids oder des Thiazolidin- oder Oxazolidin-Bausteins aus Schritt (i) entfernt ("Entschützen"); und dann in einem zweiten Schritt die "Kupplung" an ein weiteres, nicht an eine feste Phase gebundenes, Aminosäurederivat der Formel (VII), Peptid umfassend (vorzugsweise bestehend aus) zwei bis fünf Aminosäurederivate der Formel (VII) und keine anderen Aminosäurederivate, oder an einen, nicht an eine feste Phase gebundenen, Thiazolidin- oder Oxazolidin-Baustein der Formel (II) wie in Bezug auf den zweiten Aspekt der vorliegenden Erfindung detailliert beschrieben (vorausgesetzt dass in Schritt (i) kein Thiazolidin- oder Oxazolidin-Baustein der Formel (II) verwendet wurde), durchgeführt. Dabei ist die terminale Aminogruppe des weiteren Aminosäurederivats oder Peptids, bzw. des Thiazolidin- oder Oxazolidin-Baustein der Formel (II) vorzugsweise durch mindestens eine, mehr bevorzugt durch genau eine, Schutzgruppe geschützt.

In dieser exemplarischen bevorzugten Ausführungsform wird diese erste Kupplungsreaktion eins, zwei, drei oder vier Mal wiederholt, unter Verwendung des jeweiligen, an die feste Phase gebundenen, Produkts der vorhergehenden Kupplungsreaktion; wodurch schrittweise weitere Aminosäurederivate der Formel (VII), Peptide umfassend ausschließlich Aminosäurederivate der Formel (VII), bzw. ein Thiazolidin- oder Oxazolidin-Baustein der Formel (II) wie in Bezug auf den zweiten Aspekt der vorliegenden Erfindung detailliert beschrieben, vorausgesetzt, dass in den vorherigen Schritten noch kein Thiazolidin- oder Oxazolidin-Baustein der Formel (II) verwendet wurde, jeweils enthaltend in der terminalen Aminogruppe mindestens (ein PG das) eine Schutzgruppe (ist), in die wachsende lineare, an die feste Phase gebundene, Verbindung eingefügt werden; um schließlich eine lineare, an die feste Phase gebundene Verbindung umfassend fünf Aminosäurederivate der Formel (VII), wobei i gleich 1 bis 5 ist, und ein Thiazolidin- oder Oxazolidin-Baustein der Formel (II) zu erhalten.

Es versteht sich von selbst, dass jeweils das weitere nicht an eine feste Phase gebundene Aminosäurederivat der Formel (IV), das C-terminale Aminosäurederivat im weiteren nicht an eine feste Phase gebundenen Peptid, bzw. der nicht an eine feste Phase gebundene Thiazolidin- oder Oxazolidin-Baustein, am C-Atom welches direkt mit dem Substituenten Y₁₋₅, bzw. X verbunden ist, eine absolute stereochemische Konfiguration aufweist die der absoluten stereochemische Konfiguration des C-Atoms, welches im an die feste Phase gebundenen Aminosäurederivat, im N-terminalen Aminosäurederivat des an die feste Phase gebundenen Peptids, bzw. im an die feste Phase gebundenen Thiazolidin- oder Oxazolidin-Baustein direkt mit dem Substituenten Y₁₋₅, bzw. X verbunden ist, entgegensetzt ist. Dies gilt natürlich auch für jede weitere Kupplungsreaktion.

In Schritt (iii) des erfindungsgemäßen Verfahrens zur Festphasen-Peptidsynthese einer erfindungsgemäßen zyklischen Verbindung der Formel (I) erfolgt das Entfernen der Schutzgruppen ("Entschützen") des linearen, an die feste Phase gebundene, Produkts aus Schritt (ii), und Abspalten von der festen Phase unter Bildung einer linearen, nicht an die feste Phase gebundenen, Verbindung. Vorzugsweise weist dieses lineare, an die feste Phase gebundene, Produkt aus Schritt (ii) lediglich am N-Terminus, eine oder zwei Schutzgruppen (vorzugsweise eine Schutzgruppe und eine N-H Bindung) auf, die in Schritt (iii) dann entfernt wird bzw. werden. Das Entfernen der Schutzgruppen und Abspalten von der festen Phase kann beispielsweise mit aus dem Stand der Technik bekannten Reaktionsbedingungen und Reagenzien, beispielsweise simultan aber auch sequenziell, durchgeführt werden.

In einer bevorzugten Ausführungsform des Schritts (iii) erfolgt das Entschützen und Abspalten von der festen Phase mit 0.5 bis 4% (v/v) DBU (1,8-Diazabicyclo[5.4.0]undec-7-en) und 5 bis 15% (v/v) Morpholin in DMF, sowie nachfolgend mit einem Gemisch aus TFA (Trifluoressigsäure), Triisopropylsilan (TIPS) und Wasser, in einem bevorzugten Verhältnis von 90-92,5 : 2,5-5 : 5 (v/v/v).

Schließlich in Schritt (iv) des erfindungsgemäßen Verfahrens zur Festphasen-Peptidsynthese einer erfindungsgemäßen zyklischen Verbindung der Formel (I) erfolgt die Makrozyklisierung der linearen, nicht an die feste Phase gebundenen, Verbindung aus Schritt (iii), vorzugsweise durch Makrolaktamisierung, wodurch die erfindungsgemäße zyklische Verbindung der Formel (I) erhalten wird. Die Makrozyklisierung bzw. bevorzugt Macrolaktamisierung kann beispielsweise mit aus dem Stand der Technik bekannten Reaktionsbedingungen und Reagenzien durchgeführt werden.

Überraschenderweise wurde gefunden, dass durch Makrozyklisierung die Bildung der neuen zyklischen Verbindungen (ohne das übliche Muster von [NH-CH(H oder organische Reste)-C=O-]ₙ), erreicht werden kann, wobei diese zyklischen Verbindungen jeweils einen vollständig intakten beispielsweise alkyl- aryl- oder alkinylsubstituierten Thiazolidin- oder Oxazolidin-Ring mit unterschiedlichen Substitutionsmustern enthält. Die Alkyl- Aryl- oder Alkinyl-tragenden (beispielsweise R=H und R'= Methyl, Ethyl, Propyl, Benzyl, Propargyl; oder R= CH3 und R' = Methyl, Ethyl, Propyl, Benzyl, Propargyl) Thiazolidin- und Oxazolidin-Ringe unterbrechen dadurch das übliche Peptidrückgratmuster.

Die Makrozyklisierung der linearen, nicht an die feste Phase gebundenen, Verbindung aus Schritt (iii) wird bevorzugt bei 15 bis 25 °C, beispielsweise mit EDC/NMM/HOBt, HATU/DIPEA/HOBt, oder HATU/DIPEA/HOAt, bevorzugt HATU/DIPEA/HOBt, oder HATU/DIPEA/HOAt, mehr bevorzugt HATU/DIPEA/HOAt, in einem polar-aprotischen Lösungsmittel (siehe oben), durchgeführt. Die Zyklisierung wird bevorzugt in DMF, Acetonitril und/oder NMP, mehr bevorzugt in DMF, als polar-aprotisches Lösungsmittel durchgeführt. Die Zyklisierung wird vorzugsweise für mindestens 15 Stunden, mehr bevorzugt 15 bis 30 Stunden, noch bevorzugter 24 Stunden durchgeführt. Das Reaktionsgemisch wird vorzugsweise mit Wasser in einem Verhältnis von ≥ 1 : 1 (v/v), mehr bevorzugt ≥ 2 : 1 (v/v), mehr bevorzugt um 3 : 1 (v/v) verdünnt und mit einem, vorzugsweise binären, Gemisch aus einem halogenierten, bspw. chlorierten, Lösungsmittel (beispielsweise DCM oder Chloroform) oder einem Lösungsmittel der Carbonsäureester-Gruppe, und einem Alkohol (bspw. *n-*Butanol), vozugsweise im Verhältnis ≥ 3 : 1 (v/v), mehr bevorzugt ≥ 5 : 1 (v/v), mehr bevorzugt 6 : 1 (v/v), oder bevorzugt mit höheren Alkoholen (bspw. n-Butanol) und/oder Ethern (bevorzugt zyklische Etherlösungsmittel wie THF und 2-Me-THF), d.h. alleine oder als Lösungsmittelgemisch, extrahiert.

Im Hinblick auf die Substituenten und die anderen Parameter der durch das erfindungsgemäße Verfahren zur Festphasen-Peptidsynthese hergestellten erfindungsgemäßen zyklischen Verbindung der Formel (I) und Synthese-Vorstufen gilt das unter dem ersten bis dritten Aspekt der vorliegenden Erfindung Beschriebene entsprechend.

Eine exemplarische bevorzugte Ausführungsform des Verfahrens zur Festphasen-Peptidsynthese der erfindungsgemäßen zyklischen Verbindung der Formel (I), umfasst die folgenden Schritte (i) bis (iv):
(i) Bereitstellen eines geschützten Pentapeptids der Formel (VII): 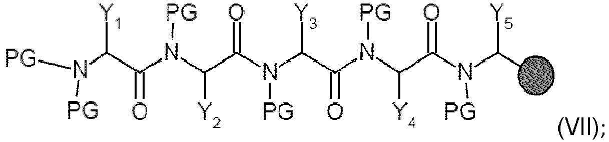 welches über seinen C-Terminus an eine feste Phase gebunden ist; wobei das Pentapeptid durch Festphasenpeptidsynthese, vorzugsweise durch konsekutives Einfügen von Aminosäurederivaten mit jeweils alternierender absoluter stereochemischer Konfiguration des α-Kohlenstoffs (Cα), hergestellt wurde.
(ii) Durchführen einer Kupplungsreaktion, durch Entfernen der Schutzgruppe vom N-Terminus des geschützten Pentapeptids aus Schritt (i) und Koppeln des resultierenden entschützten Pentapeptids an einen Thiazolidin- oder Oxazolidin-Baustein der Formel (II) wie bereits unter dem zweiten Aspekt der vorliegenden Erfindung beschrieben, und Bildung einer Verbindung der Formel (VIII): 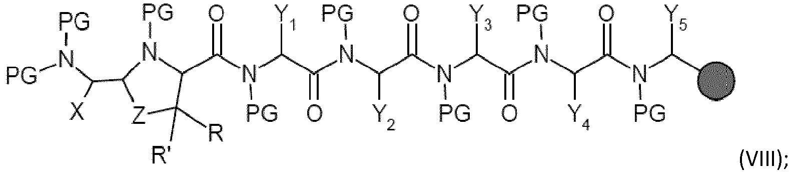 wobei die absolute stereochemische Konfiguration des C-Atoms welches im Thiazolidin- oder Oxazolidin-Baustein direkt mit dem Substituenten X verbunden ist, entgegengesetzt ist zu der absoluten stereochemischen Konfiguration des C-Atoms, welches im N-terminalen Aminosäurederivat des Pentapeptids aus Schritt (i) direkt mit dem Rest Y₁ verbunden ist.
(iii) Entfernen der Schutzgruppen (Entschützen) der Seitenketten der Verbindung (VIII) aus Schritt (ii) und Abspalten der Verbindung (VIII) von der festen Phase unter Bildung einer linearen Verbindung der Formel (IX): 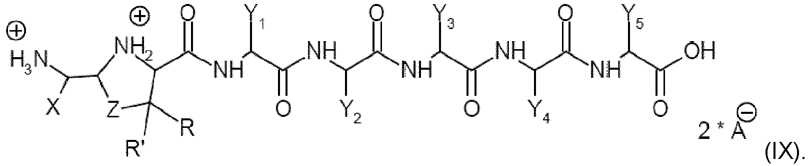
(iv) Makrozyklisierung der linearen Verbindung (IX) aus Schritt (iii), vorzugsweise durch Makrolaktamisierung, um die erfindungsgemäße zyklische Verbindung der Formel (I) zu erhalten (der ausgefüllte dunkelgraue Kreis stellt die feste Phase dar, und A ist ein Anion).

In einer weiteren bevorzugten Ausführungsform des vorliegenden Verfahrens zur Festphasen-Peptidsynthese der erfindungsgemäßen zyklischen Verbindung der Formel (I), sind jeweils lediglich die N-terminalen Aminogruppen, durch Schutzgruppen geschützt, vorzugsweise lediglich durch ein Schutzgruppenmolekül (sekundäre Aminogruppe).

Gemäß einer bevorzugten Ausführungsform des vorliegenden Verfahrens zur Festphasen-Peptidsynthese der erfindungsgemäßen zyklischen Verbindungen der Formel (I) wird der in Schritt (i) oder Schritt (ii) eingesetzte Thiazolidin- oder Oxazolidin-Baustein nach dem Verfahren zur Synthese eines erfindungsgemäßen Thiazolidin- oder Oxazolidin-Bausteins der Formel (II), wie in Bezug auf den dritten Aspekt der vorliegenden Erfindung bereits beschrieben, hergestellt.

### Kosmetische Zubereitung enthaltend zyklische Verbindungen der Formel (I)

In einem fünften Aspekt ist die vorliegende Erfindung gerichtet auf eine kosmetische Zubereitung, enthaltend (a) eine oder mehrere erfindungsgemäße zyklische Verbindungen der Formel (I); und (b) einen oder mehrere kosmetisch akzeptable Träger.

Die eine oder mehreren zyklischen Verbindungen der Formel (I) sind dabei jene, wie sie in den oberen Abschnitten definiert wurden. Vorteilhaft können diese erfindungsgemäßen zyklischen Verbindungen der Formel (I) durch das neue erfindungsgemäße Verfahren zur Festphasen-Peptidsynthese einer zyklischen Verbindung der Formel (I) hergestellt werden. In der erfindungsgemäßen kosmetischen Zubereitung kann eine oder mehrere verschiedene zyklische Verbindungen der Formel (I) enthalten sein. In einer bevorzugten Ausführungsform enthält die erfindungsgemäße kosmetische Zubereitung eine erfindungsgemäße zyklische Verbindung der Formel (I), d.h. lediglich eine konkrete erfindungsgemäße molekulare Ausgestaltung der Formel (I). In einer alternativ bevorzugten Ausführungsform enthält die erfindungsgemäße kosmetische Zubereitung mindestens zwei verschieden erfindungsgemäße zyklische Verbindungen der Formel (I).

Der eine oder die mehreren kosmetisch akzeptablen Träger sind nicht weiter beschränkt. Geeignete kosmetisch akzeptable Träger sind dem Fachmann bekannt. Geeignete kosmetisch akzeptable Träger sind beispielsweise kosmetische Hilfsstoffe, Verdünnungsmittel, Trägerstoffe, Bindemittel, Antihaftmittel, Dispergiermittel und andere kosmetische Zusatzstoffe, wie beispielsweise Konservierungsmittel, Bakterizide, desodorierend wirkende Substanzen, Antitranspirantien, Vitamine, Mittel zum Verhindern des Schäumens, Farbstoffe, Pigmente, Verdickungsmittel, weichmachende Substanzen, anfeuchtende und/oder feuchthaltende Substanzen, Fette, Öle, Wachse, Wasser, Alkohole, Polyole, Polymere, Schaumstabilisatoren, Elektrolyte, UV-Strahlung absorbierende Substanzen, organische Lösungsmittel oder Silikonderivate. Die kosmetische Zubereitung kann optional auch noch weitere kosmetische Wirkstoffe enthalten, die sich vorteilhaft mit den erfindungsgemäßen zyklischen Verbindungen der Formel (I) kombinieren lassen.

In einer bevorzugten Ausführungsform der kosmetischen Zubereitung, beispielsweise für den topischen Einsatz, weisen die darin enthaltenen erfindungsgemäßen zyklischen Verbindungen der Formel (I) bevorzugt eine antimikrobielle, insbesondere eine antivirale, antibakterielle (bakterizide, insbesondere eine nicht-selektive antimikrobielle Wirksamkeit vorzugsweise konkret gegen grampositive Bakterien, jedoch vorzugsweise nicht oder nur minimal gegen gramnegative Bakterien) und/oder antimykotische (fungizide), besonders bevorzugt eine antivirale und/oder nicht-selektive antimikrobielle, Wirksamkeit auf. Außerdem weisen die in der kosmetischen Zubereitung enthaltenen erfindungsgemäßen zyklischen Verbindungen der Formel (I), bevorzugt eine unterstützende Wirkung auf die angeborene Immunantwort, bspw. der Haut, vorzugsweise gegen nicht-kommensale, Mikroorganismen, bspw. nicht-kommensale Bakterien, beispielsweise beim topischen Einsatz auf der Haut, auf.

Die kosmetische Zubereitung liegt dabei vorzugsweise in Form einer dermatologischen Zubereitung zur topischen Anwendung vor. Die dabei verwendeten erfindungsgemäßen zyklischen Verbindungen der Formel (I) weisen bevorzugt eine antimikrobielle, insbesondere eine antivirale, antibakterielle (bakterizide, insbesondere eine nicht-selektive antimikrobielle Wirksamkeit vorzugsweise gegen grampositive Bakterien, vorzugsweise aber nicht gegen gramnegative Bakterien) und/oder antimykotische (fungizide), besonders bevorzugt eine antivirale und/oder eine nicht-selektive antibakterielle, Wirksamkeit auf.

Weiterhin weisen die dabei verwendeten erfindungsgemäßen zyklischen Verbindungen der Formel (I), bevorzugt eine unterstützende Wirkung auf die angeborene Immunantwort, bspw. der Haut, vorzugsweise gegen nicht-kommensale, Mikroorganismen, bspw. nicht-kommensale Bakterien, auf.

### Beispiele

Die folgenden Beispiele erläutern die Erfindung.

### Verwendete Abkürzungen:

Ac₂O, Essigsäureanhydrid; CDI, 1,1'-Carbonyldiimidazol; DBU, 2,3,4,6,7,8,9,9,10-Octahydropyrimido[1,2-a]azepin; DIPEA, *N*-Ethyl-*N*-(propan-2-yl)propan-2-amin; DMP, Dess-Martin-Periodinan; Et₂O, Diethylether; HATU, 1-[Bis(dimethylamino)methylen]-1*H*-1,2,3-triazolo[4,5-b]pyridinium-3-oxid-hexafluorophosphat; HOAt, 3-Hydroxytriazolo[4,5-b]pyridin; HOBt, Benzotriazol-1-ol; IPA, Isopropylalkohol; MHMPA, 3-Methoxy-4-(hydroxymethyl)phenoxyessigsäure; MHK, minimale Hemmkonzentration; *n*-BuOH, *n*-Butanol; NMM, 4-Methylmorpholin; Pra, Propargylglycin; PyBOP, (Benzotriazol-1-yloxy)tripyrrolidino-phosphoniumhexafluoro-phosphat; Pyr, Pyridin; SPPS, Festphasenpeptid-synthese; TFA, Trifluoressigsäure; Thz, Thiazolidin; TIPS, Triisopropylsilan.

### Beispiel 1: Synthese von neuen Thiazolidin- oder Oxazolidin-Bausteinen

### a) Synthese Thiazolidinbaustein

In Kürze, die Synthese wurde in drei Schritten umgesetzt. Zunächst wurde die Carbonsäure in Fmoc-geschützem L-Valin durch Carbonyldiimidazol (CDI) aktiviert und anschließende mit Natriumborhydrat (NaBH₄) zum entsprechenden Alkohol Fmoc-L-Valinol reduziert. Fmoc-L-Valinol wurde anschließend durch Des-Martin-Parodinan (DMP) zum entsprechenden Aldehyd reoxidiert. Schließlich ergab die Kondensation mit 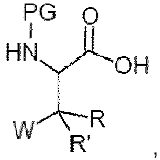 wobei W = SH ist, den gewünschten Thiazolidinbaustein, welcher während der Reaktion vorteilhaft ausfiel und abfiltriert wurde, gebrauchsfertig für bequeme Fmoc-SPPS-Protokolle durch Solubilisierung in DMF.

Im Detail wurde *N*-alpha-(9-Fluorenylmethyloxycarbonyl)-L-valin (5,00 g, 14,75 mmol) in THF (50 mL) gelöst und 1,1'-Carbonyldiimidazol (3,1 g, 19,1 mmol, 1,3 Äquiv.) in einer Portion hinzugefügt. Die Lösung wurde 15 min lang bei Raumtemperatur (RT) gerührt und 10 min lang auf 0 °C abgekühlt. Zu dieser Lösung wurde NaBH₄ (945 mg, 25,0 mmol, 1,7 Äquiv.) in Wasser (15 mL) schnell und gleichmäßig injiziert und 25 min lang bei 0 °C gerührt. Die Reaktion wurde durch Zugabe von 1 N HCl (10 mL) und 50 mL Wasser gequenscht. Das Reaktionsgemisch wurde mit EtOAc (3 x 60 mL) extrahiert. Die kombinierten organischen Extrakte wurden mit gesättigtem NaHCO₃ (3 x 100 mL), und Salzlake (3 x 100 mL) gewaschen und über Na₂SO₄ getrocknet. Die Lösung wurde durch ein dünnes Celite-Pad filtriert, das Lösungsmittel wurde verdampft und vakuumgetrocknet, um den entsprechenden Alkohol N-alpha-(9-Fluorenylmethyloxycarbonyl)-L-valinol als weißen Feststoff zu erhalten.

Das *N*-alpha-(9-Fluorenylmethyloxycarbonyl)-L-valinol wurde in DCM (100 mL) gelöst und in einem Eiswasserbad auf 0 °C abgekühlt. Dess-Martin-Periodinan (DMP, 9,3 g, 22 mmol, 1,5 Äquiv.) wurde portionsweise über 10 min bei 0 °C zugegeben. Nach abgeschlossener DMP-Zugabe wurde das Eiswasserbad entfernt und das Reaktionsgemisch auf Raumtemperatur erwärmt. Während der ersten Stunde wurde Wasser (250 µL, 1,1 Äquiv.) in vier Portionen zugegeben. Die Reaktionsmischung wurde über Nacht bei Raumtemperatur gerührt. Die Reaktionsmischung wurde durch Zugabe von Na₂S₂O₃ (4,5 g) und NaHCO₃ (1,5 g) in Wasser (300 mL) gequenscht und 30 Minuten lang gerührt. Die entstandene Suspension wurde zentrifugiert und die restlichen Feststoffe wurden abfiltriert. Die Phasen wurden getrennt und die organische Phase wurde mit 1N Na₂S₂O₃ (5 x 75 mL), KHSO₄ 10% (v/v, 3 x 75 mL), gesättigter NaHCO₃ (3 x 75 mL), gesättigter NaCl (3 x 75 mL), gewaschen über Na₂SO₄ getrocknet und bis zur Trockne verdampft, um das entsprechende Aldehyd *N*-alpha-(9-Fluorenylmethyloxycarbonyl)-L-valinal als beige-farbenen Feststoff zu erhalten.

Das *N*-alpha-(9-Fluorenylmethyloxycarbonyl)-L-valinal und 3 Äquiv. der Verbindung der Formel 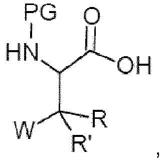 wobei W = SH ist, wurden in MeOH/H₂O (2 : 1, 100 mL + 50 mL) suspendiert. Die resultierende Suspension wurde auf 65 °C erhitzt und 20 Stunden gerührt. Während der Reaktion bildete sich ein weißer Niederschlag. Das Methanol wurde im Vakuum entfernt, Wasser (100 mL) hinzugefügt und die Suspension über Nacht bei 4 °C gelagert. Der weiße Niederschlag wurde filtriert und mit einem Überschuss an Wasser (1 L) gewaschen. Restwasser im nassen Feststoff wurde verdampft und 48 Stunden lang vakuumgetrocknet. Der resultierende Feststoff wurde in Toluol/Pentan (3 : 1; 45 mL + 15 mL), CHCl₃/THF (1 : 1; 30 mL + 30 mL), IPA/Aceton/Pentan (1 : 1 : 1; 20 mL + 20 mL+ 20 mL) aufgeschlämmt und unter Vakuum getrocknet, um den Thiazolidinbaustein als weißen Feststoff zu erhalten.

### Beispiel 2: Festphasen-Peptidsynthese (SPPS) von neuen zyklischen Verbindungen

Eine voraktivierte Lösung von geschütztem Aminosäurederivaten (6 Äquiv.), HATU (6 Äquiv.), HOBt (6 Äquiv.) und N-Methylmorpholin (NMM, 8 Äquiv.) in DMF wurde dem Harz zugegeben und 30-45 min lang umgesetzt. Das Verfahren wurde unter Verwendung von PyBOP anstelle von HATU als Kopplungsreagenz wiederholt. Das Waschen nach der Kopplung wurde mit DMF (3 x 2 mL), DCM (3 x 2 mL) und DMF (3 x 2 mL) durchgeführt. Hinweis: Die erste D-Val→L-Val-Kopplung erfolgte in DMF/DCM (1 1, 2 mL) aufgrund der besseren Harzquellungseigenschaften von DCM und HOAt anstelle von HOBt, um höhere Ausbeuten zu erzielen. Kappung: Pyridin/Ac₂O/DMF (1 : 3 : 6 v/v/v, 2 mL) wurde dem Harz zugegeben und reagierte 30 Minuten lang.

*Entschützen*: Fmoc-D-Val AC TG-Harz wurde in DMF (2 mL) 30 min lang gequollen. Die Fmoc-Gruppe wurde durch Behandlung mit einer Lösung von 2% DBU/10% Morpholin (v/v) in DMF (2 mL) für 3 min und weitere 12 min entfernt. Das Harz wurde mit DMF (3 x 2 mL), DCM (3 x 2 mL) und DMF (3 x 2 mL) gewaschen.

*Kopplungreaktionen*: Für die erste aufeinanderfolgende Val→Val Kopplung wurde dem Harz eine Lösung von Fmoc-L-Val-OH (6 Äquiv.), HATU (6 Äquiv.), HOAt (6 Äquiv.) und NMM (8 Äquiv.) in DMF/DCM (2 mL) zugegeben und 30-45 min gerührt. Die Doppelkupplung wurde mit PyBOP (6 Äquiv.) für 30-45 min durchgeführt. Das Harz wurde mit DMF (3 x 2 mL), DCM (3 x 2 mL), DMF (3 x 2 mL) gewaschen, und nicht umgesetzte harzgebundene Aminogruppen wurden durch Behandlung mit Pyridin/Ac2O/DMF (1 : 3 : 6 v/v/v, 2 mL) für 30 min verkappt. Das Harz wurde mit DMF (3 x 2 mL), DCM (3 x 2 mL), IPA (3 x 2 mL) und DMF (3 x 2 mL) gewaschen. Der harzgebundene Rückstand wurde einer iterativen Peptidassemblierung (Fmoc-SPPS) unter Verwendung von 2% DBU/10% Morpholin (v/v) in DMF (2 mL, 3 und 12 min) zur Fmoc-Entschützung und Fmoc-D/L-AA-OH (6 Äquiv.), HATU + PyBOP (6 Äquiv.), HOBt (6 Äquiv.) und NMM (8 Äquiv.) in DMF (2 mL) für 2 x 30-45 min, um jede Aminosäure doppelt zu kuppeln. Aminosäure-Kopplungs-Äquivalenz: Fmoc-D-Leu-OH, Fmoc-L-Trp(Boc)-OH, Fmoc-D-Val-OH, und schliesslich einem Thiazolidinbaustein.

*Spaltung:* Nach vollständiger Synthese der linearen Verbindung auf der festen Phase wurde die letzte Fmoc-Schutzgruppe entfernt und das Harz mit DMF (3 x 2 mL), DCM (3 x 2 mL), Toluol (3 x 2 mL), IPA (3 x 2 mL), Et₂O (3 x 2 mL) gewaschen und unter reduziertem Druck 3 h lang getrocknet. Das Peptid wurde durch Behandlung mit TFA/TIPS/H₂O (90 : 5 : 5 v/v/v, 2 mL) für 3 x 1 h und einen Waschschritt mit TFA (2 mL) für 10 min gespalten. Das Lösungsmittel wurde unter reduziertem Druck entfernt und die linearen Peptide wurden mit tBuOH/H₂O (1 : 1 v/v, 10 mL) lyophilisiert. Die lyophilisierten lineare Verbindung wurde anschließend ohne weitere Reinigung für die Makrolaktamisierung verwendet.

*Makrolaktamisierung*: Zu einer gerührten Lösung der linearen Verbindung in DMF (10 mL) wurde HOAt (21,0 mg, 0,156 mmol, 6 Äquiv.) und DIPEA (34 µL, 0,2 mmol, 8 Äquiv.) bei Raumtemperatur hinzugefügt. HATU (38 mg, 0,1 mmol, 4 Äquiv.) wurde in DMF (3 mL) gelöst und über 1 h zu der Lösung, die das lineare Peptid enthält, zugegeben. Die Endkonzentration des linearen Peptids betrug 2 mM. Die gelbe Lösung wurde 24 Stunden lang bei Raumtemperatur gerührt. Zu der Reaktionsmischung wurden die binären Lösungsmittelgemische *n*-Butanol (5 mL) und Chloroform (20 mL) oder bevorzugt n-Butanol (5 mL) und andere Alkohole oder Ether zugegeben und dann Wasser (30 mL) und gesättigte NaCl (5 mL) zugegeben. Nach Phasentrennung wurde die wässrige Phase mit Chloroform (2 x 20 mL) oder bevorzugt höheren Alkoholen oder Ether extrahiert. Die kombinierten organischen Extrakte wurden mit KHSO₄ (10% v/v, 4 x 35 mL), gesättigter NaHCO₃ (3 x 35 mL), ACN/H₂O (10% v/v, 2 x 35 mL) und gesättigter NaCl (3 x 35 mL) gewaschen. Das Lösungsmittel wurde unter reduziertem Druck entfernt und mit *tert*-Butanol/Wasser (1 : 1 v/v, 10 mL) lyophilisiert.

### Beispiel 3: Bioassay, MS und NMR mit zyklischen Verbindungen I. 1, I.2, I.3 und I.4

### A) Bioassay mit zyklischen Verbindungen I.1, I.2, I.3 und I.4

Die bakterizide Aktivitäten der zyklischen Verbindungen I.1 (ISW 1-0), I.2 (ISW 1-1), I.3 (ISW 1-2) und I.4 (ISW 1-4) (siehe Fig. 1) wurden mit einer seriellen Verdünnungsmethode bestimmt, wie zuvor bspw. in Schilling et al. Angew. Chem. Int. Ed. 2019, 58 (27), 9234-9238 beschrieben. Dazu wurde eine Zweifach-Verdünnungsreihe in Mikrotiterplatten (Stammlösung: 10 mg/mL bzw. 10 mM gelöst in DMSO) mit verschiedenen Konzentrationen der zyklischen Verbindungen I.1, I.2, I.3 und I.4 in Müller-Hinton-Bouillon (MHB; 0,2% Fleischextrakt, 1,75% Säurehydrolysat von Casein, 0,15% Stärke; Carl Roth GmbH + Co. KG) hergestellt. Die Verdünnungsreihe wurde jeweils mit einer Konzentration von 100 µg/mL bzw. 100 µM begonnen und mit zweifachen Verdünnungen (d.h. jeweils 1:1) durch eine Reihenverdünnung bis zur finalen Konzentration von 0,2 µg/mL bzw. 0,2 µM fortgesetzt.

Die Mikrotiterplatten wurden mit *S*. *aureus* USA300 LAC aus einer Übernachtkultur bis zu einer Enddichte von 1 × 10⁶ koloniebildenden Einheiten (cfu) pro mL beimpft (100 µL Kultur, OD600Start = 0,00125). Die inokulierten Mikrotiterplatten (inkl. der zyklischen Verbindungen in verschiedenen Konzentrationen oder DMSO als Negativkontrolle) wurden bei 37 °C für 22 h unter ständigem Schütteln bei 160 U/min inkubiert.

Der OD600-Wert jedes Wells wurde mit einem Mikrotiterplatten-Lesegerät gemessen und für die zyklischen Verbindungen die minimale Hemmkonzentration (MHK) bestimmt. Die Werte wurden bereits von den Blank-Proben bereinigt. Die Resultate sind in Fig. 2 dargestellt. Farbkodierung: Minimale Hemmkonzentration (MHK; in Engl.: minimal inhibitory contentration, MIC); rot: Wachstum; weiß: kein Wachstum. Der MHK-Wert kann beispielsweise als die Konzentration einer zu testenden antimikrobiellen Verbindung definiert werden, bei der die optische Dichte (OD600) weniger als 75% der Kontrolle ohne die zu testende antimikrobielle Verbindung erreicht (MHK75). Alternativ kann der MHK-Wert beispielsweise definiert werden als niedrigste Konzentration der zu testenden antimikrobiellen Verbindung bei der der OD600-Wert < 0,1 (a.u.) ist.

Die Durchführung des Bioassays mit den zyklischen Verbindungen unter Verwendung von *Streptococcus pneumoniae ATCC49619* und *Staphylococcus epidermidis,* als Beispiele weiterer grampositiver Bakterien, führt zu vergleichbaren Resultaten in Bezug auf die antibakterielle Wirksamkeit wie für *S*. *aureus* USA300 LAC. Gramnegative Bakterien wie *Pseudomonas aeruginosa PAO1* und *Escherichia coli DH5α* dagegen zeigen keine oder nur eine minimale (bspw. eine bis zu mehr als 30-fach größere MHK im Vergleich zu den grampositiven Bakterien) antibakterielle Wirksamkeit.

### B) Gekoppelte HPLC-Massenspektrometrie(MS)-Analyse der zyklischen Verbindungen

Die zyklischen Verbindungen I.1, I.2, I.3 und I.4 wurden in Methanol (MeOH, HPLC-MS-Qualität, 0,1 mgmL-1) gelöst, und der Analyt wurde einer Hochleistungsflüssigkeitschromatographie (HPLC) mit dem Dionex Ultimate 3000 HPLC-System (Thermo Fisher Scientific) mit einem Diodenarray-Detektor für das UV-Spektrum unterzogen.

*Stationäre Phase, Säule:* Nucleoshell EC RP-C18 (2.7 µm, 3Å, 150x3 mm, Macherey-Nagel GmbH & Co. KG). *Mobile Phase, Lösungsmittel:* Gradient mit 0 min (90 % Lösungsmittel A und 10 % Lösungsmittel B) bis 20 min (100 % Lösungsmittel B) mit einer Flussrate von 0,3 mL/min. Lösungsmittel A = MeOH mit 0,06 % Ameisensäure; Lösungsmittel B = H₂O mit 0,1 % Ameisensäure.

Die Massenspektren wurden mit einem hochauflösenden ESI-QTOF-Massenspektrometer (MaXis 4G, Bruker Daltonics GmbH) aufgenommen, das an das HPLC-Gerät gekoppelt war. Die ESI-Quelle wurde mit einem Zerstäuberdruck von 2,0 bar betrieben, und das Trockengas wurde auf 8,0 L/min bei 200 °C (Natriumformiat als internes Kalibriergas) eingestellt. Die MS/MS-Spektren wurden mit aktiviertem *Collision Energy Stepping* (d.h. unter Verwendung einer zeitlichen Abstufung der Kollisionsenergien was die Erfassung von MS-Spektren bei niedrigeren und höheren Kollisionsenergien ermöglicht) aufgenommen. Die Resultate Massensektrometer-Analse sind in Fig. 3 dargestellt.

### C) ¹H-NMR-Analyse der zyklischen Verbindungen

Es wurden Routine-¹H-NMR-Spektren der zyklischen Verbindungen I.1, I.2 und I.3 in DMSO-D₆[(CD₃)₂SO] als Lösungsmittel mit einem BrukerAMX-600 NMR-Spektrometer(¹H-NMR: 600 MHz; Bruker BioSpin GmbH) oder einem Bruker Avancelll-700 NMR-Spektrometer (¹H-NMR: 700 MHz; Bruker BioSpin GmbH) bei 30 °C (303 K) aufgenommen.

Die Resultate der ¹H-NMR-Analyse sind in Fig. 4 dargestellt. Alle chemischen Verschiebungen sind in Parts Per Million (ppm) relativ zu dem Lösungsmittel-Referenzpeak von (CD₃)₂SO (δH 2,50) angegeben. Kopplungskonstanten sind in Hertz angegeben.

### Beispiel 4: Bioassay in S. aureus LugIEFGH vs. S. aureus Wildtyp

Die minimalen Hemmkonzentrationen (MHK) der erfindungsgemäßen zyklischen Verbindung I.1 (ISW 1-0), sowie der bekannten Verbindungen Lugdunin und CCCP (Carbonylcyanid-3-chrlophenylhydrazon) wurden für zwei *Staphylococcus aureus* Stämme verglichen.

Bei den beiden *Staphylococcus aureus* Stämmen handelte es sich um 1) *Staphylococcus aureus* N315 Wildtyp (*S*. *aureus* Wt), und 2) den genetisch veränderten Stamm S. *aureus* N315 LugIEFGH. Dieser Stamm (*S. aureus* LugIEFGH) trägt zusätzlich die Gene für die Lugdunin ABC-Transporter LuglEFGH aus dem Lugdunin-Produzenten *S*. *lugdunensis.* Die Zellen der beiden Stämme wurden in seriellen Verdünnungen mit der Verbindung I.1, oder den bekannten Verbindungen Lugdunin oder CCCP versetzt und jeweils die MHK bestimmt. Die MHK-Werte wurden wie in Beispiel 3 A) beschrieben bestimmt, wobei die MHK als die Konzentration einer zu testenden antimikrobiellen Verbindung definiert ist, bei der die optische Dichte (OD600) weniger als 75% der Kontrolle ohne die zu testende antimikrobielle Verbindung erreicht (MHK75).

Die Ergebnisse sind in Fig. 5 dargestellt. Gezeigt ist das Verhältnis der MHK der drei Verbindungen für den *S*. *aureus* Stamm mit den ABC-Transporter-Genen LuglEFGH und den *S*. *aureus* Wildtyp: MHK_{(*S*. *aureus* LugIEFGH)} / MHK_{(*S*.*aureus* Wt)}. Beispielsweise ergibt dies für Lugdunin [MHK_{(*S*. *aureus* LugIEFGH)} : MHK_{(*S*}. _{*aureus* Wt)}] = 33,7 µg/mL : 8,15 µg/mL (bzw., ca. 10 µM : 41 µM). Während das MHK-Verhältnis für Lugdunin median 4,36 beträgt, weist die erfindungsgemäße Verbindung I.1 ein Verhältnis von 1,064 auf. Ist das Verhältnis nahe 1, so liegt keine Resistenzerhöhung durch die Transporter vor. Das bedeutet, dass für die erfindungsgemäße Verbindung I.1 keine Resistenzbildung zu erwarten ist, und es deutet darauf hin, dass sich die erfindungsgemäße Verbindung I.1 in Bezug auf das Wirkungsgeschehen von Lugdunin unterscheidet

## Patentansprüche

1. Zyklische Verbindung der Formel (I): 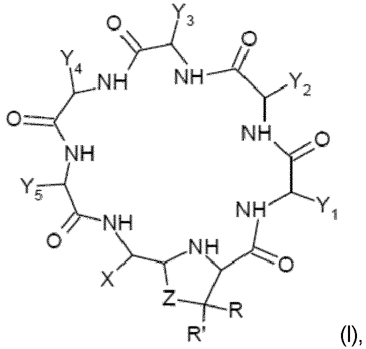 wobei:
- X und Y₁ bis Y₅ jeweils ausgewählt sind aus der Gruppe bestehend aus: Methyl, Ethyl, *n*-Propyl, 2-Propenyl (H₂C=CH-CH₂-), 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl (HC≡C-CH₂-), 1*H*-Indol-3-ylmethyl ( 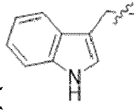 ), 1*N*-Methyl-1*H-*indol-3-ylmethyl ( 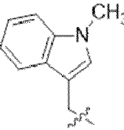 ), 3-Benzothienylmethyl ( 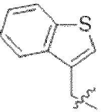 ), 1-Naphtylmethyl ( 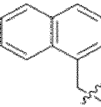 ), 9Anthracenylmethyl ( 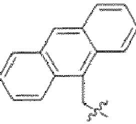 ) und Pyrenylmethyl;
- Z gleich O oder S ist;
- R und R' jeweils ausgewählt sind aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
- die C-Atome, die direkt mit den Substituenten Y₁, Y₂, Y₃, Y₄, Y₅ und X verbunden sind, in dieser Reihenfolge, eine jeweils alternierende absolute stereochemische Konfiguration aufweisen; und
- das C-Atom, welches mit dem Substituenten X verbunden ist, und das C-Atom in Position 4 des Thiazolidin- oder Oxazolidin-Rings, die gleiche absolute stereochemische Konfiguration aufweisen, wenn Z gleich O ist, und eine entgegengesetzte absolute stereochemische Konfiguration aufweisen, wenn Z gleich S ist;
unter der Bedingung, dass, wenn X, Y₁, Y₄ und Y₅ gleich 1-Methylethyl sind, Y₂ gleich 1*H*-Indol-3-ylmethyl ist, Y₃ gleich 2-Methylpropyl ist, R gleich H ist und R' gleich Methyl ist, Z nicht O ist;
und die Salze hiervon, die Solvate hiervon und die Solvate der Salze hiervon.

2. Zyklische Verbindung gemäß Anspruch 1, wobei
- R und R' beide Methyl sind; Y₂ gleich 1*H*-Indol-3-ylmethyl oder Pyrenylmethyl ist, falls Y₅ gleich 1-Methylethyl ist, oder Y₂ gleich 1-Methylethyl ist, falls Y₅ gleich 1*H-*Indol-3-ylmethyl oder Pyrenylmethyl ist; und X gleich 1-Methylethyl ist;
- R gleich H ist; R' gleich Methyl, Ethyl, *n*-Propyl, 1-Methylethyl, Benzyl oder Propargyl ist; Y₂ gleich 1*H*-Indol-3-ylmethyl oder Pyrenylmethyl ist, falls Y₅ gleich 1-Methylethyl ist, oder Y₂ gleich 1-Methylethyl ist, falls Y₅ gleich 1*H*-Indol-3-ylmethyl oder Pyrenylmethyl ist; und X gleich 1-Methylethyl ist;
- R gleich H ist; R' gleich Methyl, *n*-Propyl, 1-Methylethyl, Benzyl oder Propargyl ist; Y₂ gleich 1*H*-Indol-3-ylmethyl oder Pyrenylmethyl ist, falls Y₅ gleich 1-Methylethyl ist, oder Y₂ gleich 1-Methylethyl ist, falls Y₅ gleich 1*H*-Indol-3-ylmethyl oder Pyrenylmethyl ist; und X gleich 1-Methylethyl oder 2-Methylpropyl ist; oder
- R und R' beide 1-Methylethyl sind; und Y₂ gleich 1*H*-Indol-3-ylmethyl ist; und/oder
- **gekennzeichnet durch** eine der folgenden Formeln:

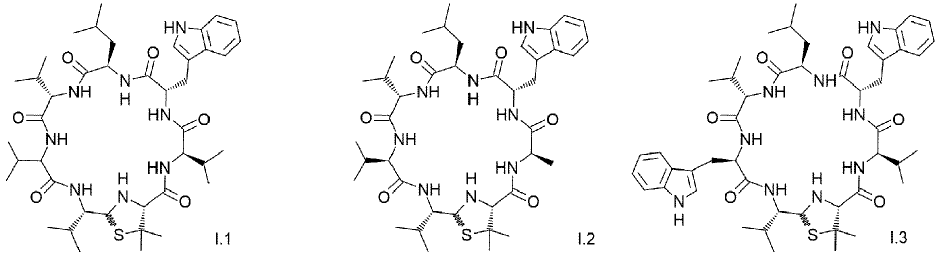

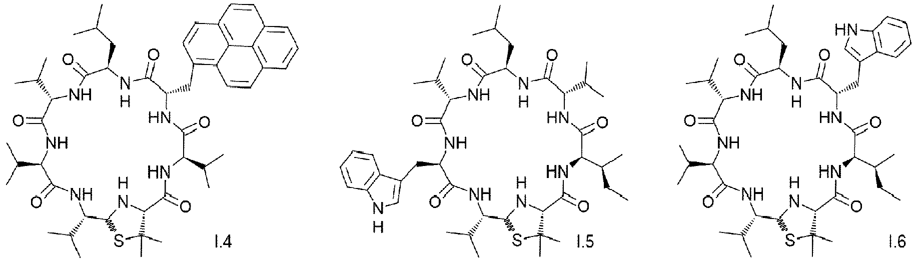

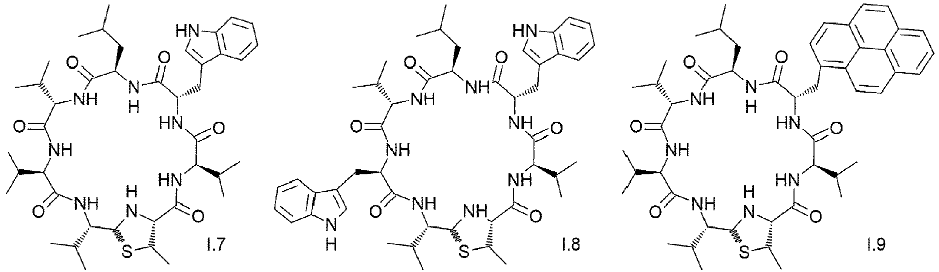

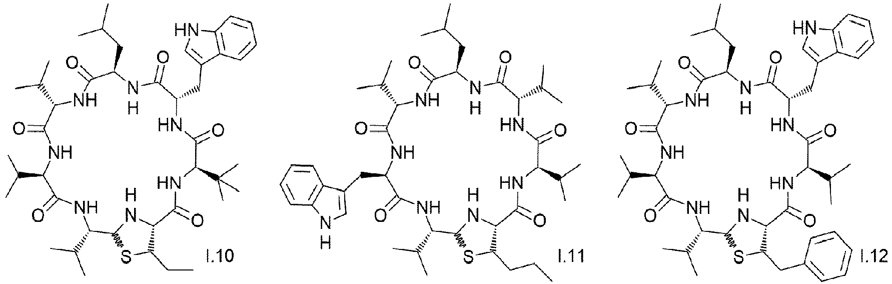

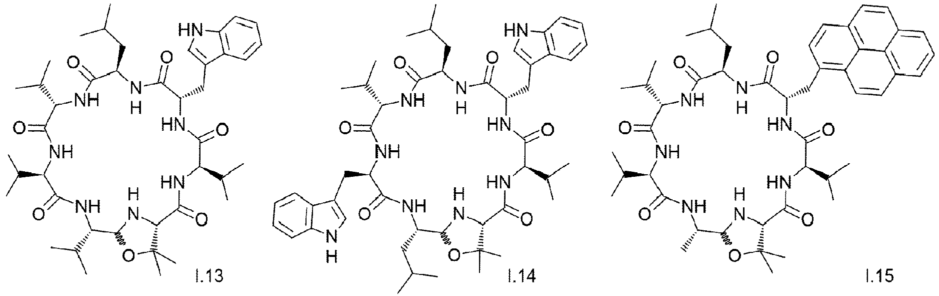

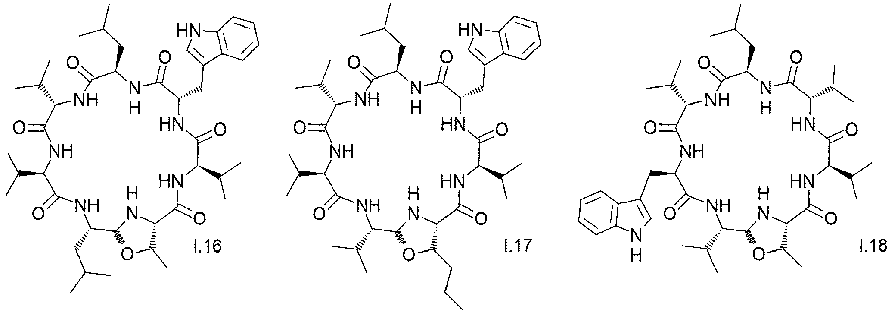

3. Zyklische Verbindung gemäß Anspruch 1 oder 2 mit antimikrobieller, insbesondere antiviraler und/oder nicht-selektiver antibakterieller, Wirksamkeit und/oder unterstützender Wirkung auf die angeborene Immunantwort, insbesondere der Haut.

4. Thiazolidin- oder Oxazolidin-Baustein der Formel (II): 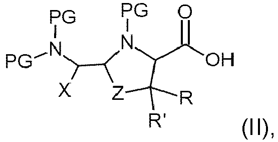 wobei:
- Z gleich O oder S ist;
- R und R' jeweils ausgewählt sind aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
- PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
- X ausgewählt ist aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl, Propargyl, 1*H*-Indol-3-ylmethyl, 1*N-*Methyl-1*H*-indol-3-ylmethyl, 3-Benzothienylmethyl, 1-Naphtylmethyl, 9-Anthracenylmethyl und Pyrenylmethyl;
- das C-Atom, welches mit dem Substituenten X verbunden ist, und das C-Atom in Position 4 des Thiazolidin- oder Oxazolidin-Rings, die gleiche absolute stereochemische Konfiguration aufweisen, wenn Z gleich O ist, und eine entgegengesetzte absolute stereochemische Konfiguration aufweisen, wenn Z gleich S ist;
unter der Bedingung, dass, wenn Z gleich S ist und X gleich Methyl, Benzyl oder 1H-Indol-3-ylmethyl ist, R nicht Methyl ist;
und die Salze hiervon, die Solvate hiervon und die Solvate der Salze hiervon.

5. Thiazolidin- oder Oxazolidin-Baustein gemäß Anspruch 4, **gekennzeichnet durch** eine der folgenden Formeln:

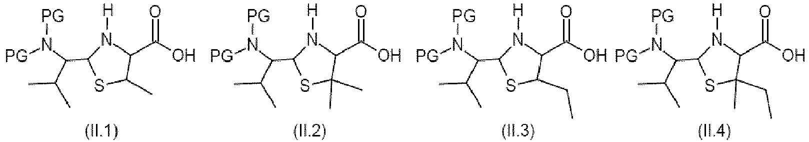

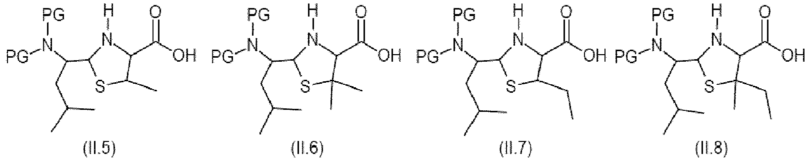

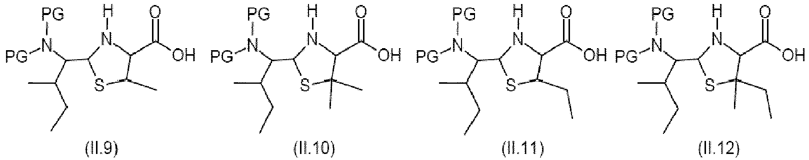

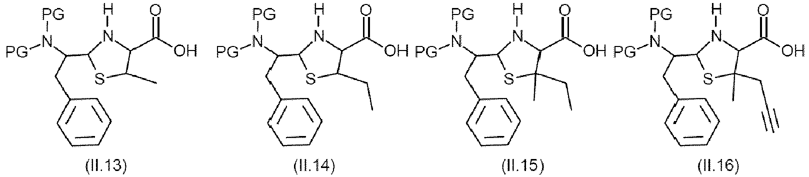

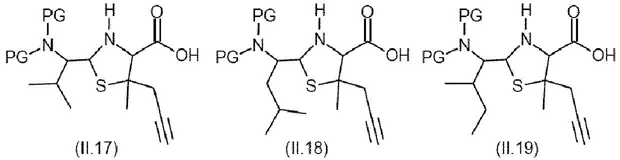

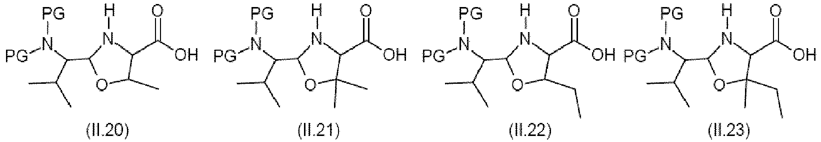

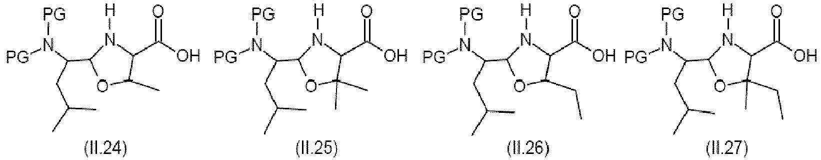

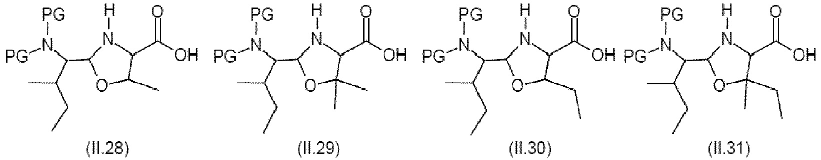

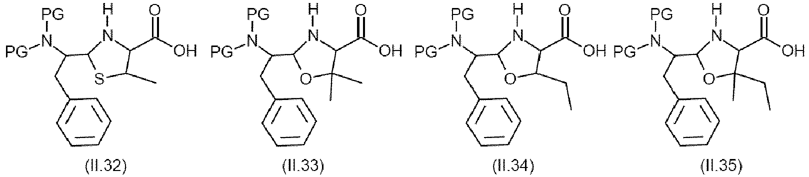

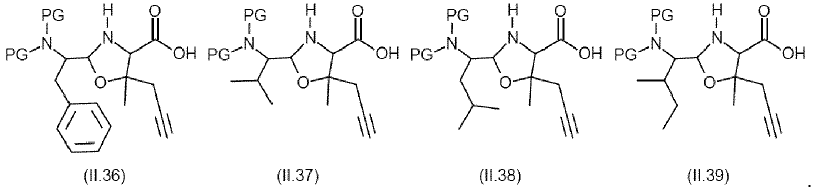

6. Verfahren zur Synthese eines Thiazolidin- oder Oxazolidin-Bausteins nach Anspruch 4 oder 5, umfassend die folgenden Schritte (a) bis (d):
(a) Bereitstellen eines geschützten Aminosäurederivats der Formel (III): 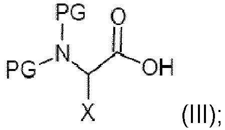
(b) Reduzieren des Aminosäurederivats (III) aus Schritt (a) zum Alkohol der Formel (IV) durch
(b1) in einem ersten Aktivierungsschritt, Aktivieren des Aminosäurederivats der Formel (III), mit einem Aktivierungsreagenz, in einem inerten Lösungsmittel; und
(b2) in einem zweiten Schritt, Reduzieren des Aktivierungsprodukts aus Schritt (b1), zu einer Verbindung der Formel (IV): 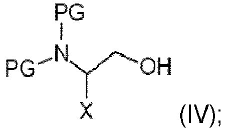
(c) Oxidieren der Verbindung (IV) aus Schritt (b), mit einem Oxidationsmittel, in einem aprotischen Lösungsmittel, und gegebenenfalls einem Wasserzusatz, zum entsprechenden Aldehyd der Formel (V): 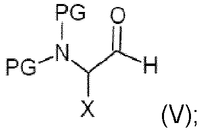
(d) Umsetzen (Kondensation) der Verbindung (V) aus Schritt (c), vorzugsweise in einem Lösungsmittelgemisch aus Wasser und einem polar-protischen Lösungsmittel, in einem Verhältnis Wasser: polar-protisches Lösungsmittel von ≤ 1 : 1 (v/v) und bei einer Reaktionstemperatur von mindestens 30 °C, mit einer Verbindung der Formel (VI): 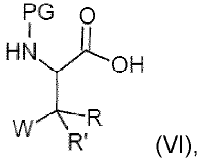 um einen Thiazolidin- oder Oxazolidin-Baustein gemäß Anspruch 4 oder 5 zu erhalten; wobei:
- W gleich SH oder OH ist;
- R und R' jeweils ausgewählt sind aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
- PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
- X ausgewählt ist aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl;
unter der Bedingung, dass, wenn Z gleich S ist und X gleich Methyl, Benzyl oder 1*H-*Indol-3-ylmethyl ist, R nicht Methyl ist.

7. Verfahren zur Synthese eines Thiazolidin- oder Oxazolidin-Bausteins nach Anspruch 6
- wobei in Schritt (b1) das Aktivierungsreagenz ausgewählt ist aus Thionylchlorid, Ethylenchloroformiat, Oxalylchlorid, N,O-Dimethylhydroxylamin, oder CDI, bevorzugt CDI; ein inertes zyklisches Etherlösungsmittel wie 1,4-Dioxan, Cyclopentylmethylether, 2-Methyltetrahydrofuran (2-Me-THF), Tetrahydrofuran (THF), bevorzugt THF, verwendet wird; und/oder die Aktivierung bei 15 bis 25 °C für mindestens 5 min, durchgeführt wird;
- wobei in Schritt (b2) das Aktivierungsprodukt unter Verwendung von Pd/C/H₂, Pd/C/Triethylsilan, NiCl₂/NaBH₄, Lithiumaluminumhydrid (LiAlH₄), Diisobutylaluminiumhydrid (DIBAL-H), Natriumcyanoborhydrid (NaBH₃CN) oder Natriumborhydrid (NaBH₄), bevorzugt NaBH₃CN oder NaBH₄, bei 0 °C für mindestens 15 min, reduziert wird;
- wobei in Schritt (c) das Oxidationsmittel Oxalylchlorid/DMSO/NEt₃ oder DMP, mehr bevorzugt DMP, ist; das aprotische Lösungsmittel Ethylacetat, Aceton, Toluol, THF, Chloroform, oder Dichlormethan (DCM), bevorzugt Toluol, THF, Chloroform oder DCM, bevorzugt DCM, ist; das Oxidationsmittel zwischen 1.0 bis 2.0 Äquiv.; und/oder zur Reaktion Wasser, bevorzugt zwischen 1.0 bis 1.5 Äquiv. zugegeben wird; und/oder
- wobei in Schritt (d) das Verhältnis von Wasser zu polar-protischem Lösungsmittel ≤ 1 : 1 (v/v) beträgt; das polar-protische Lösungsmittel Ameisensäure, Essigsäure, Ethanol, Isopropanol oder Methanol, bevorzugt Ethanol, Isopropanol oder Methanol, mehr bevorzugt Methanol, ist; und/oder die Reaktion bei mindestens 50 °C, bevorzugt zwischen 55 °C bis 75 °C durchgeführt wird.

8. Verfahren zur Festphasen-Peptidsynthese einer zyklischen Verbindung gemäß einem der Ansprüche 1 bis 3, umfassend die folgenden Schritte (i) bis (iv):
(i) Bereitstellen eines, über seine terminale Carboxylgruppe an eine feste Phase gebundenen,
- Aminosäurederivats der Formel (VII): 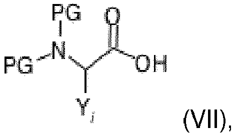 wobei i gleich 1 bis 5 ist;
- linearen Peptids bestehend aus zwei, drei, vier, oder fünf Aminosäurederivaten der Formel (VII); oder
- Thiazolidin- oder Oxazolidin-Bausteins gemäß Anspruch 4 oder 5;
wobei die terminale Aminogruppe durch mindestens eine Schutzgruppe geschützt ist;
(ii) ausgehend von dem Aminosäurederivat der Formel (VII), dem Peptid, bzw. dem Thiazolidin- oder Oxazolidin-Baustein aus Schritt (i), durch Durchführen einer oder mehrerer konsekutiver Kupplungsreaktionen, unter Anfügen
- eines oder mehrerer Aminosäurederivate der Formel (VII),
- eines oder mehrerer linearer Peptide bestehend aus zwei bis fünf Aminosäurederivaten der Formel (VII), oder
- eines Thiazolidin- oder Oxazolidin-Bausteins gemäß Anspruch 4 oder 5, wobei die terminale Aminogruppe durch mindestens eine Schutzgruppe geschützt ist,
schrittweise Festphasensynthese einer linearen, an die feste Phase gebundene, Verbindung, umfassend fünf Aminosäurederivate der Formel (VII), wobei i gleich 1 bis 5 ist, und ein Thiazolidin- oder Oxazolidin-Baustein nach Anspruch 4 oder 5;
(iii) Entfernen der Schutzgruppen des linearen, an die feste Phase gebundenen, Produkts aus Schritt (ii), und Abspalten von der festen Phase unter Bildung einer linearen, nicht an die feste Phase gebundenen, Verbindung;
(iv) Makrozyklisierung der linearen, nicht an die feste Phase gebundenen, Verbindung aus Schritt (iii), vorzugsweise durch Makrolaktamisierung, wodurch die zyklische Verbindung gemäß einem der Ansprüche 1 bis 3 erhalten wird;
wobei:
- X und Y₁ bis Y₅ jeweils ausgewählt sind aus der Gruppe bestehend aus: Methyl, Ethyl, *n*-Propyl, 2-Propenyl, 1-Methylethyl, *n*-Butyl, 2-Methylpropyl, 1-Methylpropyl, 1,1-Dimethylethyl, *n*-Pentyl, 3-Methylbutyl, Benzyl (Bn), Propargyl, 1*H*-Indol-3-ylmethyl, 1*N*-Methyl-1*H*-indol-3-ylmethyl, 3-Benzothienylmethyl, 1-Naphtylmethyl, 9-Anthracenylmethyl und Pyrenylmethyl;
- Z gleich O oder S ist;
- PG jeweils für H oder eine Schutzgruppe steht, wobei einzelne PG gleich oder verschieden sein können, und wobei die Schutzgruppe vorzugsweise ausgewählt ist aus der Gruppe bestehend aus 9-Fluorenylmethoxycarbonyl (Fmoc), tert-Butyloxycarbonyl (Boc), Benzyloxycarbonyl (Cbz), Benzyl (Bn), Trityl (Trt), Acetyl (Ac) und Tosyl (Ts);
- R und R' jeweils ausgewählt sind aus der Gruppe bestehend aus H, Methyl, Ethyl, *n*-Propyl, 1-Methylethyl, *n*-Butyl, 2-Methylpropyl, 1-Methylpropyl, Benzyl und Propargyl, unter der Bedingung, dass, wenn R gleich H ist, R' nicht H ist;
- unter der Bedingung, dass, wenn X, Y₁, Y₄ und Y₅ gleich 1-Methylethyl sind, Y₂ gleich 1*H*-Indol-3-ylmethyl ist, Y₃ gleich 2-Methylpropyl ist, R gleich H ist und R' gleich Methyl ist, Z nicht O ist.

9. Verfahren gemäß Anspruch 8, wobei der in Schritt (i) oder Schritt (ii) eingesetzte Thiazolidin- oder Oxazolidin-Baustein nach dem Verfahren gemäß Anspruch 6 oder 7 hergestellt wird.

10. Verfahren gemäß Anspruch 8 oder 9, wobei
- in Schritt (i) ein lineares Peptid bestehend aus zwei bis fünf, vorzugsweise fünf, Aminosäurederivaten der Formel (VII), wobei die terminale Aminogruppe durch mindestens eine Schutzgruppe geschützt ist, bereitgestellt wird;
- in Schritt (ii) die anzufügenden Aminosäurederivate der Formel (VII) oder Peptide, oder der anzufügende Thiazolidin- oder Oxazolidin-Baustein nicht an eine feste Phase gebunden sind bzw. ist; und in jeder der einen oder mehreren Kupplungsreaktionen, jeweils zunächst die mindestens eine Schutzgruppe von dem Aminosäurederivat der Formel (VII), dem Peptid, bzw. dem Thiazolidin- oder Oxazolidin-Baustein aus Schritt (i), entfernt wird, und dann die Kupplung mit dem, nicht an eine feste Phase gebunden, Aminosäurederivat der Formel (VII), dem Peptid, bzw. dem Thiazolidin- oder Oxazolidin-Baustein durchgeführt wird;
- in Schritt (iii) das Entschützen und Abspalten vom festen Träger durch eine erste Behandlung mit 0,5-4 % (v/v) DBU (1,8-Diazabicyclo[5.4.0]undec-7-en) und 5-15 % (v/v) Morpholin in DMF und eine zweite Behandlung mit Trifluoressigsäure (TFA), Triisopropylsilan (TIPS) und Wasser, vorzugsweise in einem Verhältnis von 90-92,5 : 2,5-5 : 5 (v/v/v), durchgeführt wird; und/oder
- in Schritt (iv) eine Makrolaktamisierung durchgeführt wird, wobei die lineare, an die feste Phase gebundene, Verbindung aus Schritt (iii) bei 15 bis 25 °C mit HATU, DIPEA und HOAt, unter Verwendung eines polaren aprotischen Lösungsmittels, vorzugsweise DMF, zyklisiert wird.

11. Zyklische Verbindung gemäß Anspruch 1 oder 3, Thiazolidin- oder Oxazolidin-Baustein gemäß Anspruch 4, Verfahren gemäß Anspruch 6 oder 7, und/oder Verfahren gemäß einem der Ansprüche 8 bis 10, wobei:
- X ausgewählt ist aus der Gruppe bestehend aus: 1-Methylethyl, 2-Methylpropyl, 1-Methylpropyl, Benzyl, Propargyl, 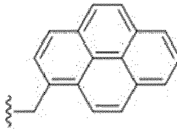 (1-Pyrenylmethyl) und 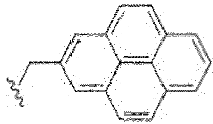 (2-Pyrenylmethyl);
- Y₁ bis Y₅ jeweils ausgewählt sind aus der Gruppe bestehend aus: Methyl, 1-Methylethyl, 2-Methylpropyl, 1-Methylpropyl, Benzyl, 1*H*-Indol-3-yl-methyl, Propargyl, 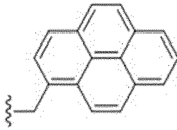 (1-Pyrenylmethyl) und 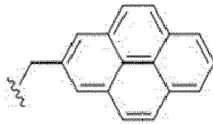 (2-Pyrenylmethyl);
- W gleich OH oder SH ist;
- Z gleich O oder S ist; und/oder
- R und R' jeweils ausgewählt sind aus der Gruppe bestehend aus H, Methyl, Ethyl, und Propargyl.

12. Kosmetische Zubereitung, enthaltend
(a) eine oder mehrere zyklische Verbindungen nach einem der Ansprüche 1 bis 3; und
(b) einen oder mehrere kosmetisch akzeptable Träger.

13. Kosmetische Zubereitung nach Anspruch 12, wobei die kosmetische Zubereitung in Form einer dermatologischen Zubereitung zur topischen Anwendung vorliegt; und/oder wobei die eine oder mehreren zyklischen Verbindungen eine antimikrobielle, vorzugsweise antivirale, antibakterielle und/oder antimykotische, insbesondere antivirale und/oder nicht-selektive antibakterielle, Wirksamkeit aufweisen, und/oder eine unterstützende Wirkung auf die angeborene Immunantwort, insbesondere der Haut, haben.
